(19) Europäisches Patentamt
European Patent Office
Office européen des brevets

(11) EP 2 487 175 A1

(12) **EUROPEAN PATENT APPLICATION**
published in accordance with Art. 153(4) EPC

(43) Date of publication:
**15.08.2012 Bulletin 2012/33**

(21) Application number: **10822029.4**

(22) Date of filing: **06.10.2010**

(51) Int Cl.:
***C07D 471/04*** (2006.01) ***A61K 31/4745*** (2006.01)
***A61P 11/02*** (2006.01) ***A61P 11/06*** (2006.01)
***A61P 17/04*** (2006.01) ***A61P 37/08*** (2006.01)
***A61P 43/00*** (2006.01) ***C07D 498/04*** (2006.01)

(86) International application number:
**PCT/JP2010/067511**

(87) International publication number:
**WO 2011/043359 (14.04.2011 Gazette 2011/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2009 JP 2009232201**
**24.05.2010 JP 2010118474**

(71) Applicant: **Kyowa Hakko Kirin Co., Ltd.**
**Tokyo 100-8185 (JP)**

(72) Inventors:
- **TAKADA, Chie (JP)**
- **SAEKI, Koji (JP)**
- **KAWASAKI, Hirokazu (JP)**
- **TAGAYA, Miho (JP)**
- **KOMAI, Masato (JP)**
- **IKEMURA, Toshihide (JP)**
- **YAMAGUCHI, Shinpei (JP)**
- **IIDA, Kyoichiro (JP)**

(74) Representative: **Harding, Charles Thomas**
**D Young & Co LLP**
**120 Holborn**
**London**
**EC1N 2DY (GB)**

(54) **PHARMACEUTICAL PRODUCT CONTAINING AROMATIC HETEROCYCLIC COMPOUND**

(57) A pharmaceutical composition comprising as an active ingredient, an aromatic heterocyclic compound represented by the formula (I):

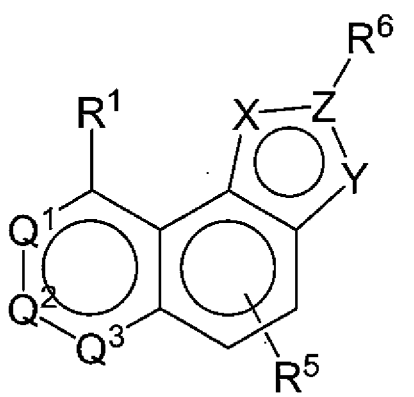

(I)

[wherein $Q^1$ represents $CR^2$ (wherein $R^2$ represents a hydrogen atom or the like) or the like; $Q^2$ represents $CR^3$ (wherein $R^3$ represents a hydrogen atom or the like) or the like; $Q^3$ represents a nitrogen atom or the like; $R^1$ represents $-C(=O)OR^{16}$ (wherein $R^{16}$ represents a hydrogen atom or the like) or the like; $R^5$ represents a hydrogen atom or the like; $R^6$ represents optionally substituted cycloalkyl or the like; X and Y may be the same or different and each represent CH in which H may be substituted with a substituent or the like; and Z represents a nitrogen atom or the like] or the like is provided.

EP 2 487 175 A1

## Description

### TECHNICAL FIELD

The present invention relates to a pharmaceutical composition, such as an inhibitor of hematopoietic prostaglandin $D_2$ synthase (H-PGDS) comprising an aromatic heterocyclic compound, or the like.

### BACKGROUND ART

H-PGDS is mainly expressed in a mast cell and Th2 cell and functions as an enzyme which catalyzes prostaglandin $D_2$ ($PGD_2$) production in a peripheral tissue (The Japanese Journal of Pharmacology, 2004, 123, 5-13). $PGD_2$ is considered to be involved in the onset and progress of allergic inflammation, and there have been reported that $PGD_2$ is detected at high concentration in bronchoalveolar lavage fluid in asthmatic patients (N. Eng. J. Med., 1986, 315, 800-804); that H-PGDS, which is a $PGD_2$ synthase, is highly expressed in nasal mucosa in allergic rhinitis patients (Am. J. Rhinol., 2006, 20, 342-348); that the rate of $PGD_2$ receptor positive cells correlates with the severity of atopic dermatitis (J. Invest. Dermatol., 2002, 119, 609-616); and the like.

Also, $PGD_2$ exerts physiological actions via two different receptors, that is, prostaglandin $D_2$ receptor (DP1) and chemoattractant receptor-homologous molecule expressed on Th2 cells (CRTH2). In both cases, $PGD_2$ acts in the direction of exacerbation of allergic inflammation (The Japanese Journal of Pharmacology, 2004, 123, 15-22; Nat. Rev. Drug Discov., 2007, 6, 313-325). Further, a CRTH2 antagonist is reported to correlate with chronic obstructive pulmonary disease (COPD) (Mol Cell Pharmacol, 2010, 2 (3), 89-96).

Meanwhile, it is known that H-PGDS is expressed in necrotic muscle of polymyositis patients and Duchenne muscular dystrophy (DMD) known as the most frequent muscular dystrophy (Acta Neuropathol., 2002, 104, 377-384), and it is reported that a H-PGDS inhibitor inhibits progressive muscle necrosis in mdx mice which is a model mice of muscular dystrophy (Am. J. Pathol., 2009, 174, 1735-1744).

Therefore, a compound which inhibits H-PGDS is expected to inhibit $PGD_2$ production, to inhibit the functions of DP1 and CRTH2 at the same time, and to be a therapeutic and/or preventive agent for, for example, a disease involving allergic inflammation (such as asthma, allergic rhinitis, or atopic dermatitis); COPD; a myodegenerative disease (such as muscular dystrophy or polymyositis); and the like.

As examples of a compound which inhibits H-PGDS, HQL-79 and the like are known (J. Biol. Chem., 2006, 281, 15277-15286) and are reported to inhibit airway inflammatory reaction in animal models (Jpn. J. Pharmacol., 1998, 78, 1-10 and Jpn. J. Pharmacol., 1998, 78, 11-22).

Meanwhile, as a 5,6-azole-fused quinoline derivative, 2-phenyl-2H[1,2,3]triazolo[4,5-f]quinoline-9-carboxylic acid derivatives are registered in CAS REGISTRY database as a chemical library (Registry Number: 663947-48-6, 575496-29-6, 508226-84-4, 491858-20-9, 303100-54-1, 303100-53-0, 303100-52-9, and 959290-53-0) (Non Patent Literature 1).

Also, 7-(furan-2-yl)-2-phenyl-2H-[1,2,3]triazolo [4,5-f]quinoline-9-carboxylic acid can be purchased from ASINEX (JSU code: JSU-0025748).

Table 1

Table 1

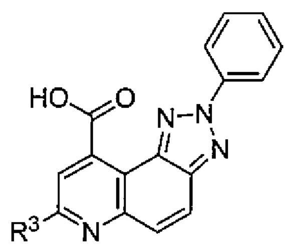

| Registry No. | $R^3$ | Registry No. | $R^3$ |
|---|---|---|---|
| 663947-48-6 | 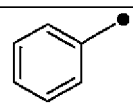 | 303100-54-1 | 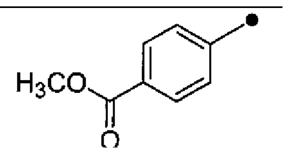 |

(continued)

| Registry No. | $R^3$ | Registry No. | $R^3$ |
|---|---|---|---|
| 575496-29-6 | 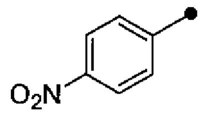 | 303100-53-0 | 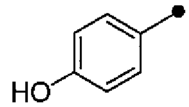 |
| 508226-84-4 | 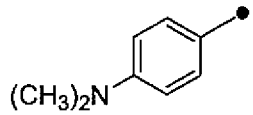 | 303100-52-9 | 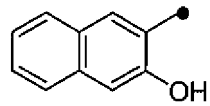 |
| 491858-20-9 | 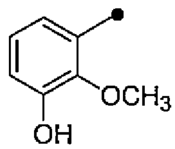 | 959290-53-0 | 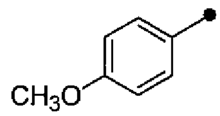 |

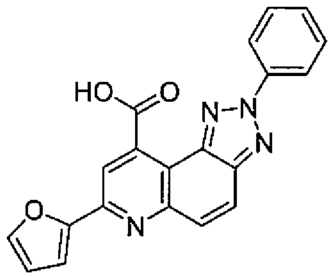

7-(furan-2-yl)-2-phenyl-2H-[1,2,3]triazolo[4,5-f]quinoline-99-carboxylic acid
JSU code:JSU-0025748

CITATION LIST

[Non Patent Literature]

[Non Patent Literature 1]

CAS REGISTRY Database Registry Number: 663947-48-6, 575496-29-6, 508226-84-4, 491858-20-9, 303100-54-1, 303100-53-0, 303100-52-9, and 959290-53-0

SUMMARY OF INVENTION

TECHNICAL PROBLEM

An object of the present invention is to provide a pharmaceutical composition comprising an aromatic heterocyclic compound or a pharmaceutically acceptable salt thereof [for example, a H-PGDS inhibitor, a therapeutic and/or preventive agent or the like for, for example, a disease involving allergic inflammation (such as asthma, allergic rhinitis, or atopic dermatitis); COPD; a myodegenerative disease (such as muscular dystrophy or polymyositis); and the like]; and an aromatic heterocyclic compound, a pharmaceutically acceptable salt thereof, or the like, having H-PGDS inhibitory activity and, for example, being useful as a therapeutic and/or preventive agent or the like for, for example, a disease involving allergic inflammation (such as asthma, allergic rhinitis, or atopic dermatitis); COPD; a myodegenerative disease (such as muscular dystrophy or polymyositis); and the like.

SOLUTION TO PROBLEM

The present invention relates to the following (1) to (49).

(1) A pharmaceutical composition comprising, as an active ingredient, an aromatic heterocyclic compound represented by the formula (I):

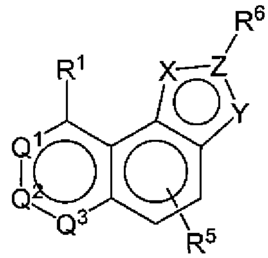

(I)

{wherein $Q^1$ represents $CR^2$ [wherein $R^2$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^7$ (wherein $R^7$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^8R^9$ (wherein $R^8$ and $R^9$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^8$ and $R^9$ are combined together with the adjacent nitrogen atom to form an optionally substituted nitrogen-containing heterocyclic group)], or a nitrogen atom;

when $Q^1$ is $CR^2$ (wherein $R^2$ has the same meaning as defined above),

$Q^2$ represents $CR^3$ [wherein $R^3$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{10}$ (wherein $R^{10}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{11}R^{12}$ (wherein $R^{11}$ and $R^{12}$ maybe the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{11}$ and $R^{12}$ are combined together with the adjacent nitrogen atom to form an optionally substituted nitrogen-containing heterocyclic group)], or a nitrogen atom;

when $Q^2$ is $CR^3$ (wherein $R^3$ has the same meaning as defined above), $Q^3$ represents a nitrogen atom;

when $Q^2$ is a nitrogen atom, $Q^3$ represents $CR^4$ [wherein $R^4$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{13}$ (wherein $R^{13}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{14}R^{15}$ (wherein $R^{14}$ and $R^{15}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{14}$ and $R^{15}$ are combined together with the adjacent nitrogen atom to form an optionally substituted nitrogen-containing heterocyclic group)];

when $Q^1$ is a nitrogen atom, $Q^2$ represents $CR^3$ (wherein $R^3$ has the same meaning as defined above) and $Q^3$ represents $CR^4$ (wherein $R^4$ has the same meaning as defined above) or a nitrogen atom;

$R^1$ represents -C (=O) $OR^{16}$ (wherein $R^{16}$ represents a hydrogen atom, optionally substituted lower alkyl, optionally substituted cycloalkyl, optionally substituted aryl, optionally substituted aroyl, an optionally substituted aliphatic heterocyclic group, or an optionally substituted aromatic heterocyclic group), $-C(=O)NR^{17}R^{18}$
[wherein $R^{17}$ and $R^{18}$ may be the same or different and each represent a hydrogen atom, optionally substituted lower alkyl, optionally substituted cycloalkyl, optionally substituted aryl, an optionally substituted aromatic heterocyclic group, or $-SO_2R^{19}$ (wherein $R^{19}$ represents optionally substituted lower alkyl, optionally substituted aryl, or an optionally substituted aromatic heterocyclic group), or $R^{17}$ and $R^{18}$ are combined together with the adjacent nitrogen atom to form an optionally substituted nitrogen-containing heterocyclic group], tetrazolyl, or the formula (III):

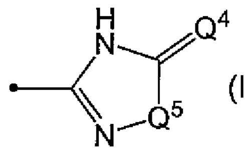

(III)

(wherein $Q^4$ and $Q^5$ may be the same or different and each represent an oxygen atom or a sulfur atom);
$R^5$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{20}$ (wherein $R^{20}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{21}R^{22}$ (wherein $R^{21}$ and $R^{22}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{21}$ and $R^{22}$ are combined together with the adjacent nitrogen atom to form an optionally substituted nitrogen-containing heterocyclic group);
$R^6$ represents optionally substituted cycloalkyl, optionally substituted aryl, or an optionally substituted aromatic heterocyclic group;
X and Y may be the same or different and each represent CH in which H may be substituted with a substituent, NH in which H may be substituted with a substituent, a nitrogen atom, an oxygen atom, or a sulfur atom; and Z represents a nitrogen atom or a carbon atom), or a pharmaceutically acceptable salt thereof.

(2) The pharmaceutical composition according to (1), wherein $Q^1$, $Q^2$, and $Q^3$ are $CR^2$ (wherein $R^2$ has the same meaning as defined above), $CR^3$ (wherein $R^3$ has the same meaning as defined above), and a nitrogen atom, respectively.

(3) The pharmaceutical composition according to (1), which comprises, as an active ingredient, an aromatic heterocyclic compound represented by the formula (I-1):

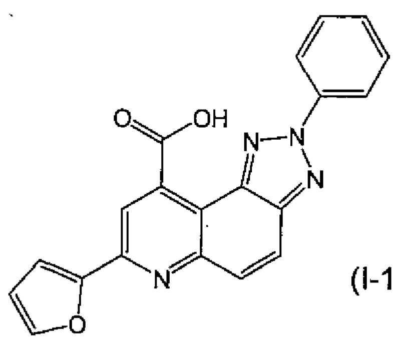

(I-1)

,

or a pharmaceutically acceptable salt thereof.

(4) The pharmaceutical composition according to any one of (1) to (3), which is an inhibitor of hematopoietic prostaglandin $D_2$ synthase (H-PGDS).

(5) The pharmaceutical composition according to any one of (1) to (3), which is a therapeutic and/or preventive agent for a disease involving H-PGDS.

(6) The pharmaceutical composition according to any one of (1) to (3), which is a therapeutic and/or preventive agent for a disease involving allergic inflammation, COPD, or a myodegenerative disease.

(7) The pharmaceutical composition according to any one of (1) to (3), which is a therapeutic and/or preventive agent for a disease selected from the group consisting of asthma, allergic rhinitis, atopic dermatitis, COPD, muscular dystrophy, and polymyositis.

(8) A method for inhibiting H-PGDS, comprising a step of administering an effective amount of the aromatic heterocyclic compound or a pharmaceutically acceptable salt thereof described in any one of (1) to (3).

(9) A method for treating and/or preventing a disease involving H-PGDS, comprising a step of administering an effective amount of the aromatic heterocyclic compound or a pharmaceutically acceptable salt thereof described in any one of (1) to (3).

(10) A method for treating and/or preventing a disease involving allergic inflammation, COPD, or a myodegenerative disease, comprising a step of administering an effective amount of the aromatic heterocyclic compound or a pharmaceutically acceptable salt thereof described in any one of (1) to (3).

(11) A method for treating and/or preventing a disease selected from the group consisting of asthma, allergic rhinitis, atopic dermatitis, COPD, muscular dystrophy, and polymyositis, comprising a step of administering an effective amount of the aromatic heterocyclic compound or a pharmaceutically acceptable salt thereof described in any one of (1) to (3).

(12) The aromatic heterocyclic compound or a pharmaceutically acceptable salt thereof described in any one of (1) to (3) for use in inhibiting H-PGDS.

(13) The aromatic heterocyclic compound or a pharmaceutically acceptable salt thereof described in any one of (1) to (3) for use in treating and/or preventing a disease involving H-PGDS.

(14) The aromatic heterocyclic compound or a pharmaceutically acceptable salt thereof described in any one of (1) to (3) for use in treating and/or preventing a disease involving allergic inflammation, COPD, or a myodegenerative disease.

(15) The aromatic heterocyclic compound or a pharmaceutically acceptable salt thereof described in any one of (1) to (3) for use in treating and/or preventing a disease selected from the group consisting of asthma, allergic rhinitis, atopic dermatitis, COPD, muscular dystrophy, and polymyositis.

(16) Use of the aromatic heterocyclic compound or a pharmaceutically acceptable salt thereof described in any one of (1) to (3) for the manufacture of a H-PGDS inhibitor.

(17) Use of the aromatic heterocyclic compound or a pharmaceutically acceptable salt thereof described in any one of (1) to (3) for the manufacture of a therapeutic and/or preventive agent for a disease involving H-PGDS.

(18) Use of the aromatic heterocyclic compound or a pharmaceutically acceptable salt thereof described in any one of (1) to (3) for the manufacture of a therapeutic and/or preventive agent for a disease involving allergic inflammation, COPD, or a myodegenerative disease.

(19) Use of the aromatic heterocyclic compound or a pharmaceutically acceptable salt thereof described in any one of (1) to (3) for the manufacture of a therapeutic and/or preventive agent for a disease selected from the group

consisting of asthma, allergic rhinitis, atopic dermatitis, COPD, muscular dystrophy, and polymyositis.

(20) An aromatic heterocyclic compound represented by the formula (I-A):

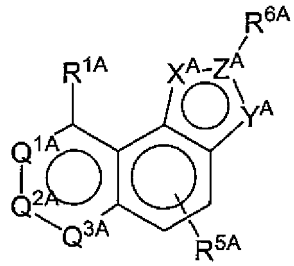

(I-A)

{wherein $Q^{1A}$ represents $CR^{2A}$ [wherein $R^{2A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{7A}$ (wherein $R^{7A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{8A}R^{9A}$ (wherein $R^{8A}$ and $R^{9A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic' group, optionally substituted aroyl, or optionally substituted lower alkanoyl, or $R^{8A}$ and $R^{9A}$ are combined together with the adjacent nitrogen atom to form an optionally substituted nitrogen-containing heterocyclic group)], or a nitrogen atom;

when $Q^{1A}$ is $CR^{2A}$ (wherein $R^{2A}$ has the same meaning as defined above),

$Q^{2A}$ represents $CR^{3A}$ [wherein $R^{3A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{10A}$ (wherein $R^{10A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{11A}R^{12A}$ (wherein $R^{11A}$ and $R^{12A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{11A}$ and $R^{12A}$ are combined together with the adjacent nitrogen atom to form an optionally substituted nitrogen-containing heterocyclic group)], or a nitrogen atom;

when $Q^{2A}$ is $CR^{3A}$ (wherein $R^{3A}$ has the same meaning as defined above), $Q^{3A}$ represents a nitrogen atom;

when $Q^{2A}$ is a nitrogen atom, $Q^{3A}$ represents $CR^{4A}$ [wherein $R^{4A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{13A}$ (wherein $R^{13A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{14A}R^{15A}$ (wherein $R^{14A}$ and $R^{15A}$ may be the same or different

and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{14A}$ and $R^{15A}$ are combined together with the adjacent nitrogen atom to form an optionally substituted nitrogen-containing heterocyclic group)];

when $Q^{1A}$ is a nitrogen atom, $Q^{2A}$ represents $CR^{3A}$ (wherein $R^{3A}$ has the same meaning as defined above) and $Q^{3A}$ represents $CR^{4A}$ (wherein $R^{4A}$ has the same meaning as defined above) or a nitrogen atom;

$R^{1A}$ represents $-C(=O)OR^{16A}$ (wherein $R^{16A}$ represents a hydrogen atom, optionally substituted lower alkyl, optionally substituted cycloalkyl, optionally substituted aryl, optionally substituted aroyl, an optionally substituted aliphatic heterocyclic group, or an optionally substituted aromatic heterocyclic group), $-C(=O)NR^{17A}R^{18A}$ [wherein $R^{17A}$ and $R^{18A}$ may be the same or different and each represent a hydrogen atom, optionally substituted lower alkyl, optionally substituted cycloalkyl, optionally substituted aryl, an optionally substituted aromatic heterocyclic group, or $-SO_2R^{19A}$ (wherein $R^{19A}$ represents optionally substituted lower alkyl, optionally substituted aryl, or an optionally substituted aromatic heterocyclic group), or $R^{17A}$ and $R^{18A}$ are combined together with the adjacent nitrogen atom to form an optionally substituted nitrogen-containing heterocyclic group], tetrazolyl, or the formula (III-A):

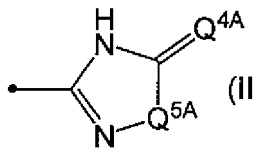

(III-A)

(wherein $Q^{4A}$ and $Q^{5A}$ may be the same or different and each represent an oxygen atom or a sulfur atom);

$R^{5A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{20A}$ (wherein $R^{20A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{21A}R^{22A}$ (wherein $R^{21A}$ and $R^{22A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{21A}$ and $R^{22A}$ are combined together with the adjacent nitrogen atom to form an optionally substituted nitrogen-containing heterocyclic group);

$R^{6A}$ represents optionally substituted cycloalkyl, optionally substituted aryl, or an optionally substituted aromatic heterocyclic group;

$X^A$ and $Y^A$ may be the same or different and each represent CH in which H may be substituted with a substituent, NH in which H may be substituted with a substituent, a nitrogen atom, an oxygen atom, or a sulfur atom; and $Z^A$ represents a nitrogen atom or a carbon atom

(excluding the case where $Q^{1A}$, $Q^{2A}$, and $Q^{3A}$ are CH, $CR^{3A}$, and, a nitrogen atom, respectively; $R^{3A}$ is phenyl, 4-nitrophenyl, 4-dimethylaminophenyl, 3-hydroxy-2-methoxyphenyl, 4-methoxycarbonylphenyl, 4-hydroxyphenyl, 3-hydroxynaphthalen-2-yl, 4-methoxyphenyl, or furan-2-yl; $R^{1A}$ is carboxy; $R^{5A}$ is a hydrogen atom; $X^A$, $Y^A$, and $Z^A$ are each a nitrogen atom; and $R^{6A}$ is phenyl)},

or a pharmaceutically acceptable salt thereof.

(21) The aromatic heterocyclic compound or a pharmaceutically acceptable salt thereof according to (20), wherein $Q^{1A}$, $Q^{2A}$, and $Q^{3A}$ are $CR^{2A}$ (wherein $R^{2A}$ has the same meaning as defined above), $CR^{3A}$ (wherein $R^{3A}$ has the same meaning as defined above), and a nitrogen atom, respectively.

(22) The aromatic heterocyclic compound or a pharmaceutically acceptable salt thereof according to (20) or (21), wherein $Q^{1A}$ is CH.

(23) The aromatic heterocyclic compound or a pharmaceutically acceptable salt thereof according to any one of (20) to (22), wherein $R^{1A}$ is $-C(=O)OR^{16B}$ (wherein $R^{16B}$ represents a hydrogen atom or optionally substituted lower

alkyl) or -C(=O)NR$^{17B}$R$^{18B}$ [wherein R$^{17B}$ and R$^{18B}$ may be the same or different and each represent a hydrogen atom, optionally substituted lower alkyl, or -SO$_2$R$^{19B}$ (wherein R$^{19B}$ represents optionally substituted lower alkyl)].

(24) The aromatic heterocyclic compound or a pharmaceutically acceptable salt thereof according to any one of (20) to (22), wherein R$^{1A}$ is -C(=O)OR$^{16C}$ (wherein R$^{16C}$ represents a hydrogen atom or lower alkyl) or -C(=O)NR$^{17C}$R$^{18C}$ [wherein R$^{17C}$ and R$^{18C}$ may be the same or different and each represent a hydrogen atom, lower alkyl, or -SO$_2$R$^{19C}$ (wherein R$^{19C}$ represents lower alkyl.

(25) The aromatic heterocyclic compound or a pharmaceutically acceptable salt thereof according to any one of (20) to (22), wherein R$^{1A}$ is carboxy.

(26) The aromatic heterocyclic compound or a pharmaceutically acceptable salt thereof according to any one of (20) to (25), wherein Q$^{2A}$ represents CR$^{3B}$ (wherein R$^{3B}$ represents halogen, optionally substituted aryl, an optionally substituted aromatic heterocyclic group, or an optionally substituted aliphatic heterocyclic group).

(27) The aromatic heterocyclic compound or a pharmaceutically acceptable salt thereof according to any one of (20) to (25), wherein Q$^{2A}$ represents CR$^{3C}$ (wherein R$^{3C}$ represents an optionally substituted aromatic heterocyclic group).

(28) The aromatic heterocyclic compound or a pharmaceutically acceptable salt thereof according to any one of (20) to (27), wherein R$^{5A}$ is a hydrogen atom.

(29) The aromatic heterocyclic compound or a pharmaceutically acceptable salt thereof according to any one of (20) to (28), wherein X$^A$, Y$^A$, and Z$^A$ are each a nitrogen atom.

(30) The aromatic heterocyclic compound or a pharmaceutically acceptable salt thereof according to any one of (20) to (28), wherein X$^A$ is an oxygen atom, Y$^A$ is a nitrogen atom, and Z$^A$ is a carbon atom.

(31) The aromatic heterocyclic compound or a pharmaceutically acceptable salt thereof according to any one of (20) to (30), wherein R$^{6A}$ is optionally substituted aryl, or an optionally substituted aromatic heterocyclic group.

(32) The aromatic heterocyclic compound or a pharmaceutically acceptable salt thereof according to any one of (20) to (30), wherein R$^{6A}$ is optionally substituted aryl.

(33) A pharmaceutical composition comprising, as an active ingredient, the aromatic heterocyclic compound or a pharmaceutically acceptable salt thereof described in any one of (20) to (32).

(34) A H-PGDS inhibitor comprising, as an active ingredient, the aromatic heterocyclic compound or a pharmaceutically acceptable salt thereof described in any one of (20) to (32).

(35) A therapeutic and/or preventive agent for a disease involving H-PGDS comprising, as an active ingredient, the aromatic heterocyclic compound or a pharmaceutically acceptable salt thereof described in any one of (20) to (32).

(36) A therapeutic and/or preventive agent for a disease involving allergic inflammation, COPD, or a myodegenerative disease comprising, as an active ingredient, the aromatic heterocyclic compound or a pharmaceutically acceptable salt thereof described in any one of (20) to (32).

(37) A therapeutic and/or preventive agent for a disease selected from the group consisting of asthma, allergic rhinitis, atopic dermatitis, COPD, muscular dystrophy, and polymyositis comprising, as an active ingredient, the aromatic heterocyclic compound or a pharmaceutically acceptable salt thereof described in any one of (20) to (32).

(38) A method for inhibiting H-PGDS, comprising a step of administering an effective amount of the aromatic heterocyclic compound or a pharmaceutically acceptable salt thereof described in any one of (20) to (32).

(39) A method for treating and/or preventing a disease involving H-PGDS, comprising a step of administering an effective amount of the aromatic heterocyclic compound or a pharmaceutically acceptable salt thereof described in any one of (20) to (32).

(40) A method for treating and/or preventing a disease involving allergic inflammation, COPD, or a myodegenerative disease, comprising a step of administering an effective amount of the aromatic heterocyclic compound or a pharmaceutically acceptable salt thereof described in any one of (20) to (32).

(41) A method for treating and/or preventing a disease selected from the group consisting of asthma, allergic rhinitis, atopic dermatitis, COPD, muscular dystrophy, and polymyositis, comprising a step of administering an effective amount of the aromatic heterocyclic compound or a pharmaceutically acceptable salt thereof described in any one of (20) to (32).

(42) The aromatic heterocyclic compound or a pharmaceutically acceptable salt thereof described in any one of (20) to (32) for use in inhibiting H-PGDS.

(43) The aromatic heterocyclic compound or a pharmaceutically acceptable salt thereof described in any one of (20) to (32) for use in treating and/or preventing a disease involving H-PGDS.

(44) The aromatic heterocyclic compound or a pharmaceutically acceptable salt thereof described in any one of (20) to (32) for use in treating and/or preventing a disease involving allergic inflammation, COPD, or a myodegenerative disease.

(45) The aromatic heterocyclic compound or a pharmaceutically acceptable salt thereof described in any one of (20) to (32) for use in treating and/or preventing a disease selected from the group consisting of asthma, allergic rhinitis, atopic dermatitis, COPD, muscular dystrophy, and polymyositis.

(46) Use of the aromatic heterocyclic compound or a pharmaceutically acceptable salt thereof described in any one of (20) to (32) for the manufacture of a H-PGDS inhibitor.

(47) Use of the aromatic heterocyclic compound or a pharmaceutically acceptable salt thereof described in any one of (20) to (32) for the manufacture of a therapeutic and/or preventive agent for a disease involving H-PGDS.

(48) Use of the aromatic heterocyclic compound or a pharmaceutically acceptable salt thereof described in any one of (20) to (32) for the manufacture of a therapeutic and/or preventive agent for a disease involving allergic inflammation, COPD, or a myodegenerative disease.

(49) Use of the aromatic heterocyclic compound or a pharmaceutically acceptable salt thereof described in any one of (20) to (32) for the manufacture of a therapeutic and/or preventive agent for a disease selected from the group consisting of asthma; allergic rhinitis, atopic dermatitis, COPD, muscular dystrophy, and polymyositis.

## ADVANTAGEOUS EFFECTS OF INVENTION

The present invention provides a pharmaceutical composition comprising an aromatic heterocyclic compound or a pharmaceutically acceptable salt thereof [for example, a H-PGDS inhibitor; a therapeutic and/or preventive agent or the like for, for example, a disease involving allergic inflammation (such as asthma, allergic rhinitis, or atopic dermatitis); COPD; a myodegenerative disease (such as muscular dystrophy or polymyositis); and the like] and an aromatic heterocyclic compound, a pharmaceutically acceptable salt thereof, or the like, having H-PGDS inhibitory effect and, for example, being useful as a therapeutic and/or preventive agent or the like for, for example, a disease involving allergic inflammation (such as asthma, allergic rhinitis, or atopic dermatitis); COPD; a myodegenerative disease (such as muscular dystrophy or polymyositis); and the like.

## DESCRIPTION OF EMBODIMENTS

Hereinafter, the compound represented by the formula (I) is referred to as Compound (I). The same applies to the other compounds having different formula numbers.
In the definitions of the respective groups in the formula (I),
examples of the lower alkyl and the lower alkyl moiety of the lower alkoxycarbonyl, the lower alkanoyl, the lower alkylsulfanyl, the lower alkylsulfinyl, the lower alkylsulfonyl, the lower alkylcarbamoyl, and the di-lower alkylcarbamoyl include linear or branched alkyl having 1 to 10 carbon atoms, and more specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl, and the like.
The two lower alkyl moieties of the di-lower alkylcarbamoyl may be the same or different.

Examples of the lower alkenyl include linear or branched alkenyl having 2 to 10 carbon atoms, and more specific examples thereof include vinyl, allyl, 1-propenyl, butenyl, pentenyl, hexenyl, heptenyl, octenyl, nonenyl, decenyl, and the like.

Examples of the lower alkynyl include linear or branched alkynyl having 2 to 10 carbon atoms, and more specific examples thereof include ethynyl, propynyl, butynyl, pentynyl, hexynyl, heptynyl, octynyl, nonynyl, decynyl, and the like. Examples of the cycloalkyl include cycloalkyl having 3 to 8 carbon atoms, and more specific examples thereof include cyclopropyl, cyclobutyl, cyclopentyl, cyclohexyl, cycloheptyl, cyclooctyl, and the like.

Examples of the aryl and the aryl moiety of the arylsulfonyl and the aroyl include aryl having 6 to 14 carbon atoms, and more specific examples thereof include phenyl, naphthyl, azulenyl, anthryl, and the like.

Examples of the aliphatic heterocyclic group include a 5- or 6-membered monocyclic aliphatic heterocyclic group which contains at least one atom selected from a nitrogen atom, an oxygen atom, and a sulfur atom, a bicyclic or tricyclic condensed aliphatic heterocyclic group in which 3- to 8-membered rings are fused and at least one atom selected from a nitrogen atom, an oxygen atom and a sulfur atom is contained, and the like, and more specific examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxiranyl, tetrahydrofuranyl, tetrahydro-2H-pyranyl, 5,6-dihydro-2H-pyranyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolidinyl, dihydrobenzoxazolyl, dihydrobenzothioxazolyl, benzodioxolinyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl, dihydrobenzodioxanyl, and the like.

Examples of the aromatic heterocyclic group and the aromatic heterocyclic moiety of the aromatic heterocyclic carbonyl include a 5- or 6-membered monocyclic aromatic heterocyclic group which contains at least one atom selected from a nitrogen atom, an oxygen atom and a sulfur atom, a bicyclic or tricyclic condensed aromatic heterocyclic group in which 3-to 8-membered rings are fused and at least one atom selected from a nitrogen atom, an oxygen atom and a sulfur atom is contained, and the like, and more specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl, pyrrolopyrimidinyl, imidazopyridinyl, purinyl, quinolinyl, isoquinolinyl, cinnolinyl, phthalazinyl, quinazolinyl, quinoxalinyl, naphthyridinyl, and the like.

Examples of the nitrogen-containing heterocyclic group formed together with the adjacent nitrogen atom include a 5-or 6-membered monocyclic heterocyclic group which contains at least one nitrogen atom (the monocyclic heterocyclic group may further contain another nitrogen atom, an oxygen atom or a sulfur atom), a bicyclic or tricyclic condensed heterocyclic group in which 3- to 8-membered rings are fused and at least one nitrogen atom is contained (the condensed heterocyclic group may further contain another nitrogen atom, an oxygen atom or a sulfur atom), and the like, and more specific examples thereof include aziridinyl, azetidinyl, pyrrolidinyl, piperidino, azepanyl, pyrrolyl, imidazolidinyl, imidazolyl, pyrazolidinyl, pyrazolinyl, pyrazolyl, piperazinyl, homopiperazinyl, oxazolidinyl, 2H-oxazolyl, thioxazolidinyl, 2H-thioxazolyl, morpholino, thiomorpholinyl, dihydroindolyl, dihydroisoindolyl, indolyl, isoindolyl, tetrahydroquinolyl, tetrahydroisoquinolyl, dihydrobenzoxazolyl, dihydrobenzothioxazolyl, benzimidazolidinyl, benzimidazolyl, dihydroindazolyl, indazolyl, benzotriazolyl, pyrrolopyridinyl pyrrolopyrimidinyl, imidazopyridinyl, purinyl, and the like.

The halogen means each atom of a fluorine atom, a chlorine atom, a bromine atom, and an iodine atom.

(i) The substituents of the optionally substituted lower alkyl, the optionally substituted lower alkenyl, the optionally substituted lower alkynyl, the optionally substituted lower alkoxycarbonyl, the optionally substituted lower alkanoyl, the optionally substituted lower alkylsulfanyl, the optionally substituted lower alkylsulfinyl, the optionally substituted lower alkylsulfonyl, the optionally substituted lower alkylcarbamoyl, and the optionally substituted di-lower alkylcarbamoyl may be the same or different and, for example, 1 to 3 in number, and specific examples thereof include halogen, hydroxy, sulfanyl, nitro, cyano, carboxy, carbamoyl, $C_{3-8}$ cycloalkyl, $C_{6-14}$ aryl, an aliphatic heterocyclic group, an aromatic heterocyclic group,

$C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy,

$C_{1-10}$ alkylsulfanyl,

tri($C_{1-10}$ alkyl) silyl,

$-NR^{20A}R^{21A}$ (wherein $R^{20A}$ and $R^{21A}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like),

$C_{2-11}$ alkanoyl, $C_{7-15}$ aroyl, $C_{1-10}$ alkoxycarbonyl, $C_{6-14}$ aryloxycarbonyl,

$-C(=O)NR^{20B}R^{21B}$ (wherein $R^{20B}$ and $R^{21B}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl,

$C_{2-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like), and the like.

(ii) The substituents of the optionally substituted aryl, the optionally substituted arylsulfonyl, the optionally substituted aroyl, the optionally substituted aromatic heterocyclic group, and the optionally substituted aromatic heterocyclic carbonyl may be the same or different and, for example, 1 to 3 in number, and specific examples thereof include halogen, hydroxy, sulfanyl, nitro, cyano, carboxy, carbamoyl,

optionally substituted $C_{1-10}$ alkyl [examples of the substituents of the optionally substituted $C_{1-10}$ alkyl include the groups illustrated in (i) as examples of the substituents of the optionally substituted lower alkyl, and the like],

$C_{3-8}$ cycloalkyl,

optionally substituted $C_{6-14}$ aryl [examples of the substituents (a) of the optionally substituted $C_{6-14}$ aryl include $C_{1-10}$ alkyl, trifluoromethyl, and the like, in addition to the groups illustrated in (i) as examples of the substituents of the optionally substituted lower alkyl, and the like],

an optionally substituted aliphatic heterocyclic group (examples of the substituents of the optionally substituted aliphatic heterocyclic group include the substituents (a) of the above optionally substituted $C_{6-14}$ aryl, and the like),

an aromatic heterocyclic group,

$C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy,

$C_{1-10}$ alkylsulfanyl, $C_{1-10}$ alkylsulfonyl,

$-NR^{22A}R^{23A}$ (wherein $R^{22A}$ and $R^{23A}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like),

$C_{2-11}$ alkanoyl, $C_{7-15}$ aroyl, $C_{1-10}$ alkoxycarbonyl, $C_{6-14}$ aryloxycarbonyl,

$-C(=O)NR^{22B}R^{23B}$ (wherein $R^{22B}$ and $R^{23B}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like), and the like.

(iii) The substituents of the optionally substituted cycloalkyl, the optionally substituted aliphatic heterocyclic group, and the nitrogen-containing heterocyclic group formed together with the adjacent nitrogen atom may be the same or different and, for example, 1 to 3 in number, and specific examples thereof include

oxo, halogen, hydroxy, sulfanyl, nitro, cyano, carboxy, carbamoyl,

optionally substituted $C_{1-10}$ alkyl [examples of the substituents of the optionally substituted $C_{1-10}$ alkyl include the groups illustrated in the above (i) as examples of the substituents of the optionally substituted lower alkyl, and the like],

$C_{3-8}$ cycloalkyl,

optionally substituted $C_{6-11}$ aryl [examples of the substituents of the optionally substituted $C_{6-14}$ aryl include the groups illustrated in the above (ii) as examples of the substituents of the optionally substituted aryl, and the like],

an aliphatic heterocyclic group, an aromatic heterocyclic group,

$C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy,

$C_{1-10}$ alkylsulfanyl, $C_{1-10}$ alkylsulfonyl, $-NR^{24A}R^{25A}$ (wherein $R^{24A}$ and $R^{25A}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di ($C_{1-10}$ alkyl) carbamoyl, $C_{1-10}$ alkylsulfonyl, $C_{6-14}$ arylsulfonyl, or the like), $C_{2-11}$ alkanoyl, $C_{7-15}$ aroyl, $C_{1-10}$ alkoxycarbonyl, $C_{6-14}$ aryloxycarbonyl, $C_{1-10}$ alkylcarbamoyl, and di($C_{1-10}$ alkyl)carbamoyl,

$-C(=O)NR^{24B}R^{25B}$ (wherein $R^{24B}$ and $R^{25B}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like), and the like.

(iv) The substituent of the CH in which H may be substituted with a substituent, and NH in which H may be substituted with a substituent is 1 in number, and

examples thereof include halogen, hydroxy, sulfanyl, nitro, cyano, carboxy, carbamoyl,

optionally substituted $C_{1-10}$ alkyl [examples of the substituents of the optionally substituted $C_{1-10}$ alkyl include the groups illustrated in the above (i) as examples of the substituents of the optionally substituted lower alkyl, and the like],

$C_{3-8}$ cycloalkyl,

optionally substituted $C_{6-14}$ aryl [examples of the substituents of the optionally substituted $C_{6-14}$ aryl include the substituents (a) of the optionally substituted $C_{6-14}$ aryl, and the like],

an optionally substituted aliphatic heterocyclic group (examples of the substituents of the optionally substituted aliphatic heterocyclic group include the substituents (a) of the optionally substituted $C_{6-14}$ aryl, and the like),

an aromatic heterocyclic group,

$C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy,

$C_{1-10}$ alkylsulfanyl, $C_{1-10}$ alkylsulfonyl,
$-NR^{26A}R^{27A}$ (wherein $R^{26A}$ and $R^{27A}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like),
$C_{2-11}$ alkanoyl, $C_{7-15}$ aroyl, $C_{1-10}$ alkoxycarbonyl, $C_{6-14}$ aryloxycarbonyl,
$-C(=O)NR^{26B}R^{27B}$ (wherein $R^{26B}$ and $R^{27B}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, $C_{7-16}$ aralkyloxycarbonyl, $C_{1-10}$ alkoxy, $C_{1-10}$ alkylcarbamoyl, di($C_{1-10}$ alkyl) carbamoyl, $C_{1-10}$ alkylsulfonyl, $C_{6-14}$ arylsulfonyl, or the like), and the like.

Examples of the $C_{1-10}$ alkyl and the $C_{1-10}$ alkyl moiety of the $C_{1-10}$ alkoxy, the $C_{1-10}$ alkoxycarbonyl, the $C_{2-11}$ alkanoyl, the $C_{2-11}$ alkanoyloxy, the tri($C_{1-10}$ alkyl)silyl, the $C_{1-10}$ alkylsulfanyl, the $C_{1-10}$ alkylsulfonyl, the $C_{1-10}$ alkylcarbamoyl and the di($C_{1-10}$ alkyl)carbamoyl described above include the groups illustrated as examples of the lower alkyl. Each of the three $C_{1-10}$ alkyl moieties of the tri($C_{1-10}$ alkyl) silyl may be the same or different, and each of the two $C_{1-10}$ alkyl moieties of the di($C_{1-10}$ alkyl)carbamoyl may be the same or different.

Examples of the $C_{3-8}$ cycloalkyl and the cycloalkyl moiety of the $C_{3-8}$ cycloalkoxy include the groups illustrated in the examples of the cycloalkyl.
Examples of the $C_{6-14}$ aryl and the aryl moiety of the $C_{6-14}$ aryloxy, the $C_{6-14}$ aryloxycarbonyl, the $C_{7-15}$ aroyl, the $C_{7-15}$ aroyloxy and the $C_{6-14}$ arylsulfonyl include the groups illustrated in the examples of the aryl.

Examples of the aryl moiety of the $C_{7-16}$ aralkyl, the $C_{7-16}$ aralkyloxy, and the $C_{7-16}$ aralkyloxycarbonyl include the groups illustrated in the examples of the aryl, and examples of the alkylene moiety thereof include $C_{1-10}$ alkylene, more specifically, groups obtainable by removing a hydrogen atom from the groups illustrated in the above lower alkyl.
Examples of the aliphatic heterocyclic group, the aromatic heterocyclic group, and the halogen include the groups illustrated in the above aliphatic heterocyclic group, the above aromatic heterocyclic group, and the above halogen, respectively.

Examples of the pharmaceutical composition of the present invention include pharmaceutical compositions comprising, as an active ingredient, Compound (I) or a pharmaceutically acceptable salt thereof, and being used for treating and/or preventing a disease, but are not particularly limited thereto.
Examples thereof include the pharmaceutical preparation described later, more specifically, a H-PGDS inhibitor; a therapeutic and/or preventive agent for a disease involving H-PGDS; a therapeutic and/or preventive agent for a disease involving allergic inflammation, COPD, or a myodegenerative disease; a therapeutic and/or preventive agent for a disease selected from the group consisting of asthma, allergic rhinitis, atopic dermatitis, COPD, muscular dystrophy, and polymyositis; or the like, comprising, as an active ingredient, Compound (I) or a pharmaceutically acceptable salt thereof.

In the definitions of the groups of Compound (I) and the like, besides the above (1) to (46), $Q^1$ and $Q^{1A}$ of Compound (I) and Compound (I-A) are preferably CH, $Q^2$ and $Q^{2A}$ thereof are preferably $CR^{2B}$ [wherein $R^{2B}$ is preferably optionally substituted aryl, an optionally substituted aromatic heterocyclic group, or an optionally substituted aliphatic heterocyclic group, more preferably optionally substituted aryl or an optionally substituted aromatic heterocyclic group; the aryl of the above optionally substituted aryl is preferably phenyl; the substituent of the above optionally substituted aryl is preferably methanesulfonylamino, methanesulfonylmethylamino, methanesulfonylaminocarbonyl, methanesulfonylmethylaminocarbonyl, benzenesulfonylamino, benzenesulfonylaminocarbonyl, benzenesulfonylmethylamino, benzenesulfonylmethylaminocarbonyl, methoxy(methyl)carbamoyl, carboxy, or 3-ethylureido, more preferably methanesulfonylamino, methanesulfonylmethylamino, methanesulfonylaminocarbonyl, methanesulfonylmethylaminocarbonyl, benzenesulfonylamino, benzenesulfonylaminocarbonyl, benzenesulfonylmethylamino, or benzenesulfonylmethylaminocarbonyl;
the aromatic heterocyclic group of the above optionally substituted aromatic heterocyclic group is preferably furyl, pyrrolyl, pyrazolyl, thienyl, oxazolyl, triazolyl, orpyridyl, more preferably furyl, pyrrolyl, or pyrazolyl; the substituent of the above optionally substituted aromatic heterocyclic group is preferably tert-butoxycarbonyl, methyl, carboxy, 2-ethoxy-2-oxoethyl, carboxymethyl, 2-methoxyethyl, 2-morpholinoethyl, benzyl, or methoxycarbonyl, more preferably carboxy or benzyl;
the aliphatic heterocyclic group of the above optionally substituted aliphatic heterocyclic group is preferably pyrrolidinyl, piperazinyl, or piperidino; and the substituent of the above optionally substituted aliphatic heterocyclic group is preferably acetyl, ethoxycarbonyl, ethylsulfonyl, benzoyl, hydroxy, dimethylamino, acetamido, 2-hydroxyethyl, hydroxymethyl, 2-ethoxy-2-oxyethyl, 3-hydroxyphenyl, or 4-ethoxycarbonylphenyl],
$Q^3$ and $Q^{3A}$ thereof are preferably a nitrogen atom, $R^1$ and $R^{1A}$ thereof are preferably carboxy, $R^5$ and $R^{5A}$ thereof are preferably a hydrogen atom, and $R^6$ and $R^{6A}$ thereof are preferably phenyl, 3-methylphenyl, 2-fluorophenyl, 3-fluorophenyl, 4-fluorophenyl, 3-methoxyphenyl, 3-chlorophenyl, or 3-trifluoromethylphenyl, more preferably phenyl, 3-methylphenyl, or 3-fluorophenyl.

Examples of the pharmaceutically acceptable salt of Compound (I) include pharmaceutically acceptable acid

addition salts, metal salts, ammonium salts, organic amine addition salts, amino acid addition salts, and the like. Examples of the pharmaceutically acceptable acid addition salts of Compound (I) include inorganic acid salts such as hydrochlorides, hydrobromides, nitrates, sulfates, and phosphates, organic acid salts such as acetates, oxalates, maleates, fumarates, citrates, benzoates, and methanesulfonates, and the like. Examples of the pharmaceutically acceptable metal salts include alkali metal salts such as sodium salts and potassium salts; alkaline earth metal salts such as magnesium salts and calcium salts; aluminum salts; zinc salts; and the like. Examples of the pharmaceutically acceptable ammonium salts include salts of ammonium, tetramethylammonium, and the like. Examples of the pharmaceutically acceptable organic amine addition salts include addition salts of morpholine, piperidine, and the like. Examples of the pharmaceutically acceptable amino acid addition salts include addition salts of lysine, glycine, phenylalanine, aspartic acid, glutamic acid, and the like.

Hereinafter, production methods of Compound (I) will be described.

In the production methods described below, in a case where a defined group changes under the conditions of the production methods or is not suitable for carrying out the production methods, it is possible to produce a desired compound with use of a method commonly used in synthetic organic chemistry for introducing and removing a protecting group (for example, the method described in T. W. Greene, Protective Groups in Organic Synthesis, 3rd edition, John Wiley & Sons Inc. (1999), or the like). Further, the order of reaction steps, such as introduction of substituents, may be changed as necessary.

Production Method 1

Among Compounds (I), Compound (I-A), in which X, Y, and Z are nitrogen atoms, can be produced according to the following production method.

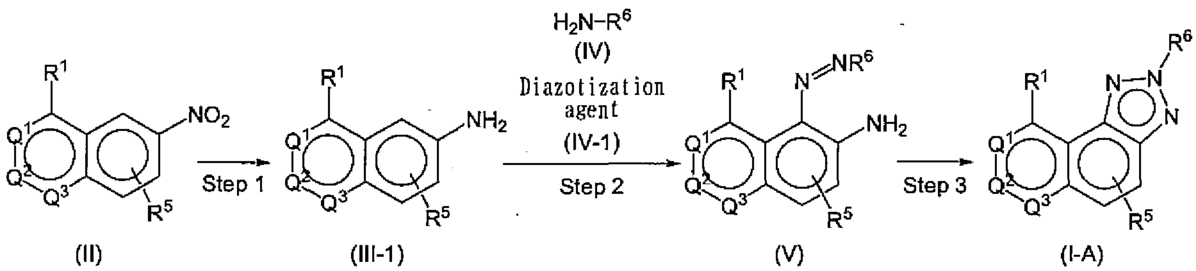


(wherein $R^1$, $R^5$, $R^6$, $Q^1$, $Q^2$, and $Q^3$ have the same meanings as defined above, respectively)

Step 1

Compound (III-1) can be produced by treating Compound (II) with 1 to 100 equivalents, preferably 1 to 10 equivalents of a reducing agent, in a solvent, in the presence of 1 equivalent to a large excess amount of an acid, at a temperature between -78˚C and the boiling point of the solvent used for 5 minutes to 72 hours.

Examples of the acid include concentrated hydrochloric acid, concentrated sulfuric acid, acetic acid, and the like. Examples of the reducing agent include tin, iron, zinc, and the like.

Examples of the solvent include methanol, ethanol, isopropyl alcohol, water, and the like. These solvents may be used alone or as a mixture thereof.

Compound (III-1) can also be produced by the following method.

Compound (III-1) can be produced by treating Compound (II) without a solvent or in a solvent, in the presence of preferably 0. 01 to 50% by weight of a suitable catalyst relative to Compound (II), at a temperature between -78˚C and the boiling point of the solvent used for 5 minutes to 72 hours, under hydrogen atmosphere at ordinary or increased pressure, or in the presence of 1 equivalent to a large excess amount, preferably 2 equivalents to a large excess amount of a suitable hydrogen source.

Examples of the catalyst include palladium carbon, palladium, palladium hydroxide, palladium acetate, palladium black, platinum black, and the like.

Examples of the hydrogen source include formic acid, ammonium formate, sodium formate, and the like.

Examples of the solvent include methanol, ethanol, toluene, ethyl acetate, acetonitrile, diethyl ether, tetrahydrofuran (THF), 1,2-dimethoxyethane (DME), dioxane, N,N-dimethylformamide (DMF), N,N-dimethylacetamide (DMA), N-methylpyrrolidone (NMP), hexane, water, and the like. These solvents may be used alone or as a mixture thereof.

Compound (II) can be obtained as a commercial product, or produced according to the step of Production

Method 1, or similar methods described in, for example, WO2007/002313, WO2001/056607, WO2001/047891, J. Med. Chem., 1970, 13, 1117-1124, Bioorg. Med. Chem. Lett., 2007, 17, 1403-1407, WO2005/035526, WO2003/045313, or the like.

Step 2

Compound (V) can be produced by reacting preferably 1 to 100 equivalents of Compound (IV) relative to Compound (III-1) without a solvent or in a solvent, in the presence of 1 to 100 equivalents of an acid relative to Compound (III-1) as necessary, with 1 to 100 equivalents, preferably 1 to 2 equivalents of a diazotization agent (IV-1) relative to Compound (III-1), at a temperature between -78˚C and the boiling point of the solvent used for 5 minutes to 72 hours, adding Compound (III-1) to the reaction mixture, and reacting in the presence of 1 to 100 equivalents of a base as necessary, at a temperature between -78˚C and the boiling point of the solvent used for 5 minutes to 72 hours.

Examples of the diazotization agent (IV-1) include sodium nitrite, potassium nitrite, methyl nitrite, ethyl nitrite, propyl nitrite, butyl nitrite, isoamyl nitrite, and the like.

Examples of the acid include inorganic acids, such as hydrochloric acid, hydrobromic acid, hydroiodic acid, sulfuric acid, and nitric acid; organic acids, such as acetic acid, trifluoroacetic acid, and formic acid; and the like.

Examples of the base include sodium acetate, sodium carbonate, sodium hydroxide, sodium formate, sulfamide, sodium hydrogensulfate, and the like.

Examples of the solvent include water, methanol, ethanol, acetonitrile, diethyl ether, THF, DME, dioxane, DMF, DMA, NMP, and the like. These solvents may be used alone or as a mixture thereof.

Compound (IV) can be obtained, for example, as a commercial product.

Step 3

Compound (I-A) can be produced by reacting Compound (V) with preferably 1 to 100 equivalents of an oxidant, without a solvent or in a solvent, at a temperature between -78˚C and the boiling point of the solvent used for 5 minutes to 72 hours.

Examples of the oxidant include copper (I) sulfate, copper (II) acetate, copper(II) oxide, chromic acid, manganese sulfate, lithium perchlorate, bis(acetoxy)iodobenzene, bromine, and the like.

Examples of the solvent include water, diethyl ether, DME, THF, 1,4-dioxane, DMF, DMA, dimethyl sulfoxide (DMSO), benzene, toluene, xylene, pyridine, dichloromethane, chloroform, carbon tetrachloride, 1,2-dichloroethane, acetonitrile, acetic acid, ethyl acetate, methyl acetate, methylethylketone, methanol, ethanol, propanol, isopropyl alcohol, butanol, hexane, and the like.

Production Method 2

Among Compounds (I), both of Compound (I-B), in which X, Y, and Z are an oxygen atom, a nitrogen atom, and a carbon atom, respectively, and Compound (I-C), in which X, Y, and Z are a nitrogen atom, an oxygen atom, and a carbon atom, respectively, can be produced according to the following production method.

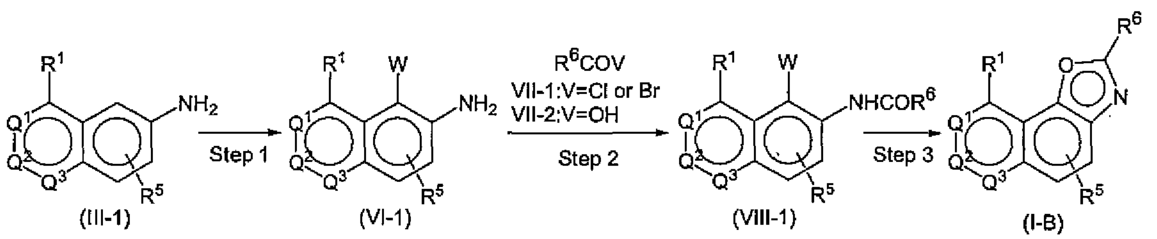


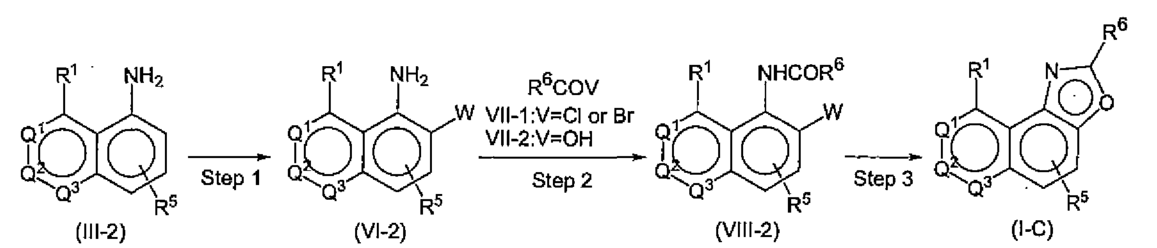

(wherein $R^1$, $R^5$, $R^6$, $Q^1$, $Q^2$, and $Q^3$ have the same meanings as defined above, respectively; V represents a chlorine atom, a bromine atom, or hydroxy; and W represents a chlorine atom, a bromine atom, or an iodine atom)

Step 1

Compound (VI-1) can be produced by reacting Compound (III-1) with 1 to 10 equivalents, preferably 1 equivalent of a halogenating agent, in the presence of preferably 0.1 to 10 equivalents of a suitable additive as necessary, in a solvent at a temperature between -78˚C and the boiling point of the solvent used for 5 minutes to 72 hours.
Examples of the halogenating agent include N-chlorosuccinimide, N-bromosuccinimide, N-iodosuccinimide, bromine, pyrimidium bromide perbromide, tetra-N-butylammonium tribromide, iodine, and the like.
Examples of the solvent include, methanol, ethanol, acetonitrile, dichloromethane, chloroform, 1,2-dichloroethane, toluene, ethyl acetate, diethyl ether, THF, DME, DMF, NMP, and the like. These solvents may be used alone or as a mixture thereof.
Examples of the additive include tetra-N-butylammonium bromide, silver sulfate, and the like.
Compound (VI-2) can be produced from Compound (III-2) in a similar manner to that described above.
Compound (III-1) and Compound (III-2) can be obtained as a commercial product, or produced according to Step 1 of Production Method 1 or a similar method. Alternatively, the compounds can be produced according to, for example, WO2007/002313, WO2001/056607, W02001/047891, J. Med. Chem., 1970, 13, 1117-1124, Bioorg. Med. Chem. Lett., 2007, 17, 1403-1407, WO2005/035526, WO2003/045313, EP581500, or the like.

Step 2

Compound (VIII-1) can be produced by reacting Compound (VI-1) with preferably 1 to 100 equivalents of Compound (VII-1), without a solvent or in a solvent, in the presence of preferably 1 to 100 equivalents of a suitable base, at a temperature between -78˚C and the boiling point of the solvent used for 5 minutes to 72 hours.
Examples of the base include triethylamine, diisopropylethylamine, N,N-4-dimethylaminopyridine, pyridine, 2,6-lutidine, and the like.
Examples of the solvent include water, methanol, ethanol, acetonitrile, dichloromethane, chloroform, 1,2-dichloroethane, toluene, ethyl acetate, diethyl ether, THF, DME, DMF, NMP, and the like. These solvents may be used alone or as a mixture thereof.
Compound (VII-1) can be obtained, for example, as a commercial product.
Compound (VIII-1) can also be produced by the following method.
Compound (VIII-1) can be produced by reacting Compound (VI-1) with preferably 1 to 100 equivalents of Compound (VII-2), without a solvent or in a solvent, in the presence of preferably 1 to 100 equivalents of a condensing agent, and preferably 0.1 to 2 equivalents of a suitable additive as necessary, at a temperature between -78˚C and the boiling point of the solvent used for 5 minutes to 72 hours.
Examples of the condensing agent include dicyclohexylcarbodiimide, 1-ethyl-3-(3-dimethylaminopropyl)carbodiimide hydrochloride, polymer-bound-1-ethyl-3-(3-dimethylaminopropyl)carbodiimide, triphenylphosphine oxide·trifluoromethanesulfonic anhydride, and the like.
Examples of the additive include 1-hydroxybenzotriazole, N-hydroxysuccinimide, and the like.
Examples of the solvent include water, diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methylethylketone, methanol, ethanol, propanol, isopropyl alcohol, butanol, hexane, and the like. These solvents may be used alone or as a mixture thereof.
Compound (VII-2) can be obtained, for example, as a commercial product.
Compound (VIII-2) can be produced using Compound (VI-2) in a similar manner to that described above.

Step 3

Compound (I-B) can be produced by treating Compound (VIII-1) in a solvent, in the presence of preferably 1 to 100 equivalents of a base, at a temperature between -78˚C and the boiling point of the solvent used, with light irradiation using a mercury lamp for 5 minutes to 72 hours.
Examples of the base include lithium hydroxide, sodium hydroxide, potassium hydroxide, sodium carbonate, potassium carbonate, sodium hydrogencarbonate, and the like.
Examples of the solvent include, methanol, ethanol, acetonitrile, dichloromethane, chloroform, 1,2-dichloroethane, toluene, ethyl acetate, diethyl ether, THF, DME, DMF, DMA, water, and the like. These solvents may be used alone or as a mixture thereof.
Compound (I-B) can also be produced by the following method.

Compound (I-B) can be produced by reacting Compound (VIII-1) without a solvent or in a solvent, in the presence of preferably 0.01 to 10 equivalents of a catalyst and preferably 0.01 to 10 equivalents of a ligand, and in the presence of preferably 1 to 100 equivalents of a base as necessary, at a temperature between -78˚C and the boiling point of the solvent used for 5 minutes to 72 hours.

Examples of the catalyst include copper(I) chloride, copper(I) bromide, copper(I) iodide, copper(I) acetate, copper(I) oxide, iron(III) chloride, iron(III) bromide, iron(III) iodide, and the like.

Examples of the ligand include 1,10-phenanthroline, 2,2,6,6-tetramethyl-3,5-heptanedione, bipyridyl, 4-dimethylaminopyridine, N,N,N',N'-tetramethylethylenediamine, and the like.

Examples of the base include lithium hydroxide, sodium hydroxide, potassium hydroxide, sodium carbonate, potassium carbonate, cesium carbonate, sodium hydrogencarbonate, sodium acetate, potassium acetate, potassium phosphate, and the like.

Examples of the solvent include water, diethyl ether, DME, THF, 1,4-dioxane, diglyme, DMF, DMA, DMSO, benzene, toluene, xylene, dichloromethane, chloroform, carbon tetrachloride, 1,2-dichloroethane, acetonitrile, ethyl acetate, methyl acetate, methylethylketone, methanol, ethanol, propanol, isopropyl alcohol, butanol, hexane, and the like. These solvents may be used alone or as a mixture thereof.

Compound (I-C) can be produced using Compound (VIII-2) in a similar manner to that described above.

Production Method 3

Among Compounds (I), Compound (I-D), in which X and Y are different from each other and either a nitrogen atom or NH, and Z is a carbon atom, can be produced according to the following production method.

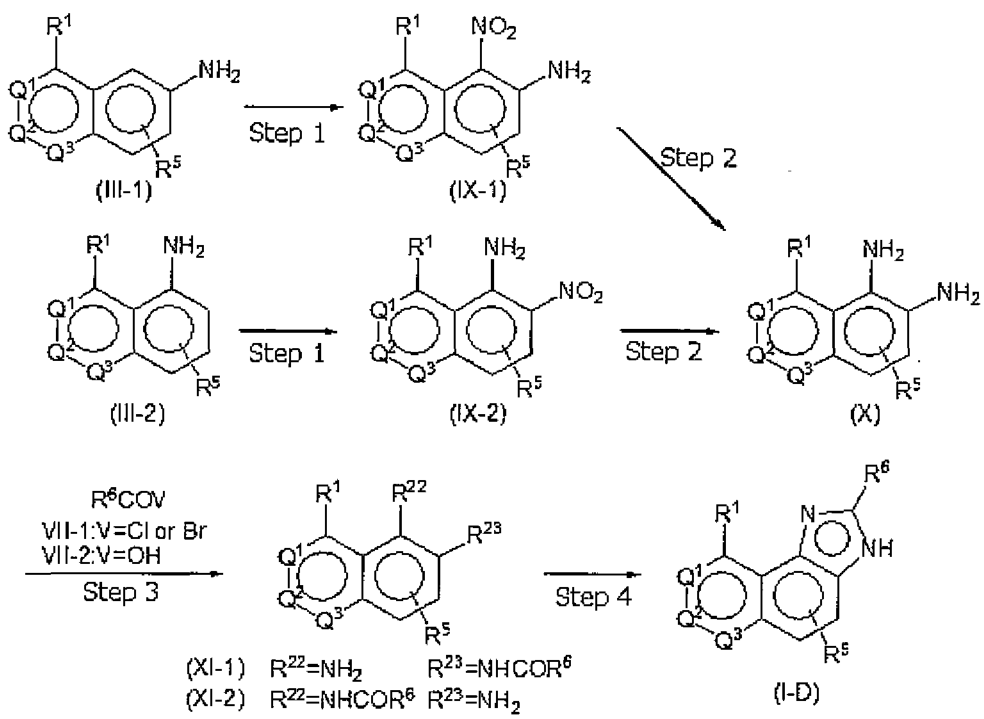


(III-1) (IX-1)

(III-2) (IX-2) (X)

(XI-1) $R^{22}=NH_2$ $R^{23}=NHCOR^6$

(XI-2) $R^{22}=NHCOR^6$ $R^{23}=NH_2$

(I-D)

(wherein $R^1$, $R^5$, $R^6$, V, $Q^1$, $Q^2$ and $Q^3$ have the same meanings as defined above, respectively)

Step 1

Compound (IX-1) can be produced by reacting Compound (III-1) in a solvent, with preferably 1 to 100 equivalents of a nitrating agent, at a temperature between -78˚C and the boiling point used of the solvent for 5 minutes to 72 hours.

Examples of the nitrating agent include nitric acid, sodium nitrate, potassium nitrate, nitrogen dioxide, and the like.

Examples of the solvent include sulfuric acid, acetic acid, trifluoroacetic acid, dichloromethane, hexane, ethyl acetate, acetonitrile, and the like. These solvents may be used alone or as a mixture thereof.

Compound (IX-2) can be produced using Compound (III-2) in a similar manner to that described above.

Compound (III-1) and Compound (III-2) can be obtained as a commercial product, or produced according to Step 1 of Production Method 1 or a similar method. Alternatively, the compounds can be produced according to, for example, WO2007/002313, WO2002/056507, WO2001/047891, J. Med. Chem., 1970, 13, 1117-1124, Bioorg. Med. Chem. Lett., 2007, 17, 1403-1407, W02005/035526, WO2003/045313, EP581500, or the like.

Step 2

Compound (X) can be produced by treating Compound (IX-1) or Compound (IX-2) with 1 to 100 equivalents, preferably 1 to 10 equivalents of a reducing agent, without a solvent or in a solvent, in the presence of preferably 1 equivalent to a large excess amount of an acid, at a temperature between -78˚C and the boiling point of the solvent used for 5 minutes to 72 hours.

Examples of the acid include hydrochloric acid, sulfuric acid, acetic acid, and the like.

Examples of the reducing agent include tin, iron, zinc, and the like.

Examples of the solvent include methanol, ethanol, isopropyl alcohol, water, and the like. These solvents may be used alone or as a mixture thereof.

Compound (X) can also be produced by the following method.

Compound (X) can be produced by treating Compound (IX-1) or Compound (IX-2) without a solvent or in a solvent, in the presence of preferably 0.01 to 50% by weight of a suitable catalyst relative to Compound (IX-1) or Compound (IX-2), at a temperature between -78˚C and the boiling point of the solvent used for 5 minutes to 72 hours, under hydrogen atmosphere at ordinary or increased pressure, or in the presence of 1 equivalent to a large excess amount, preferably 2 equivalents to a large excess amount of a suitable hydrogen source.

Examples of the catalyst include palladium carbon, palladium, palladium hydroxide, palladium acetate, palladium black, platinum black, and the like.

Examples of the hydrogen source include formic acid, ammonium formate, sodium formate, and the like.

Examples of the solvent include methanol, ethanol, toluene, ethyl acetate, acetonitrile, diethyl ether, THF, DME, dioxane, DMF, NMP, hexane, water, and the like. These solvents may be used alone or as a mixture thereof.

Step 3

Compound (XI-1) and/or Compound (XI-2) can be produced by reacting Compound (X) with preferably 1 to 100 equivalents of Compound (VII-1), without a solvent or in a solvent, in the presence of preferably 1 to 100 equivalents of a base, at a temperature between -78˚C and the boiling point of the solvent used for 5 minutes to 72 hours.

Examples of the base include triethylamine, diisopropylethylamine, N,N-4-dimethylaminopyridine, pyridine, 2,6-lutidine, and the like.

Examples of the solvent include water, methanol, ethanol, acetonitrile, dichloromethane, chloroform, 1,2-dichloroethane, toluene, ethyl acetate, diethyl ether, THF, DME, DMF, NMP, and the like. These solvents may be used alone or as a mixture thereof.

Compound (VII-1) can be obtained, for example, as a commercial product.

Compound (XI-1) and/or Compound (XI-2) can also be produced by the following method.

Compound (XI-1) and/or Compound (XI-2) can be produced by reacting Compound (X) with preferably 1 to 100 equivalents of Compound (VII-2), without a solvent or in a solvent, in the presence of preferably 1 to 100 equivalents of a condensing agent, and preferably 0.1 to 2 equivalents of a suitable additive as necessary, at a temperature between -78˚C and the boiling point of the solvent used for 5 minutes to 72 hours.

Examples of the condensing agent include dicyclohexylcarbodiimide, 1-ethyl-3-(3-dimethylaminopropyl)carbodiimide hydrochloride, polymer-bound-1-ethyl-3-(3-dimethylaminopropyl)carbodiimide, triphenylphosphine oxide-trifluoromethanesulfonic anhydride, and the like.

Examples of the additive include 1-hydroxybenzotriazole, N-hydroxysuccinimide, and the like.

Examples of the solvent include water, diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methylethylketone, methanol, ethanol, propanol, isopropyl alcohol, butanol, hexane, and the like. These solvents may be used alone or as a mixture thereof.

Compound (VII-2) can be obtained, for example, as a commercial product.

Step 4

Compound (I-D) can be produced by treating Compound (XI-1) and/or Compound (XI-2) without a solvent or in a solvent, in the presence of preferably 1 to 100 equivalents of a suitable additive, at a temperature between -78°C and the boiling point of the solvent used for 5 minutes to 72 hours.

Examples of the additive include acetic acid, trifluoroacetic acid, formic acid, hydrochloric acid, sulfuric acid, tosic acid, benzenesulfonic acid, phosphorus trichloride, phosphorus oxychloride, lithium hydroxide, sodium hydroxide, potassium hydroxide, and the like.

Examples of the solvent include water, diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methanol, ethanol, propanol, isopropyl alcohol, butanol, hexane, and the like. These solvents may be used alone or as a mixture thereof.

The above Steps 1 to 4 can also be successively performed, without isolating each product, by successively adding the reagents to the reaction solution.

Production Method 4

Among Compounds (I), both Compound (I-E-1) and Compound (I-E-2), in which X and Y are different from each other and either CH or NH, and Z is a carbon atom, can be produced according to the following production method.

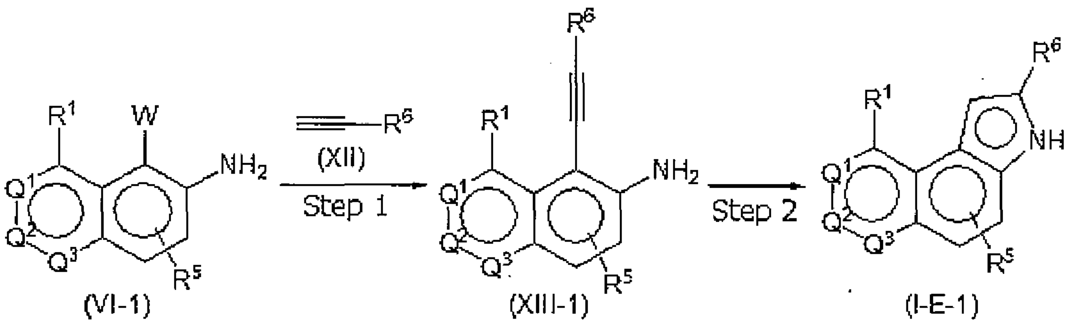


(VI-1) (XIII-1) (I-E-1)

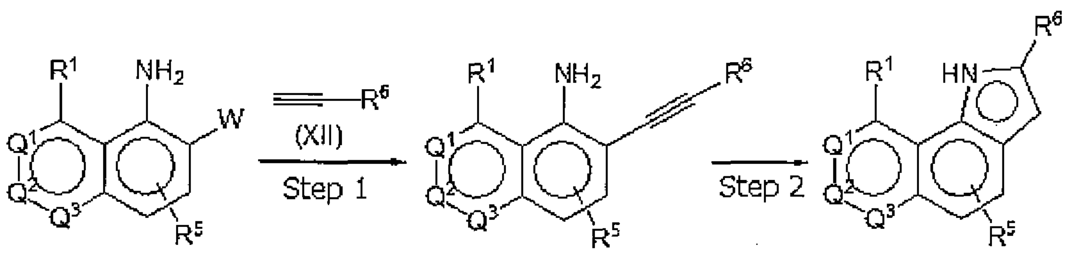


(VI-2) (XIII-2) (I-E-2)

(wherein $R^1$, $R^5$, $R^6$, $Q^1$, $Q^2$, $Q^3$, and W have the same meanings as defined above, respectively)

Step 1

Compound (XIII-1) can be produced by reacting Compound (VI-1) with preferably 1 to 100 equivalents of Compound (XII), without a solvent or in a solvent, in the presence of preferably 0.1 to 10 equivalents of a palladium compound, preferably 0.1 to 10 equivalents of a copper compound, and preferably 0.1 to 10 equivalents of an additive as necessary, at a temperature between room temperature and the boiling point of the solvent used for 5 minutes to 72 hours.

Examples of the palladium compound include [1,1'-bis(diphenylphosphino)ferrocene]palladium(II) chloride, bis(triphenylphosphine)palladium(II) chloride, tetrakis (triphenylphosphine)palladium(0),

[1,2-bis(diphenylphosphino)ethane]palladium(II) chloride, and the like.

Examples of the copper compound include copper (I) iodide, copper(I) chloride, copper (I) bromide, copper (I) acetate,

copper(I) oxide, copper(II) oxide, and the like.

Examples of the additive include cesium carbonate, potassium phosphate, sodium carbonate, potassium carbonate, sodium hydrogencarbonate, sodium hydroxide, lithium chloride, potassium chloride, silver oxide, silver nitrate, silver acetate, sodium ethoxide, triethylamine, and the like.

Examples of the solvent include water, diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methylethylketone, methanol, ethanol, propanol, isopropyl alcohol, butanol, hexane, and the like. These solvents may be used alone or as a mixture thereof.

Compound (XIII-2) can be produced using Compound (VI-2) in a similar manner to that described above.
Both of Compound (VI-1) and Compound (VI-2) can be produced according to Step 1 of Production Method 2.
Compound (XII) can be obtained, for example, as a commercial product.

Step 2

Compound (I-E-1) can be produced by reacting Compound (XIII-1) without a solvent or in a solvent, in the presence of preferably 0.1 to 100 equivalents of a base or a metal reagent, at a temperature between room temperature and the boiling point of the solvent used for 5 minutes to 72 hours.

Examples of the base include lithium hydroxide, sodium hydroxide, potassium hydroxide, potassium tert-butoxide, sodium ethoxide, sodium methoxide, potassium carbonate, cesium carbonate, sodium carbonate, sodium hydride, potassium hydride, butyllithium, tetrabutylammonium fluoride, and the like.

Examples of the metal reagent include copper(I) iodide, copper(II) acetate, copper(II) trifluoroacetate, diethylzinc, zinc bromide, zinc chloride, zinc iodide, palladium(II) chloride, palladium(II) acetate,

[1,1'-bis(diphenylphosphino)ferrocene]palladium(II) chloride, bis(triphenylphosphine)palladium(II) chloride, tetrakis (triphenylphosphine)palladium(0),

[1,2-bis(diphenylphosphino)ethane]palladium(II) chloride, and the like.

Examples of the solvent include water, diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methanol, ethanol, propanol, isopropyl alcohol, butanol, hexane, and the like. These solvents may be used alone or as a mixture thereof.

Compound (I-E-2) can be produced using Compound (XTTI-2) in a similar manner to that described above.

The above Steps 1 and 2 can also be successively performed, without isolating the product, by successively adding the reagents to the reaction solution.

Production Method 5

Among Compounds (1), both of Compound (I-F-1) and Compound (I-F-2), in which X and Y are different from each other and either a sulfur atom or a nitrogen atom, and Z is a carbon atom, can be produced according to the following production method.

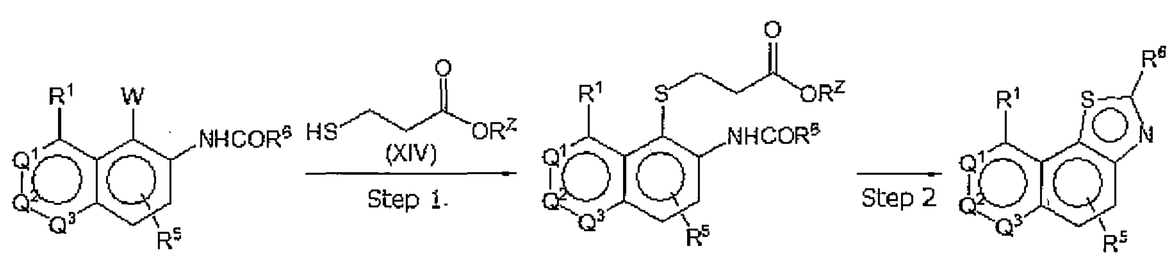


(VIII-1) (XV-1) (I-F-1)

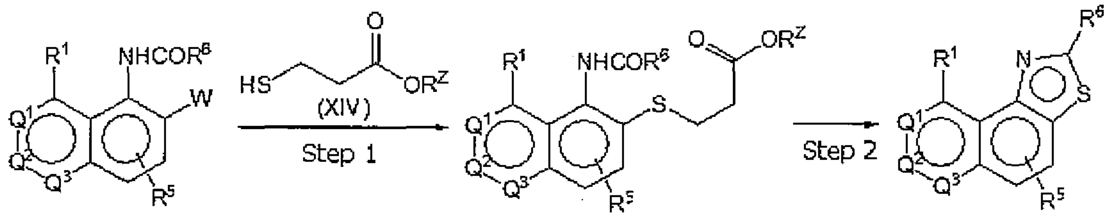


(VIII-2) (XV-2) (I-F-2)

(wherein $R^1$, $R^5$, $R^6$, $Q^1$, $Q^2$, $Q^3$, and W have the same meanings as defined above, respectively; and $R^z$ represents $C_{1-10}$ alkyl, such as methyl and ethyl, or $C_{6-14}$ aryl, such as phenyl)

Step 1

Compound (XV-1) can be produced by reacting Compound (VIII-1) with preferably 1 to 100 equivalents of Compound (XIV), in a solvent, in the presence of preferably 0.01 to 10 equivalents of a palladium compound, preferably 1 to 100 equivalents of a base, and preferably 0.01 to 20 equivalents of a ligand as necessary, at a temperature between -20˚C and the boiling point of the solvent used for 5 minutes to 72 hours.

Examples of the palladium compound include palladium(II) chloride, palladium(II) acetate, [1,1'-bis(diphenylphosphino)ferrocene]palladium(II) chloride, bis(triphenylphosphine)palladium(II) chloride, tetrakis (triphenylphosphine)palladium(0), di(benzylideneacetone)palladium(0), [1,2-bis(diphenylphosphino)ethane]palladium(II) chloride, and the like.

Examples of the ligand include triphenylphosphine, tributylphosphine, xantphos, 1,1'-bis(diphenylphosphino)ferrocene, and the like.
Examples of the base include potassium carbonate, cesium carbonate, potassium phosphate, potassium hydroxide, sodium hydroxide, potassium tert-butoxide, triethylamine, diisopropylethylamine, N-methylmorpholine, pyridine, 1,8-diazabicyclo[5.4.0]undec-7-ene(DBU), and the like.

Examples of the solvent include, methanol, ethanol, dichloromethane, chloroform, 1,2-dichloroethane, toluene, acetonitrile, diethyl ether, THF, DME, dioxane, DMF, DMA, NMP, water, and the like. These solvents may be used alone or as a mixture thereof.
Compound (XV-2) can be produced using Compound (VIII-2) in a similar manner to that described above.
Both of Compound (VIII-1) and Compound (VIII-2) can be produced according to Step 2 of Production Method 2.

Compound (XIV) can be obtained, for example, as a commercial product.

Step 2

Compound (I-F-1) can be produced by treating Compound (XV-1) without a solvent or in a solvent, with preferably 1 equivalent to a large excess amount of a base, at a temperature between -20˚C and the boiling point of the solvent used, and then reacting with preferably 1 equivalent to a large excess amount of an additive at a temperature between -20˚C and the boiling point of the solvent used for 5 minutes to 72 hours.

Examples of the base include lithium hydroxide, sodium hydroxide, potassium hydroxide, potassium tert-butoxide, sodium ethoxide, sodium methoxide, potassium carbonate, cesium carbonate, sodium carbonate, sodium hydride, potassium hydride, butyllithium, and the like.
Examples of the additive include acetic acid, trifluoroacetic acid, formic acid, hydrochloric acid, sulfuric acid, tosic acid, benzenesulfonic acid, phosphorus trichloride, phosphorus oxychloride, and the like.

Examples of the solvent include water, diethyl ether, DME, THF, 1,4-dioxane, DMF, DMA, DMSO, benzene, toluene, xylene, dichloromethane, acetonitrile, methanol, ethanol, propanol, isopropyl alcohol, butanol, hexane, and the like. These solvents may be used alone or as a mixture thereof.
Compound (I-F-2) can be produced using Compound (XV-2) in a similar manner to that described above.

The above Steps 1 and 2 can also be successively performed, without isolating the product, by successively adding the reagents to the reaction solution.

Production Method 6

Among Compounds (I), Compound (I-H-1), in which one or more of $R^2$, $R^3$, $R^4$, and $R^5$ are optionally substituted lower alkenyl, optionally substituted lower alkynyl, optionally substituted aryl, or an optionally substituted aromatic heterocyclic group, can be produced according to the following production method.

Compound (I-H-1) can be produced by reacting, among Compounds (I), Compound (I-G-1), in which one or more of $R^2$, $R^3$, $R^4$, and $R^5$ are a chlorine atom, a bromine atom, an iodine atom, trifluoromethanesulfonyloxy, methanesulfonyloxy, or p-toluenesulfonyloxy, with preferably 1 to 10 equivalents of Compound (XVI-1) represented by the following formula:

$$(R^A)_pM^1—R^{26} \qquad \text{(XVI-1)}$$

[wherein $R^A$ represents a fluorine atom, a chlorine atom, a bromine atom, an iodine atom, hydroxy, $C_{1-6}$ alkyl, $C_{1-6}$ alkoxy, aryl, or aryloxy; p represents an integer of 2 or 3; $R^{26}$ represents, among the definitions of $R^2$, $R^3$, and $R^4$, optionally substituted lower alkenyl, optionally substituted lower alkynyl, optionally substituted aryl, or an optionally substituted aromatic heterocyclic group; and $M^1$ represents, a tin atom, a boron atom, or a silicon atom (when $M^1$ is a boron atom, $(R^A)_pM^1$ may represent 4,4,5,5-tetramethyl-1,3,2-dioxaborolan-2-yl)],
without a solvent or in a solvent, in the presence of a catalytic amount of a palladium compound, and preferably 0.1 to 10 equivalents of an additive as necessary, at a temperature between 0˚C and the boiling point of the solvent used for 5 minutes to 72 hours.

Examples of the additive include cesium carbonate, potassium phosphate, sodium carbonate, potassium carbonate, sodium hydrogencarbonate, sodium hydroxide, lithium chloride, potassium chloride, sodium ethoxide, triethylamine, silver oxide, silver nitrate, silver acetate, and the like.
Examples of the solvent include water, diethyl ether, DME, THF, 1,4-dioxane, DMF, DMA, DMSO, benzene, toluene, xylene, acetonitrile, ethyl acetate, methyl acetate, methylethylketone, methanol, ethanol, propanol, isopropyl alcohol, butanol, hexane, and the like. These solvents may be used alone or as a mixture thereof.

Examples of the palladium compound include
[1,1'-bis(diphenylphosphino)ferrocene]palladium(II) chloride, bis(triphenylphosphine)palladium(II) chloride, tetrakis (triphenylphosphine)palladium(0),
[1,2-bis(diphenylphosphino)ethane]palladium(II) chloride, and the like.
Among Compounds (I), Compound (I-H-2), in which one or more of $R^2$, $R^3$, $R^4$, and $R^5$ are optionally substituted lower alkynyl, can also be produced, according to the following production method.

Compound (I-H-2) can be produced by reacting Compound (I-G-1) with preferably 1 to 100 equivalents of an alkynyl compound (XVI-2), without a solvent or in a solvent, in the presence of a catalytic amount of a palladium compound, a catalytic amount of a copper compound, and preferably 0.1 to 20 equivalents of an additive as necessary, at a temperature between 0˚C and the boiling point of the solvent used for 5 minutes to 72 hours.
Examples of the alkynyl compound (XVI-2) include the compound represented by the following formula (XVI-2):

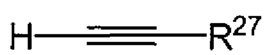

(XVI-2)

(wherein the moiety of

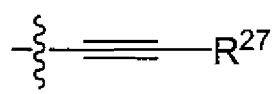

represents, among the definitions of $R^2$, $R^3$, and $R^4$, optionally substituted lower alkynyl), and the like, and this compound can be obtained, for example, as a commercial product.
Examples of the additive include cesium carbonate, potassium phosphate, sodium carbonate, potassium carbonate, sodium hydrogencarbonate, sodium hydroxide, sodium ethoxide, triethylamine, lithium chloride, potassium chloride, silver oxide, silver nitrate, silver acetate, and the like.

Examples of the solvent include water, diethyl ether, DME, THF, 1,4-dioxane, DMF, DMA, DMSO, benzene, toluene, xylene, acetonitrile, ethyl acetate, methyl acetate, methylethylketone, methanol, ethanol, propanol, isopropyl alcohol, butanol, hexane, and the like. These solvents may be used alone or as a mixture thereof.
Examples of the palladium compound include

[1,1'-bis(diphenylphosphino)ferrocene]palladium(II) chloride, bis(triphenylphosphine)palladium(II) chloride, tetrakis (triphenylphosphine)palladium(0),
[1,2-bis(diphenylphosphino)ethane]palladium(II) chloride, and the like.

Examples of the copper compound include copper (I) iodide, copper(I) chloride, copper(I) bromide, copper(I) acetate, copper(I) oxide, copper(II) oxide, and the like.
Compound (I-G-1) can be produced according to one of Production Methods 1 to 5.

Production Method 7

Among Compounds (I), Compound (I - I), in which one or more of $R^2$, $R^3$, and $R^4$ are optionally substituted lower alkylsulfanyl, -$OR^{33}$ (wherein $R^{33}$ represents optionally substituted lower alkyl, optionally substituted cycloalkyl, optionally substituted aryl, an optionally substituted aromatic heterocyclic group, or an optionally substituted aliphatic heterocyclic group), or -$NR^{34}R^{35}$ (wherein $R^{34}$ and $R^{35}$ may be the same or different and each represent a hydrogen atom, optionally substituted lower alkyl, optionally substituted cycloalkyl, optionally substituted aryl, an optionally substituted aromatic heterocyclic group, or an optionally substituted aliphatic heterocyclic group, or $R^8$ and $R^9$ are combined together with the adjacent nitrogen atom to form an optionally substituted nitrogen-containing heterocyclic group), can also be produced, according to the following production method.

Compound (I-I) can be produced by reacting Compound (I-G-2), in which one or more of $R^2$, $R^3$, and $R^4$ are a chlorine atom, a bromine atom, an iodine atom, trifluoromethanesulfonyloxy, methanesulfonyloxy, or p-toluenesulfonyloxy, with preferably 1 to 100 equivalents of Compound (XVII) represented by the following formula:

$$H—R^{28} \qquad (XVII)$$

[wherein $R^{28}$ represents, among the definitions of $R^2$, $R^3$, and $R^4$, optionally substituted lower alkylsulfanyl, -$OR^{33}$ (wherein $R^{33}$ has the same meaning as defined above), or -$NR^{34}R^{35}$
(wherein $R^{34}$ and $R^{35}$ have the same meanings as defined above)], without a solvent or in a solvent, in the presence of 1 to 100 equivalents, preferably 1 to 10 equivalents of a base, at a temperature between -78°C and the boiling point of the solvent used for 5 minutes to 96 hours.
Examples of the base include sodium hydride, lithium hydride, lithium hydroxide, sodium hydroxide, potassium hydroxide, sodium carbonate, potassium carbonate, cesium carbonate, sodium hydrogencarbonate, n-butyllithium, sec-butyllithium, and the like.

Examples of the solvent include water, diethyl ether, diisopropyl ether, DME, THF, 1,4-dioxane, DMF, DMA, DMSO, benzene, toluene, xylene, acetonitrile, ethyl acetate, methyl acetate, methylethylketone, methanol, ethanol, propanol, isopropyl alcohol, butanol, hexane, and the like. These solvents may be used alone or as a mixture thereof.

Compound (XVII) can be obtained, for example, as a commercial product.
Compound (I-G-2) can be produced according to one of Production Methods 1 to 6.

Production Method 8

Among Compounds (1), Compound (I-K), in which $R^1$ is carboxy, can also be produced according to the following production method.

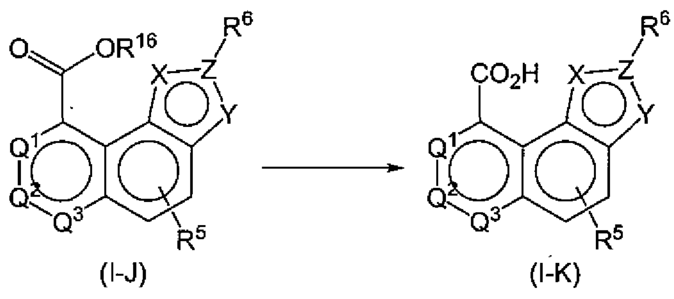

(wherein $R^5$, $R^6$, $R^{16}$, $Q^1$, $Q^2$, $Q^3$, X, Y, and Z have the same meanings as defined above, respectively)
Compound (I-K) can be produced by treating Compound (I-J) with preferably 1 equivalent to a large excess amount of a base, in a solvent at a temperature between 0°C and the boiling point of the solvent used for 5 minutes to 72 hours.

Examples of the base include potassium carbonate, lithium hydroxide, potassium hydroxide, sodium hydroxide, sodium methoxide, and the like.

Examples of the solvent include an aqueous solvent. Examples of such a solvent include methanol, ethanol, dichloromethane, chloroform, 1,2-dichloroethane, toluene, ethyl acetate, acetonitrile, diethyl ether, THF, DME, dioxane, DMF, DMA, NMP, pyridine, and the like. These solvents are used as a mixture with water. Alternatively, these solvents may be mixed with each other and then used as a mixture with water.

Compound (I-J) can be produced according to one of Production Methods 1 to 7.

Production Method 9

Among Compounds (I), Compound (I-L), in which $R^1$ is represented by $-C(=O)NR^{17}R^{18}$ (wherein $R^{17}$ and $R^{18}$ have the same meanings as defined above, respectively), can also be produced according to the following production method.

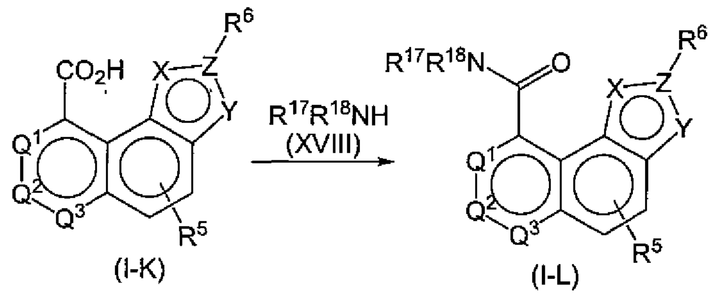


(I-K) (I-L)

(wherein $R^5$, $R^6$, $R^{17}$, $R^{18}$, $Q^1$, $Q^2$, $Q^3$, X, Y, and Z have the same meanings as defined above, respectively) Compound (I-L) can be produced by reacting Compound (I-K), which can be obtained according to Production Method 8 or the like, with preferably 1 to 100 equivalents of Compound (XVIII), without a solvent or in a solvent, in the presence of preferably 0.1 to 10 equivalents of a condensing agent, and preferably 0.1 to 2 equivalents of an additive as necessary, at a temperature between -78°C and the boiling point of the solvent used for 5 minutes to 72 hours.

Examples of the condensing agent include carbonyldiimidazole, dicyclohexylcarbodiimide, 1-ethyl-3-(3-dimethylaminopropyl)carbodiimide hydrochloride, polymer-bound-1-ethyl-3-(3-dimethylaminopropyl)carbodiimide, triphenylphosphine oxide·trifluoromethanesulfonic anhydride, and the like.

Examples of the additive include DBU, N,N-4-dimethylaminopyridine, 1-hydroxybenzotriazole, N-hydroxysuccinimide, and the like.

Examples of the solvent include water, diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methylethylketone, methanol, ethanol, propanol, isopropyl alcohol, butanol, hexane, and the like. These solvents may be used alone or as a mixture thereof.

Compound (XVIII) can be obtained, for example, as a commercial product.

Production Method 10

Among Compounds (I), Compound (I-M), in which one or more of $R^2$, $R^3$, $R^4$, and $R^5$ are pyrrolyl of which the nitrogen atom has an optionally substituted $C_{1-10}$ alkyl (examples of the substituents of the optionally substituted $C_{1-10}$ alkyl include the groups illustrated in the above (i) as examples of the substituents of the optionally substituted lower alkyl) as a substituent, can also be produced according to the following production method.

Compound (I-M) can be produced by reacting, among Compounds (I), Compound (I-H-1), in which one or more of $R^2$, $R^3$, and $R^4$ are pyrrolyl of which the nitrogen atom is unsubstituted, with preferably 1 to 100 equivalents of Compound (XIX) represented by the following formula:

$$X^1—R^{29} \qquad (XIX)$$

(wherein $R^{29}$ represents the optionally substituted $C_{1-10}$. alkyl moiety of the above-mentioned pyrrolyl of which the nitrogen atom has an optionally substituted $C_{1-10}$ alkyl; and $X^1$ represents a chlorine atom, a bromine atom, an iodine atom, trifluoromethanesulfonyloxy, methanesulfonyloxy, or p-toluenesulfonyloxy),

without a solvent or in a solvent, in the presence of preferably 1 to 100 equivalents of a base, at a temperature between -78˚C and the boiling point of the solvent used for 5 minutes to 72 hours.

Examples of the base include triethylamine, diisopropylethylamine, pyridine, N,N-4-dimethylaminopyridine, 2,6-lutidine, 1,8-bis(dimethylamino)naphthalene, sodium hydride, potassium hydride, and the like.
Examples of the solvent include diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methylethylketone, hexane, and the like. These solvents may be used alone or as a mixture thereof.

Compound (XIX) can be obtained, for example, as a commercial product.
Compound (I-H-1) can be produced according to Production Method 6, or the like.

Production Method 11

Among Compounds (I), Compound (I-N), in which one or more of $R^2$, $R^3$, $R^4$, and $R^5$ are substituted triazolyl (the substituent of the substituted triazolyl is one of the groups illustrated in the above (ii) as examples of the substituents of the optionally substituted aromatic heterocyclic group), can also be produced according to the following production method.

Compound (I-N) can be produced by reacting Compound (I-H-2), which can be obtained according to Production Method 6, with preferably 1 to 100 equivalents of Compound (XX) represented by the following formula:

$R^{30}—N_3$ (XX)

(wherein $R^{30}$ represents the substituent of the above-mentioned substituted triazolyl),
without a solvent or in a solvent, in the presence of preferably 0.1 to 20 equivalents of a copper compound and preferably 1 to 100 equivalents of a base, at a temperature between -78˚C and the boiling point of the solvent used for 5 minutes to 72 hours.
Examples of the copper compound include copper (I) chloride, copper(I) bromide, copper(I) iodide, copper(I) acetate, copper(I) trifluoroacetate, copper(I) oxide, copper(0), copper(I) sulfate, and the like.
Examples of the base include triethylamine, diisopropylethylamine, 2,6-lutidine, pyridine, 2,2'-bipyridyl, sodium carbonate, potassium carbonate, sodium hydrogencarbonate, sodium hydroxide, lithium hydroxide, potassium hydroxide, sodium ascorbate, and the like.

Examples of the solvent include benzene, toluene, xylene, THF, diethyl ether, diisopropyl ether, DME, 1,4-dioxane, dichloromethane, methanol, ethanol, isopropyl alcohol, tert-butanol, acetonitrile, acetone, water, DMF, DMA, DMSO, and the like. These solvents may be used alone or as a mixture thereof.
Compound (I-H-2) can be produced according to Production Method 6.

Compound (XX) can be obtained as a commercial product, or produced according to, for example, the method described in Jikken Kagaku Kouza, 5th edition, No. 14, Chapter 7, 480-495.

Production Method 12

Among Compounds (I), Compound (I-P), in which one or more of $R^2$, $R^3$, $R^4$, and $R^5$ are cyano, can also be produced, according to the following production method.

Compound (I-P) can be produced by reacting, among Compounds (I), Compound (I-G-1), in which one or more of $R^2$, $R^3$, $R^4$, and $R^5$ are a chlorine atom, a bromine atom, an iodine atom, trifluoromethanesulfonyloxy, methanesulfonyloxy, or p-toluenesulfonyloxy, with preferably 1 to 100 equivalents of a cyanide (XXI), in a solvent, in the presence of preferably 0.1 to 10 equivalents of a palladium compound, preferably 0.1 to 20 equivalents of a phosphine compound, and preferably 1 to 100 equivalents of zinc, at a temperature between 0˚C and the boiling point of the solvent used for 5 minutes to 72 hours.

Examples of the cyanide (XXI) include zinc cyanide, potassium cyanide, sodium cyanide, trimethylsilyl cyanide, acetone cyanohydrin, potassium hexacyanoferrate(II), sodium hexacyanoferrate(II), potassium hexacyanoferrate(III), and the like.
Examples of the palladium compound include palladium acetate, palladium trifluoroacetate, tris(dibenzylidene acetone) dipalladium or a chloroform adduct thereof, tetrakis(triphenylphosphine)palladium,
[1,1,'-bis(diphenylphosphino)ferrocene]palladium(II) chloride dichloromethane adduct, and the like.

Examples of the phosphine compound include
2,2'-bis(diphenylphosphino)-1,1'-binaphthyl,
o-tolylphosphine, tributylphosphine,
di-tert-butyldiphenylphosphine,

2--(di-tert-butylphosphino)biphenyl,
2-(dicyclohexylphosphino)biphenyl,
1,1'-bis(diphenylphosphino)ferrocene, and the like.
Examples of the solvent include toluene, xylene, ethyl acetate, acetonitrile, diethyl ether, THF, DME, dioxane, DMF, NMP, and the like. These, solvents may be used alone or as a mixture thereof.

Production Method 13

Among Compounds (I), Compound (I-Q), in which $R^1$ is 1H-tetrazol-5-yl, can also be produced according to the following production method.
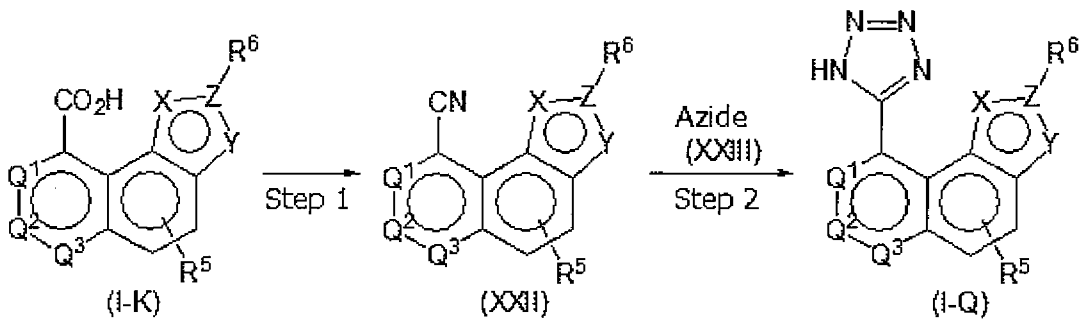


(wherein $R^5$, $R^6$, $Q^1$, $Q^2$, $Q^3$, X, Y, and Z have the same meanings as defined above, respectively)

Step 1

Compound (XXII) can be produced by reacting Compound (I-K), which can be obtained according to Production Method 8, with preferably 1 to 100 equivalents of an ammonia source, in a solvent, in the presence of preferably 1 to 100 equivalents of an additive, at a temperature between 0˚C and the boiling point used for 5 minutes to 72 hours.
Examples of the ammonia source include ammonia, ammonium chloride, ammonium carbonate, and the like. Examples of the additive include thionyl chloride, phosphorus trichloride, phosphorus pentachloride, oxalyl chloride, and the like.
Examples of the solvent include diethyl ether, DME, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methylethylketone, hexane, and the like. These solvents may be used alone or as a mixture thereof.

Step 2

The compound (I-Q) can be produced by reacting Compound (XXII) with preferably 1 to 10 equivalents of an azide (XXIII) in a solvent, in the presence of preferably 1 equivalent to a large excess amount of a weak acid or preferably 0.01 to 10 equivalents of an additive, at a temperature between -20˚C and the boiling point of the solvent used for 5 minutes to 120 hours.
Examples of the azide (XXIII) include sodium azide, potassium azide, and the like.
Examples of the weak acid include ammonium chloride, triethylamine hydrochloride, and the like.
Examples of the additive include tributyltin chloride, trimethyltin chloride, dibutyltin oxide, and the like.
Examples of the solvent include DMF, DMA, NMP, DMSO, and the like. These solvents may be used alone or as a mixture thereof.

Production Method 14

Among Compounds (I), Compound (I-R), in which $R^1$ is 1,2,4-oxadiazol-5 (4H) -on-3-yl, can also be produced according to the following production method.
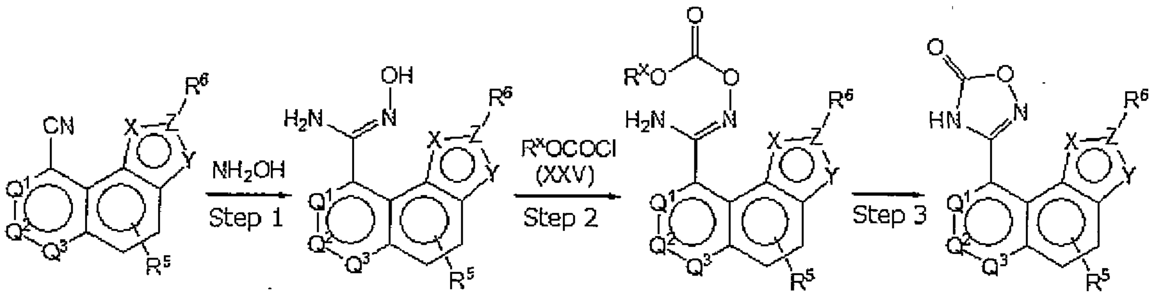


(XXII) (XXIV) (XXVI) (I-R)

(wherein $R^5$, $R^6$, $Q^1$, $Q^2$, $Q^3$, X, Y, and Z have the same meanings as defined above, respectively; and $R^X$ represents $C_{1-10}$ alkyl, such as methyl and ethyl, or $C_{6-14}$ aryl, such as phenyl)

Step 1

Compound (XXIV) can be produced by reacting Compound (XXII), which can be obtained according to Step 1 of Production Method 13 or the like, with preferably 1 equivalent to a large excess amount of hydroxylamine, without a solvent or in a solvent, at a temperature between -20˚C and the boiling point of the solvent used for 5 minutes to 120 hours.

Examples of the solvent include methanol, ethanol, DMF, DMA, DMSO, and the like. These solvents may be used alone or as a mixture thereof.

Step 2

The compound (XXVI) can be produced by reacting Compound (XXIV) with preferably 1 equivalent to a large excess amount of chlorocarbonic ester (XXV) in a solvent, in the presence of preferably 1 equivalent to a large excess amount of a base, at a temperature between -20˚C and the boiling point of the solvent used for 5 minutes to 72 hours.

Examples of the chlorocarbonic ester (XXV) include methyl chlorocarbonate, ethyl chlorocarbonate, propyl chlorocarbonate, phenyl chlorocarbonate, and the like.

Examples of the base include triethylamine, pyridine, 4-dimethylaminopyridine, sodium hydroxide, sodium hydride, potassium tert-butoxide, sodium methoxide, and the like.

Examples of the solvent include THF, DMF, DMA, toluene, xylene, and the like. These solvents may be used alone or as a mixture thereof.

Step 3

Compound (I-R) can be produced by reacting Compound (XXVI) in the presence of preferably a catalytic amount to 10 equivalents of a base as necessary, in a solvent, at a temperature between -20˚C and the boiling point of the solvent used for 5 minutes to 72 hours.

Examples of the base include sodium hydroxide, sodium hydride, potassium tert-butoxide, sodium methoxide, and the like.

Examples of the solvent include THF, DMF, DMA, toluene, xylene, and the like. These solvents may be used alone or as a mixture thereof.

The above Steps 1 to 3 can also be successively performed, without isolating each product, by successively adding the reagents to the reaction solution.

Production Method 15

Among Compounds (I), Compound (I-S), in which $R^1$ is 1,2,4-oxadiazole-5(4H)-thion-3-yl, can also be produced according to the following production method.

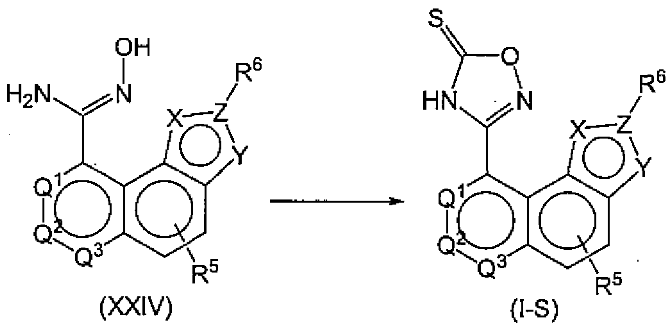

(XXIV) (I-S)

(wherein $R^5$, $R^6$, $Q^1$, $Q^2$, $Q^3$, X, Y, and Z have the same meanings as defined above, respectively)
The compound (I-S) can be produced by reacting Compound (XXIV), which can be obtained according to Step 1 of Production Method 14, with preferably 1 equivalent to a large excess amount of N,N'-thiocarbonyldiimidazole, in a solvent, in the presence of preferably 1 equivalent to a large excess amount of a base, at a temperature between -20˚C and the boiling point of the solvent used for 5 minutes to 72 hours.

Examples of the base include triethylamine, pyridine, 4-dimethylaminopyridine, DBU, and the like.
Examples of the solvent include THF, 1,4-dioxane, dichloromethane, chloroform, acetonitrile, acetone, and the like. These solvents may be used alone or as a mixture thereof.

Production Method 16

Among Compounds (I), Compound (I-T), in which $R^1$ is 1,2,4-thiadiazol-5(4H)-on-3-yl, can also be produced according to the following production method.

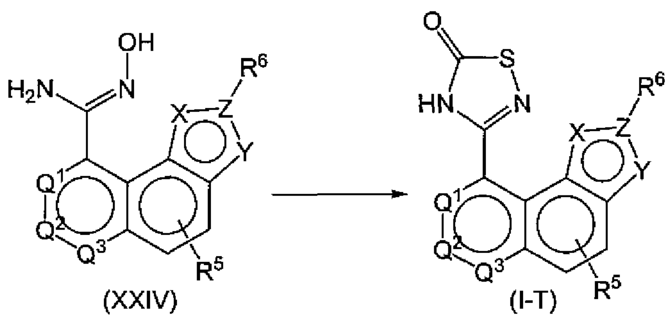

(XXIV) (I-T)

(wherein $R^5$, $R^6$, $Q^1$, $Q^2$, $Q^3$, X, Y, and Z have the same meanings as defined above, respectively.)
The compound (I-T) can be produced by reacting Compound (XXIV), which can be obtained according to Step 1 of Production Method 14, with preferably 1 equivalent to a large excess amount of N,N'-thiocarbonyldiimidazole, in a solvent, in the presence of preferably 1 equivalent to a large excess amount of a Lewis acid, at a temperature between -20˚C and the boiling point of the solvent used for 5 minutes to 72 hours.

Examples of the Lewis acid include a boron trifluoride diethylether complex, stannous chloride, zinc chloride, silica gel, and the like.
Examples of the solvent include THF, 1,4-dioxane, dichloromethane, chloroform, methanol, ethanol, and the like. These solvents may be used alone or as a mixture thereof.
Transformation of a functional group contained in $R^1$ to $R^{22}$ of Compound (I) and $R^{1A}$ to $R^{22A}$ of Compound (I-A) can also be carried out by a known method [for example, a method described in Comprehensive Organic Transformations 2nd edition, R. C. Larock, Vch Verlagsgesellschaft Mbh (1999), or the like] or a similar manner to that.

Compound (I) can also be obtained as a commercial product. For example, Compound 133 can be purchased from AsInEx (JSU code: JSU-0025748).
The intermediate and the desired compound in the above-mentioned respective Production Methods can be isolated and purified by a separation and purification method commonly used in organic synthetic chemistry, for example, filtration, extraction, washing, drying, concentration, recrystallization, various types of chromatography, and the like. Further, the intermediate can be subjected to the subsequent reaction without any particular purification.

In Compound (I), geometric isomer, stereoisomer such as optical isomer, tautomer, and the like may be present.

All possible isomers including these and mixtures thereof can be used or are included in the present invention.
In the case where a salt of Compound (I) is desired, and when Compound (I) is obtained in the form of a salt, the salt may be simply purified as it is or when Compound (I) is obtained in a free form, the compound, after dissolved or suspended in a suitable solvent, may be allowed to form a salt by addition of an acid or a base, and then, the resulting salt may be isolated and purified.

Compounds (I) and pharmaceutically acceptable salts thereof may exist in the form of adducts with water or one of various solvents, and these adducts can also be used or are included in the present invention.
Specific examples of Compounds (I) obtained by the present invention are shown in Tables 2 to 12. However, the compounds of the invention are not limited to these. In the tables, Ph represents phenyl, Me represents methyl, Et represents ethyl, BOC represents tert-butoxycarbonyl, Ac represents acetyl, Bn represents benzyl, and Bz represents benzoyl.

Table 2

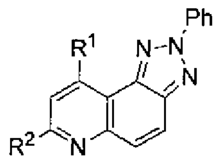

| Compound No. | $R^1$ | $R^2$ | Compound No. | $R^1$ | $R^2$ |
|---|---|---|---|---|---|
| 1 | $CO_2Me$ | 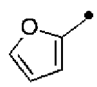 | 11 | $CO_2H$ | 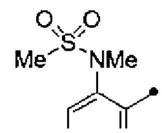 |
| 2 | CONHMe | 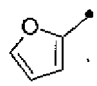 | | | |
| 3 | $CONMe_2$ | 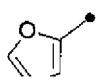 | 12 | $CO_2Me$ | 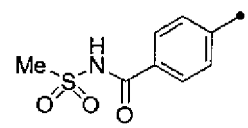 |
| 4 | $CO_2Me$ | 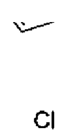 | 13 | $CO_2H$ | 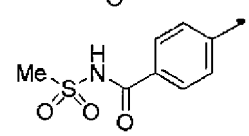 |
| 5 | $CO_2Me$ | 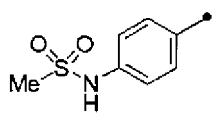 | 14 | $CO_2Me$ | 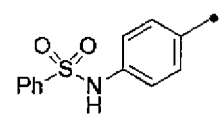 |
| 6 | $CO_2H$ | 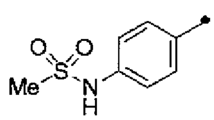 | 15 | $CO_2H$ | 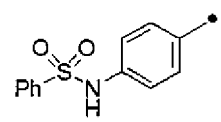 |
| 7 | $CO_2Me$ | 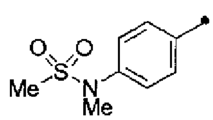 | 16 | $CO_2Me$ | 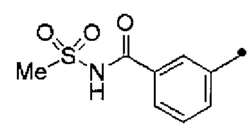 |
| 8 | $CO_2H$ | 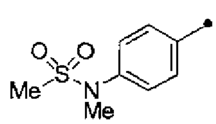 | 17 | $CO_2H$ | 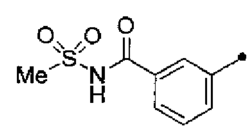 |

(continued)

| Compound No. | $R^1$ | $R^2$ | Compound No. | $R^1$ | $R^2$ |
|---|---|---|---|---|---|
| 9 | 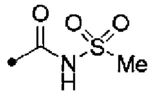 | 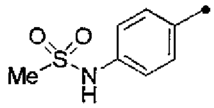 | 18 | $CO_2H$ | EtO |
| | | | 19 | $CO_2H$ | Cl |
| 10 | $CO_2Me$ | 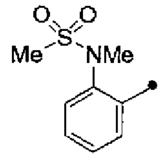 | 20 | $CO_2H$ | 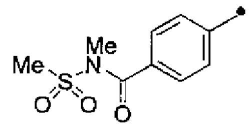 |

Table 3

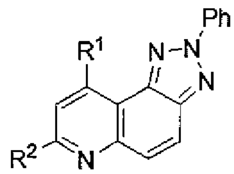

| Compound No. | $R^1$ | $R^2$ | Compound No. | $R^1$ | $R^2$ |
|---|---|---|---|---|---|
| 21 | $CO_2Me$ | 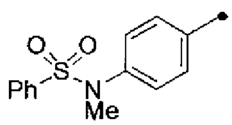 | 28 | $CO_2H$ | 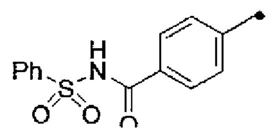 |
| 22 | $CO_2H$ | 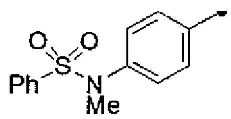 | 29 | $CO_2H$ | 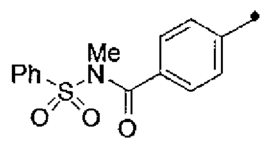 |
| 23 | $CO_2H$ | 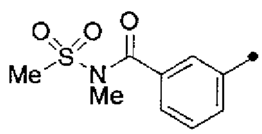 | 30 | $CO_2Me$ | 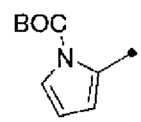 |
| 24 | $CO_2H$ | 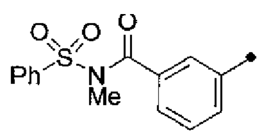 | 31 | $CO_2Me$ | 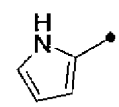 |
| 25 | $CO_2Me$ | 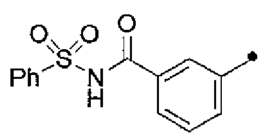 | 32 | $CO_2H$ | 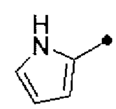 |
| 26 | $CO_2H$ | 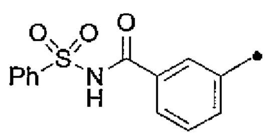 | 33 | $CO_2Me$ | 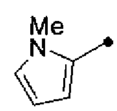 |
| 27 | $CO_2Me$ | 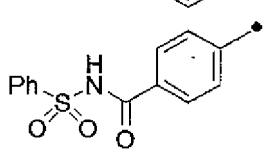 | 34 | $CO_2H$ | 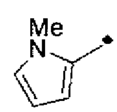 |

Tablet 4

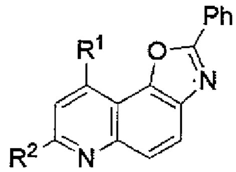

| Compound No. | $R^1$ | $R^2$ | Compound No. | $R^1$ | $R^2$ |
|---|---|---|---|---|---|
| 35 | $CO_2H$ | Cl | 46 | $CO_2H$ | 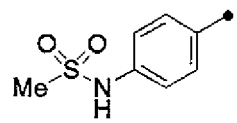 |
| 36 | $CO_2Me$ | Cl | 47 | $CO_2H$ | 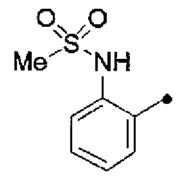 |
| 37 | $CO_2Me$ | 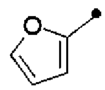 | | | |
| 38 | $CO_2H$ | 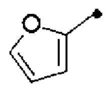 | 48 | $CO_2H$ | 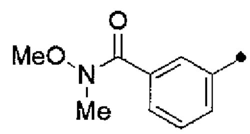 |
| 39 | $CO_2Me$ | Ph | 49 | $CO_2H$ | 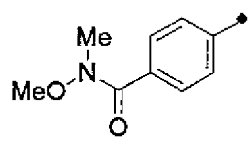 |
| 40 | $CO_2H$ | Ph | | | |
| 41 | $CO_2Me$ | 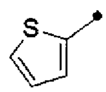 | 50 | $CO_2H$ | 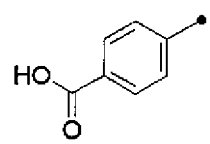 |
| 42 | $CO_2H$ | 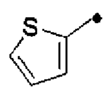 | 51 | $CO_2H$ | 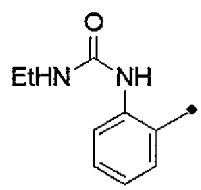 |
| 43 | $CO_2H$ | 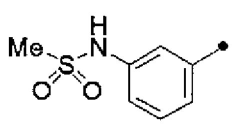 | | | |
| 44 | $CO_2Me$ | 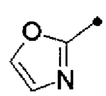 | 52 | $CO_2H$ | 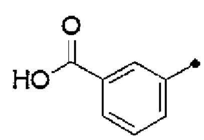 |
| 45 | $CO_2H$ | 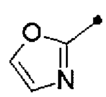 | 53 | $CO_2H$ | 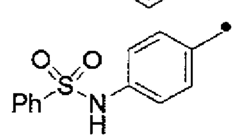 |

Table 5

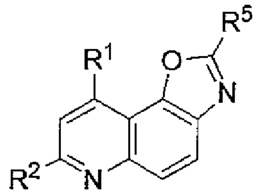

| Compound No. | $R^1$ | $R^2$ | $R^5$ |
|---|---|---|---|
| 54 | $CO_2H$ | Cl | 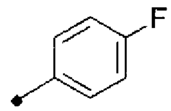 |
| 55 | $CO_2H$ | 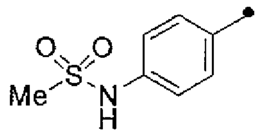 | 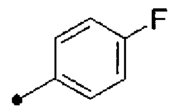 |
| 56 | $CO_2H$ | Cl | 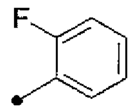 |
| 57 | $CO_2H$ | 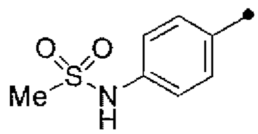 | 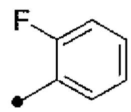 |
| 58 | $CO_2H$ | Cl | 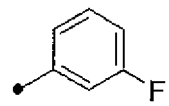 |
| 59 | $CO_2H$ | 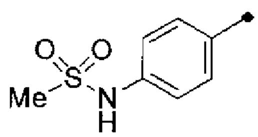 | 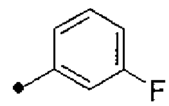 |
| 60 | $CO_2Me$ | Cl | 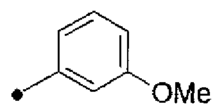 |
| 61 | $CO_2Me$ | 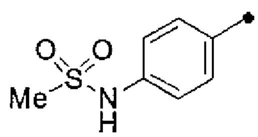 | 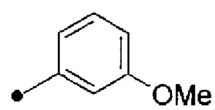 |
| 62 | $CO_2H$ | 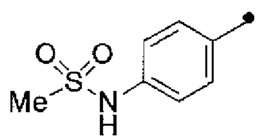 | 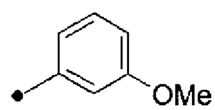 |
| 63 | $CO_2H$ | Cl | 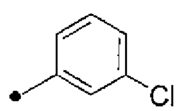 |

Table 6

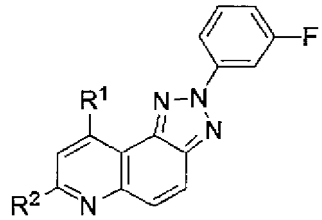

| Compound No. | $R^1$ | $R^2$ |
|---|---|---|
| 64 | $CO_2Me$ | Cl |
| 65 | $CO_2Me$ | 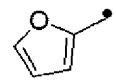 |
| 66 | $CO_2H$ | 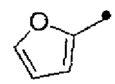 |
| 67 | $CO_2Me$ | 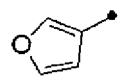 |
| 68 | $CO_2H$ | 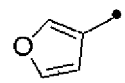 |
| 69 | $CO_2Me$ | 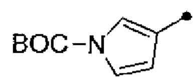 |
| 70 | $CO_2Me$ | 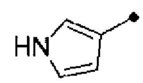 |
| 71 | $CO_2H$ | 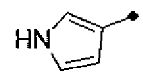 |
| 72 | $CO_2Me$ | 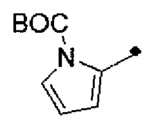 |
| 73 | $CO_2Me$ | 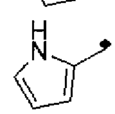 |
| 74 | $CO_2H$ | 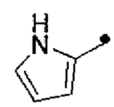 |
| 75 | $CO_2H$ | Cl |

Table 7

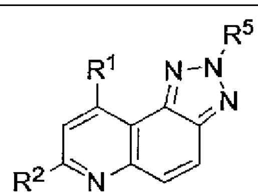

| Compound No. | $R^1$ | $R^2$ | $R^5$ |
|---|---|---|---|
| 76 | $CO_2H$ | 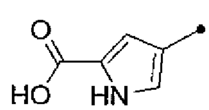 | 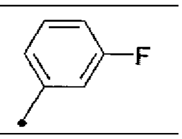 |

(continued)

| Compound No. | $R^1$ | $R^2$ | $R^5$ |
|---|---|---|---|
| 77 | $CO_2Me$ | 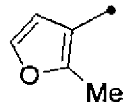 | 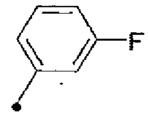 |
| 78 | $CO_2H$ | 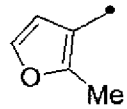 | 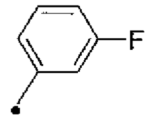 |
| 79 | $CO_2Me$ | CN | 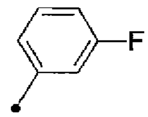 |
| 80 | $CO_2H$ | CN | 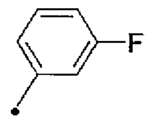 |
| 81 | $CO_2Me$ | Cl | 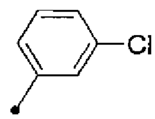 |
| 82 | $CO_2Me$ | 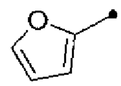 | 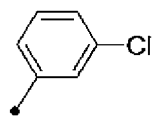 |
| 83 | $CO_2H$ | 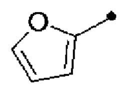 | 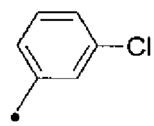 |
| 84 | $CO_2H$ | 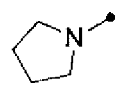 | 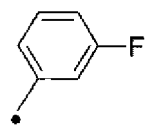 |
| 85 | $CO_2Me$ | 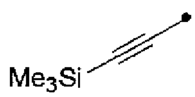 | 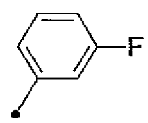 |
| 86 | $CO_2Me$ | 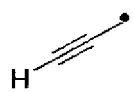 | 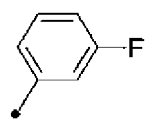 |

Table 8

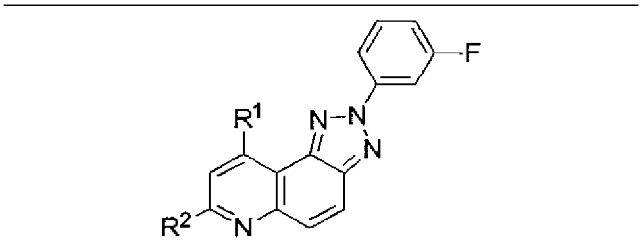

(continued)

| Compound No. | $R^1$ | $R^2$ |
|---|---|---|
| 87 | $CO_2Me$ | 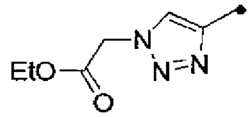 |
| 88 | $CO_2H$ | 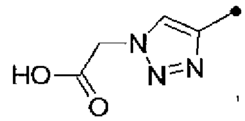 |
| 89 | $CO_2H$ | 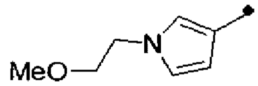 |
| 90 | $CO_2Me$ | 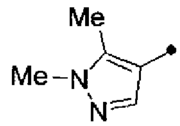 |
| 91 | $CO_2H$ | 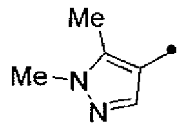 |
| 92 | $CO_2Me$ | 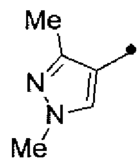 |
| 93 | $CO_2H$ | 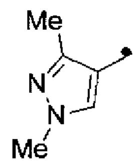 |
| 94 | $CO_2Me$ | 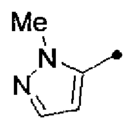 |
| 95 | $CO_2H$ | 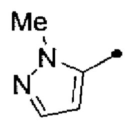 |
| 96 | $CO_2Me$ | 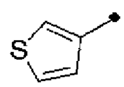 |

Table 9

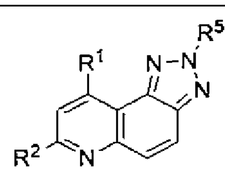

(continued)

| Compound No. | $R^1$ | $R^2$ | $R^5$ |
|---|---|---|---|
| 97 | $CO_2H$ | 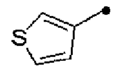 | 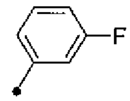 |
| 98 | $CO_2Me$ | 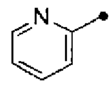 | 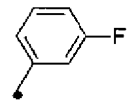 |
| 99 | $CO_2H$ | 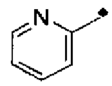 | 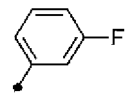 |
| 100 | $CO_2Me$ | Cl | 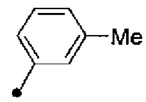 |
| 101 | $CO_2Me$ | 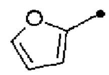 | 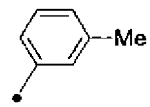 |
| 102 | $CO_2H$ | 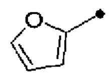 | 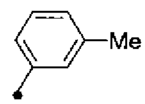 |
| 103 | $CO_2Me$ | Cl | 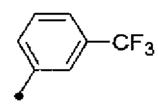 |
| 104 | $CO_2Me$ | 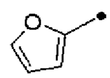 | 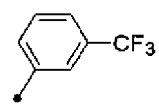 |
| 105 | $CO_2H$ | 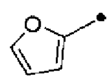 | 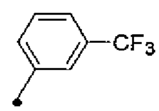 |
| 106 | $CO_2H$ | 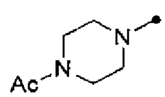 | 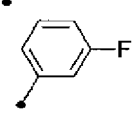 |
| 107 | $CO_2Me$ | 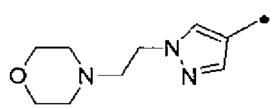 | 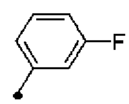 |
| 108 | $CO_2H$ | 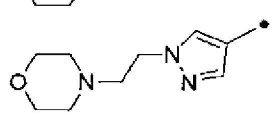 | 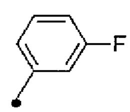 |

Table 10

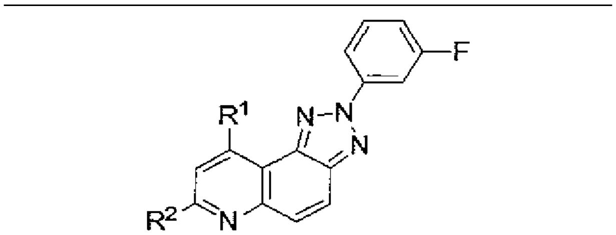

(continued)

| Compound No. | $R^1$ | $R^2$ |
|---|---|---|
| 109 | $CO_2H$ | 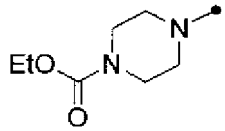 |
| 110 | $CO_2H$ | 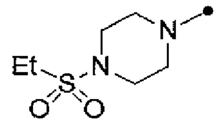 |
| 111 | $CO_2Me$ | 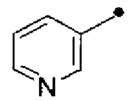 |
| 112 | $CO_2H$ | 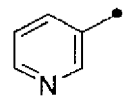 |
| 113 | $CO_2Me$ | 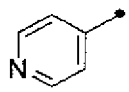 |
| 114 | $CO_2H$ | 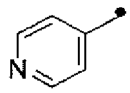 |
| 115 | $CO_2Me$ | 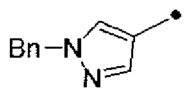 |
| 116 | $CO_2H$ | 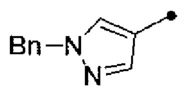 |
| 117 | $CO_2H$ | 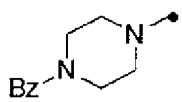 |
| 118 | $CO_2H$ | 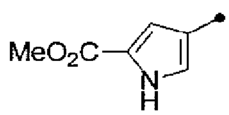 |
| 119 | $CO_2H$ | 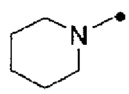 |
| 120 | $CO_2H$ | 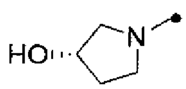 |

Table 11

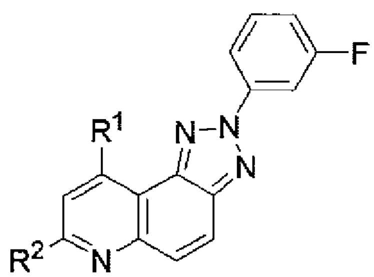

(continued)

| Compound No. | $R^1$ | $R^2$ |
|---|---|---|
| 121 | $CO_2H$ | 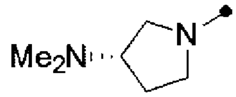 |
| 122 | $CO_2H$ | 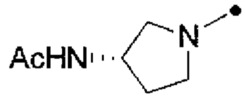 |
| 123 | $CO_2H$ | 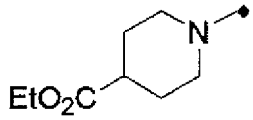 |
| 124 | $CO_2H$ | 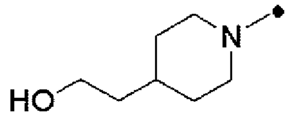 |
| 125 | $CO_2H$ | 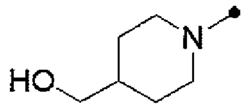 |
| 126 | $CO_2H$ | 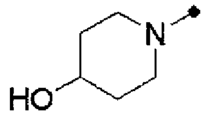 |
| 127 | $CO_2H$ | 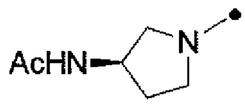 |
| 128 | $CO_2H$ | 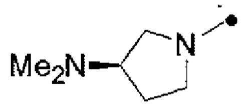 |
| 129 | $CO_2H$ | 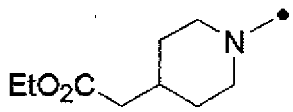 |

Table 12

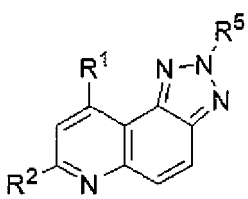

| Compound No. | $R^1$ | $R^2$ | $R^5$ |
|---|---|---|---|
| 130 | $CO_2H$ | 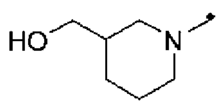 | 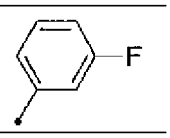 |

(continued)

| Compound No. | $R^1$ | $R^2$ | $R^5$ |
|---|---|---|---|
| 131 | $CO_2H$ | 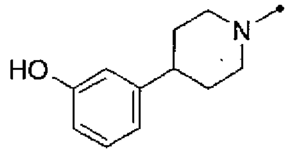 | 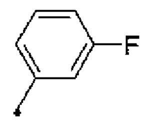 |
| 132 | $CO_2H$ | 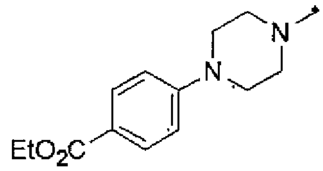 | 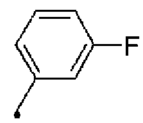 |
| 133 | $CO_2H$ | 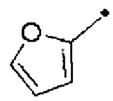 | 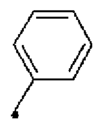 |

Next, pharmacological effects of some typical Compounds (I) will be specifically described with reference to Test Examples.

Test Example 1

<H-PGDS inhibitory activity>

The H-PGDS inhibitory activity of Compound (I) was calculated according to the following protocol attached to Prostaglandin D Synthase Inhibitor Screening Assay Kit (Cat. No.10006595, Cayman Chemical, Ann Arbor, MI, USA).

First, to a 96-well round-bottomplate (Cat. No. 3882-096, Asahi Techno Glass, Chiba), 60 μL/well of an enzyme assay buffer (0.1 mol/L Tris-HCl, pH 8.0), 10 μL/well of a human H-PGDS [Cat. No. 10006593 (human), Cayman Chemical, Ann Arbor, MI, USA] at a final concentration of 0.125 μg/mL, 10 μL/well of reduced glutathione at a final concentration of 1 mmol/L, and 10 μL/well of Compound (I) diluted with the enzyme assay buffer were added and lightly mixed. The plate was allowed to stand at room temperature for 10 minutes, and then 10 μL/well of prostaglandin $H_2$ ($PGH_2$) (Cat. No. 17020, Cayman Chemical) at a final concentration of 50 μmol/L was added thereto to start enzyme reaction. The reaction was allowed to proceed with mixing by a plate mixer for 1 minute. After the enzyme reaction, 10 μL/well of a 4 mg/mL $FeCl_2$-HCl solution prepared by dissolving $FeCl_2$ in 1 mol/L hydrochloric acid was added to stop the reaction and reduce $PGH_2$ simultaneously. The enzyme reaction solution (100 μL/well) was transferred to a 96-well UV-Star half area plate (Cat. No. 675801, Greiner, Frickenhausen, Germany), and measured for absorbance at 232 nm using a microplate reader Power Wave (BioTek Instrument Inc., Winooski, VT, USA).

The H-PGDS inhibitory activity (inhibitory rate) of Compound (I) was calculated by the following formula.

$$\textit{Inhibitory rate}\,(\%) = \frac{\begin{pmatrix}\text{absorbance in the presence of}\\ \text{Compound (I) and H-PGDS}\end{pmatrix} - \begin{pmatrix}\text{absorbance in the absence of Compound (I)}\\ \text{and in the presence of H-PGDS}\end{pmatrix}}{\begin{pmatrix}\text{absorbance in the absence of}\\ \text{Compound (I) and H-PGDS}\end{pmatrix} - \begin{pmatrix}\text{absorbance in the absence of Compound (I)}\\ \text{and in the presence of H-PGDS}\end{pmatrix}} \times 100$$

As a result, Compounds 6, 8, 13, 15, 17, 20, 22, 26, 28, 29, 53, 76, 102 and 116 exhibited an inhibitory rate of 80% or more at a concentration of 1 μmol/L.

Test Example 2

<H-PGDS inhibitory activity of Compound 133>

The H-PGDS inhibitory activity of Compound 133 was calculated according to the following protocol attached to Prostaglandin D Synthase Inhibitor Screening Assay Kit (Cat. No.10006595, Cayman Chemical, Ann Arbor, MI, USA).
First, to a 96-well round-bottomplate (Cat. No. 3882-096, Asahi Techno Glass, Chiba), 60 μL/well of an enzyme assay buffer (0.1 mol/L Tris-HCl, pH 8.0), 10 μL/well of a human H-PGDS [Cat. No. 10006593 (human), Cayman Chemical, Ann Arbor, MI, USA] at a final concentration of 0.125 μg/mL, 10 μL/well of reduced glutathione at a final concentration of 1 mmol/L, and 10 μL/well of Compound 133 diluted with the enzyme assay buffer were added and lightly mixed. The plate was allowed to stand at room temperature for 10 minutes, and then 10 μL/well of prostaglandin $H_2$($PGH_2$) (Cat. No. 17020, Cayman Chemical) at a final concentration of 50 μmol/L was added thereto to start enzyme reaction. The reaction was allowed to proceed with mixing by a plate mixer for 1 minute. After the enzyme reaction, 10 μL/well of a 4 mg/mL $FeCl_2$-HCl solution prepared by dissolving $FeCl_2$ in 1 mol/L hydrochloric acid was added to stop the reaction and reduce $PGH_2$ simultaneously. The enzyme reaction solution (100 μL/well) was transferred to a 96-well UV-Star half area plate (Cat. No. 675801, Greiner, Frickenhausen, Germany), and measured for absorbance at 232 nm using a microplate reader Power Wave (BioTek Instrument Inc., Winooski, VT, USA).
The H-PGDS inhibitory activity (inhibitory rate) of Compound 133 was calculated according to the method in Test Example 1.
As a result, Compound 133 inhibited enzymatic activity of H-PGDS with an inhibitory rate of 73% at a concentration of 1 μmol/L. In contrast, Compound 133 inhibited the activity of enzymes, such as human lipocalin-prostaglandin $D_2$ synthase (human lipocalin-PGDS), cyclooxygenase-1 (COX-1), and cyclooxygenase-2 (COX-2) with a inhibitory rate of only 20% or less at a concentration of 10 μmol/L.
This test showed that Compound 133 has a selective inhibitory effect on H-PGDS.
From the above Test Examples 1 and 2, Compounds (I), such as Compound 133, or a pharmaceutically acceptable salt thereof is considered to inhibit H-PGDS and is useful as a therapeutic and/or preventive agent for a disease involving H-PGDS, for example, a disease involving allergic inflammation (such as asthma, allergic rhinitis, or atopic dermatitis); COPD; a myodegenerative disease (such as muscular dystrophy or polymyositis); or the like.

Test Example 3

<Inhibitory effect on $PGD_2$ production in mouse ear caused by allergic reaction>

A test of $PGD_2$ production in the mouse ear caused by allergic reaction was conducted according to the evaluation method of passive cutaneous anaphylaxis in the mouse ear by Inagaki et al. (Int. Arch. Allergy Appl. Immunol., 1988, 86 (3), 325-30) with some alteration. That is, 8-week-old male BALB/c mice (Charles River Japan, Atsugi, Kanagawa) were anesthetized using an isoflurane small animal anesthetizer (MK-A110, Muromachi Kikai, Tokyo), and then anti-TNP mouse monoclonal IgE antibody diluted to 10 μg/mL with a phosphate buffer solution (PBS: Cat. No. 2810305, MP Biomedicals, Solon, OH, USA) [prepared by culturing anti-TNP mouse monoclonal IgE antibody-producing cells (IGEL-b4, ATCC, Manassas, VA, USA) in ascitic fluid, collecting the ascitic fluid containing anti-TNP mouse monoclonal IgE antibody followed by purification thereof, measuring the protein concentration, and then adjusting the final stock concentration to 6 mg/mL] was intradermally injected in an amount of 10 μL each into the center of the right and left ears for passive sensitization. The day after the sensitization, a 1% solution of evans blue (Evans Blue: Cat. No. 20633-4, Aldrich Chemical Company, Milwaukee, WI, USA) in saline containing 10 μg/mL of 2,4,6-trinitrophenyl bovine serum albumin (TNP-BSA: Cat. No. LG-1117, LSL, Tokyo) was injected with an amount of 0.2 mL per animal into the tail vein to induce antigen-antibody reaction.
Compound 102 was pounded in an agate mortar, suspended in a 0.5% methyl cellulose aqueous solution containing 0.6% sodium hydrogencarbonate (solvent; methyl cellulose 400: Cat. No. 132-05055, Wako Pure Chemical Industries, Osaka), and orally administered at a dose of 3 mg/kg 1 hour before inducing antigen-antibody reaction.
One hour after the induction of antigen-antibody reaction, the mice were killed by cervical dislocation and the ears were cut off with scissors. Then, the ear tissue centering around the EB leakage site was punched out with a trephine biopsy having a diameter of 8 mm (Biopsy Punch, Cat. No. BP-80F, Kai Industries, Gifu). The harvested pieces of ear tissue were immediately frozen in liquid nitrogen, and preserved at -80˚C until measurement of the quantity of $PGD_2$.
The quantity of $PGD_2$ was determined in the following manner. First, to a tube in which a frozen ear piece had been placed, 500 μL of acetone containing 10 μmol/L of indomethacin was added, and then the ear piece was completely crushed with a tissue lyser (TissueLyser: QIAGEN, Retsch GmbH, Haan, Germany). Centrifugation was performed under cooling (15000 rpm, 10 minutes, 4˚C; an inverter micro refrigerated centrifuge, KUBOTA1720, Kubota Corporation, Tokyo), and 400 μL of the supernatant was collected into a new tube. The collected fluid was evaporated to dryness in

a nitrogen stream, and the dried substance was dissolved in 100 μL of a buffer solution for enzyme immunoassay (Cat. No. 400060, Cayman Chemical). To this, 400 μL of ethanol was added with mixing, and the mixture was allowed to stand for 5 minutes. The mixture was centrifuged again, and 400 μL of the supernatant was collected, which was then evaporated to dryness in a nitrogen stream. To the dried substance was added 300 μL of a 1 mol/L acetic acid buffer solution (pH 4.0), and the mixture was dissolved. The sample solution was passed through a purification column [Sep-Pak (registered trademark) Light C18, Cat. No. WAT023501, Waters, Milford, MA, USA], which had been activated with 3 mL of methanol and then washed with 3 mL of purified water beforehand. Then, the column was washed again with 3 mL of purified water. Next, 3 mL of hexane was passed through the column, and finally 2 mL of ethyl acetate containing 1% of methanol was applied to elute $PGD_2$. The collected fluid was evaporated to complete dryness in a nitrogen stream and the dried substance was dissolved in a buffer solution for enzyme immunoassay to give a purified sample.

Equal amounts of the obtained purified sample and a pre-prepared reaction solution for O-methyloximation [prepared by dissolving 100 mg of methoxylamine hydrochloride (Cat. No. A19188, Alfa Aesar, Ward Hill, MA, USA) in 5 mL of a 10% aqueous ethanol solution, dissolving 820 mg of sodium acetate in 5 mL of a 10% aqueous ethanol solution, and then mixing the two solutions] were mixed, and allowed to react at 60˚C for 30 minutes to obtain a sample containing O-methyloximation $PGD_2$, which is a stable form. The quantity of the O-methyloximation $PGD_2$ was determined according to the protocol of Prostaglandin D2-MOX Express EIA Kit (Cat. No. 500151, Cayman Chemical).

As a result, Compound 102 at a dose of 3 mg/kg inhibited 84% of the $PGD_2$ production in mouse ear caused by allergic reaction.

Test Example 4

<Inhibitory effect on $PGD_2$ production in mouse ear caused by allergic reaction>

A test of $PGD_2$ production in the mouse ear mediated by allergic reaction was conducted according to the evaluation method of passive cutaneous anaphylaxis in the mouse ear by Inagaki et al. (Int. Arch. Allergy Appl. Immunol., 1988, 86 (3), 325-30) with some alteration. That is, 10-week-old male BALB/c mice (Charles River Japan, Atsugi, Kanagawa) were given general anesthesia using an isoflurane small animal anesthetizer (MK-A110, Muromachi Kikai, Tokyo), 10 μL each of anti-TNP mouse monoclonal IgE antibody diluted to 10 μg/mL with a phosphate buffer solution (PBS: Cat. No. 2810305, Lot 8313J, MP Biomedicals, Solon, OH, USA) [prepared by culturing anti-TNP mouse monoclonal IgE antibody-producing cells (IGEL-b4, ATCC, Manassas, VA, USA) in ascitic fluid, collecting the ascitic fluid containing anti-TNP mouse monoclonal IgE antibody followed by purification thereof, measuring the protein concentration, and then adjusting the final stock concentration to 6 mg/mL] was intradermally injected into the center of the right and left ears for passive sensitization. The day after the sensitization, a 1% solution of evans blue [Evans Blue (EB) : Cat. No. 20633-4, Aldrich Chemical Company, Milwaukee, WI, USA] in saline containing 10 μg/mL of 2,4,6-trinitrophenyl bovine serum albumin (TNP-BSA: Cat. No. LG-1117, LSL, Tokyo) was injected with an amount of 0.2 mL per animal into the tail vein to induce antigen-antibody reaction.

Compound 133 was pounded in an agate mortar, suspended in a 0.5% methyl cellulose aqueous solution (solvent; methyl cellulose 400: Cat. No. 132-05055, Wako Pure Chemical Industries, Osaka), and orally administered at a dose of 10 mg/kg 1 hour before inducing antigen-antibody reaction.

One hour after the induction of antigen-antibody reaction, the mice were killed by cervical dislocation and the ears were cut off with scissors. Then, the ear tissue centering around the EB leakage site was punched out with a trephine biopsy having a diameter of 8 mm (Biopsy Punch, Cat. No. BP-80F, Lot 07K33, Kai Industries, Gifu). The harvested pieces of ear tissue were immediately frozen in liquid nitrogen, and preserved at -80˚C until measurement of the quantity of $PGD_2$.

The quantity of $PGD_2$ was determined in the following manner. First, to a tube in which a frozen ear piece had been placed, 500 μL of acetone containing 10 μmol/L of indomethacin was added, and then the ear piece was completely crushed with a tissue lyser (TissueLyser: QIAGEN, Retsch GmbH, Haan, Germany). Centrifugation was performed under cooling (15000 rpm, 10 minutes, 4˚C; an inverter micro refrigerated centrifuge, KUBOTA1720, Kubota Corporation, Tokyo), and 400 μL of the supernatant was collected into a new tube. The collected fluid was evaporated to dryness in a nitrogen stream, and the dried substance was dissolved in 100 μL of a buffer solution for enzyme immunoassay (Cat. No. 400060, Cayman Chemical). To this, 400 μL of 100% ethanol was added with mixing, and the mixture was allowed to stand for 5 minutes. The mixture was centrifuged again, and 400 μL of the supernatant was collected, which was then evaporated to dryness in a nitrogen stream. To the dried substance was added 300 μL of a 1 mol/L acetic acid buffer solution (pH 4.0), and the mixture was dissolved. The sample solution was passed through a purification column [Sep-Pak. (registered trademark) Light C18, Cat. No. WAT023501, Waters, Milford, MA, USA], which had been activated with 3 mL of ethanol and then washed with 3 mL of purified water beforehand. Then, the column was washed again with 3 mL of purified water. Next, 3 mL of hexane was passed through the column, and finally 2 mL of ethyl acetate containing 1% of methanol was applied to elute $PGD_2$. The collected fluid was evaporated to complete dryness in a nitrogen stream and the dried substance was dissolved in a buffer solution for enzyme immunoassay to give a purified sample.

Equal amounts of the obtained purified sample and a pre-prepared reaction solution for O-methyloximation [prepared by dissolving 100 mg of methoxylamine hydrochloride (Cat. No. A19188, Alfa Aesar, Ward Hill, MA, USA) in 5 mL of a 10% aqueous ethanol solution, dissolving 820 mg of sodium acetate in 5 mL of a 10% aqueous ethanol, solution, and then mixing the two solutions] were mixed, and allowed to react at 60˚C for 30 minutes to obtain a sample containing O-methyloximation $PGD_2$, which is a stable form. The quantity of the O-methyloximation $PGD_2$ was determined according to the protocol of Prostaglandin D2-MOX Express EIA Kit (Cat. No. 500151, Cayman Chemical).

As a result, Compound 133 at a dose of 10 mg/kg inhibited 88% of the $PGD_2$ production in mouse ear caused by allergic reaction.

Therefore, it was shown that Compound (I) or a pharmaceutically acceptable salt thereof inhibits $PGD_2$ production and is useful as a therapeutic and/or preventive agent for, for example, a disease involving allergic inflammation (such as asthma, allergic rhinitis, or atopic dermatitis).

Test Example 5

<Effect on antigen-induced nasal congestion in rats>

Allergic rhinitis is a type I hypersensitivity disorder whose three main symptoms are sneeze, watery rhinorrhea, and nasal congestion. Among them, nasal congestion, on which existing drugs are thought to have little effect, is a factor which greatly reduces the QOL of the patients (Laryngoscope, 2003, 113, 1118-1122). Therefore, the effect of Compound 133 on antigen-induced nasal congestion in rats was examined.

Six-week-old male BN/Crlj Crlj SPF/VAF rats were purchased from Charles River Japan (Hino, Kanagawa) and used for experiments. Equal amounts of a 0.4 mg/mL ovalbumin/saline solution (OVA: Grade III, Cat. No. A-5378, Sigma-Aldrich, St. Louis, MO, USA) and a 20 mg/mL suspension of aluminum hydroxide (alum: Cat. No. 014-01925, Wako Pure Chemical Industries) in saline were mixed to give a sensitizing fluid. First, a suspension of inactivated killed pertussis bacteria (Cat. No. 15330-91, Nacalai Tesque, Tokyo) was doubly diluted with saline. The diluted suspension was subcutaneously administered on the back of each rat at a dose of 0.1 mL (containing 10 billion bacterial cells) per rat, and immediately after that, the sensitizing fluid was intraperitoneally administered at a dose of 1 mL per rat. About one week later, the sensitizing fluid administration was repeated in a similar manner, and the rats were actively sensitized. About two weeks after the last administration of the sensitizing fluid, a 10 w/v% of OVA/saline solution in physiological saline was administered to both nostrils at a dose of 2 5 $\mu$L per nostril to induce nasal congestion. Compound 133 was pounded in an agate mortar, suspended in a 0.5% methyl cellulose aqueous solution (solvent; methyl cellulose 400: Cat. No. 132-05055, Wako Pure Chemical Industries, Osaka), and orally administered at a dose of 10 mg/kg 1 hour before nasal administration of the antigen.

The nasal congestion was evaluated based on the elevation of the intranasal pressure determined in the following manner. First, the rats were anesthetized by intraperitoneal administration of sodium pentobarbital [Somnopentyl (registered trademark), Kyoritsu Seiyaku, Tokyo] at a dose of 50 mg/kg. Next, each rat was restrained in a supine position, and the area around the trachea was cut open, and the trachea was exposed. A tracheal cannula was inserted into the lung side of the trachea for securing respiration. Apart from this cannula, the trachea of the nasal side was cut open, and a cannula for measurement (JMS cutdown tube, JMS, Hiroshima) was inserted in the direction of the nasal cavity. On a hot plate (FHP-4505, OMRON, Kyoto) set at 37˚C, each rat was connected to an artificial ventilator for small animals (RODENT VENTILATOR MODEL683, HARVARD APPARATUS, Holliston, MA, USA), and 2.5 mL of air was injected into the nasal cavity 65 times per minute. The pressure inside the nasal cavity was detected using a pressure transducer branched from the end of the cannula for measurement, and analyzed using Win-PULMOS-III (M. I.P.S. Inc., Osaka). The pressure inside the nasal cavity was measured for 3 minutes immediately after the connection to the cannula for measurement and the artificial ventilator, and the data was calculated for a 3-minute average.

As a result, Compound 133 at a dose of 10 mg/kg inhibited 55% of the antigen-induced nasal congestion in rats. That is, it was shown that Compound (I) or a pharmaceutically acceptable salt thereof is useful as a therapeutic and/or preventive agent for allergic rhinitis.

Test Example 6

<Effect of the compound on expression of airway hyperresponsiveness and eosinophil infiltration in the airway in a mouse model of asthma>

It is clinically known that a cyclooxygenase (COX) inhibitor, which inhibits upstream of arachidonate metabolism, widely inhibits the production of various prostanoids including $PGD_2$ but is not effective on bronchial asthma. The reason for this is thought to be that the inhibitor also inhibit prostanoids which affect respiratory inflammation defensively (Nat. Immunol., 2005, 6 (5), 524-531). Therefore, it is expected that the drug which selectively inhibit $PGD_2$ production may

be useful for bronchial asthma. Therefore, the effect of Compound 133 on expression of airway hyperresponsiveness and eosinophil infiltration in the airway in a mouse model of asthma was examined.

The mouse model of asthma was prepared as follows. For experimental use, 6-week-old male BALB/c mice were purchased from Charles River Japan (Atsugi, Kanagawa). Equal amounts of a 0.5 mg/mL solution of ovalbumin in physiological saline and a 10 mg/mL suspension of aluminum hydroxide in physiological saline were mixed to give a sensitizing fluid. With well mixing of the sensitization fluid, 0.2 mL of the fluid was intraperitoneally administered to each mouse for sensitization. About 1 week after the initial sensitization, the same procedure was performed for additional sensitization. From about 2 weeks after the additional sensitization, antigen exposure was performed every 4 days, 3 times in total, to induce asthmatic response (expression of airway hyperresponsiveness and eosinophil infiltration in the airway). For the antigen exposure, each mouse was placed in a plastic cage (26×42×15 cm; about 16 L) and forced to inhale a mist of a 10 mg/mL solution of ovalbumin in physiological saline produced using an ultrasonic nebulizer (Model NE-U17, OMRON, Kyoto) for 30 minutes each time. The negative control group was forced to inhale a physiological saline solution instead of the OVA solution. Compound 133 was pounded in an agate mortar, suspended in a 0.5% methyl cellulose aqueous solution, and orally administered at a dose of 3 mg/kg once daily from the first day to the last day of the antigen exposure.

The expression of airway hyperresponsiveness (increase of airway reactivity) was evaluated by measuring, under anesthesia, airway reactivity to methacholine (MCh: acetyl-β-methylcholine chloride, Cat. No. A-2251, Sigma-Aldrich) using a respiratory function analyzer [BioSystem XA; version 2.0.2.21 for Windows (registered trademark), preamplifier: MAX2215, body plethysmography box: PLY3011, pressure transducer: Validyne DP45-28, flaw transducer: Validyne DP45-14, Buxco Electronics, Troy, NY, USA]. That is, on the next day after the last antigen exposure, each of the above mice was anesthetized with Somnopentyl, and after the introduction of a tracheal cannula into the trachea and a venous cannula into the jugular vein, the mouse was placed still in the supine position in a body plethysmography box. The tracheal cannula was connected to an artificial ventilator for small animals (RODENT VENTILATOR MODEL683, HARVARD APPARATUS). Mandatory ventilation was performed by injecting 0.25 mL of air 120 times per minute, and immediately pancuronium bromide (Cat. No. P1918, Sigma-Aldrich) was intravenously administered at a dose of 0.2 mg/kg and 50 μL/head to stop spontaneous respiration. As an indicator of airway reactivity, respiratory resistance (RL) values recorded every 5 seconds by the respiratory function analyzer were used. After fixing a mouse to the device and confirming the RL stability, the value after intravenous administration of a physiological saline solution was recorded first. Then, 50 μL each of solutions of different concentrations of MCh (corresponding to 0.01 to 0.64 mg/kg) in physiological saline were intravenously administered in the order of increasing concentration, and the reaction was recorded. Using the peak value of each reaction as the RL value at the corresponding MCh concentration, the rate to the RL at the administration of a physiological saline solution was calculated to draw a RL-MCh concentration curve. The expression of airway hyperresponsiveness was evaluated by comparing the airway reactivities in each concentration of MCh.

Immediately after the airway reactivity measurement, bronchoalveolar lavage (BAL) was performed. That is, after taking out a mouse from the body plethysmography box, the abdomen of the mouse was opened, an incision was made in the diaphragm, and a cannula for BAL was introduced. Through the cannula, 0.75 mL of a physiological saline solution was injected, and BAL fluid was collected. This procedure was repeated twice. The BAL fluids collected from the same individual were combined and centrifuged at 1500 rpm at 4°C for 10 minutes using a refrigerated centrifuge (model: CF7D2, rotor model: RT-3S3, Hitachi, Tokyo). The supernatant was removed by suction, and the residue was suspended in 200 μL of a physiological saline solution added thereto. This suspension was measured for total cell concentration with a fully automatic hematology analyzer for animals (Celltac α, MEK-6358, Nihon Kohden, Tokyo). Then, using a cellular specimen preparation machine (Cytospin 4, Thermo Fisher Scientific, Waltham, MA, USA), smear preparations were prepared. Morphological cell classification (macrophage, neutrophil, eosinophile, and lymphocyte) was carried out under the microscope, and the number of infiltrated eosinophil in the airway was calculated by multiplying the total cell concentration by the percent of eosinophile obtained by the cell classification.

As a result of the above test, Compound 133 at a dose of 3 mg/kg inhibited 98% of the expression of airway hyperresponsiveness and 55% of the eosinophil infiltration in the airway.
That is, it was shown that Compound (I) or a pharmaceutically acceptable salt thereof is useful as a therapeutic and/or preventive agent for asthma (such as bronchial asthma).
In addition, Compound 133 or a pharmaceutically acceptable salt thereof exhibited the effect when orally administered.

Test Example 7

<Effect of the compound on myodegeneration in spontaneous muscular dystrophy model mice>

It is known that H-PGDS is expressed in necrotic muscle of polymyositis patients and Duchenne muscular dystrophy (DMD) known as the most common muscular dystrophy (Acta Neuropathol. , 2002, 104, 377-384), and it is reported that a H-PGDS inhibitor inhibits progressive muscle necrosis in mdx mice as model mice of muscular dystrophy

(Am. J. Pathol., 2009, 174, 1735-1744). Therefore, the effect of Compound 133 on myodegeneration in mdx mice was examined.

mdx Mice (C57BL/10scSn-Dmd$^{mdx}$) were purchased in male and female pairs from Jackson Laboratories, and bred in-house for use. After weaned at 3 weeks old, male mdx mice were selected, which were subjected to the test from 4 weeks old. Compound 133 was dissolved in MEYLON 84 (registered trademark, 8.4% sodium bicarbonate injection, Otsuka) to prepare 12 mL of a 3 mg/mL solution thereof. This was added to and mixed with 188 mL of sterile tap water (final concentration of the compound: 180 μg/mL), and the mixture was administered by unrestricted intake (actual dose of the compound was about 30 mg/kg). For the vehicle administration group, MEYLON 84 (12 mL) added to 188 mL of sterile tap water was used. Six days after the start of administration, a physiological saline solution containing 1% EB dye was intraperitoneally injected in a volume of 0.2 mL per mouse. Twenty four hours after the EB dye administration, dissection was performed. In the dissection, the diaphragm was harvested first, and preserved in a physiological saline solution at room temperature until the measurement of EB-stained areas. Further, the left hind foot was decorticated, and muscle of the facies posterior cruris was harvested and frozen for preservation (-80˚C).

As evaluation of the diaphragm muscle, the degenerated and/or necrotic muscle area ratio of the diaphragm was determined. That is, in the physiological saline solution, unnecessary portions, such as connective tissue, were removed, and the diaphragm was cut into two or three pieces for distortion correction. The diaphragm piece was spread onto a glass slide with its abdominal cavity side up, a drop of physiological saline solution was put thereon, and a cover glass was placed thereon. The thus prepared specimen was digitally shot at 20-fold magnification using a digital microscope (VHX-1000, KEYENCE, Osaka) and the images were recorded in TIFF format. Using the nature that degenerated and/or necrotic muscle absorbs EB and turns blue (J. Anat., 2002, 200, 69-79), the area of the diaphragm piece and the area of blue region showing the distribution of degenerated and/or necrotic muscle were determined with an image analyzer (LUZEX AP, Nireco, Tokyo). The degenerated and/or necrotic muscle area ratio is expressed as a percent by dividing the sum of the degenerated and/or necrotic area of each diaphragm piece divided by the sum of the area of each diaphragm piece.

For evaluation of the.posterior crural muscle, the degenerated and/or necrotic muscle area ratio of the gastrocnemius muscle was determined. That is, three frozen 10 μm-thick sections of the left posterior crural muscle were prepared by cross-sectionally slicing the central part of the muscle at intervals of about 100 μm. The sections were air-dried, penetrated with xylene, and encapsulated using a non-aqueous mounting agent (DPX, Merck, Tokyo). Then, using a fluorescence microscope (BZ-8100, KEYENCE, Osaka), a green fluorescence image (OP-66836 BZ filter set, excitation wavelength: 470 plus/minus 20 nm, dichroic mirror wavelength: 495 nm, absorption wavelength: 535 plus/ minus 25 nm, KEYENCE, Osaka) and a red fluorescence image (OP-66836 BZ filter set, excitation wavelength: 470 plus/minus 20 nm, dichroic mirror wavelength: 495 nm, absorption wavelength: 535 plus/minus 25 nm, KEYENCE, Osaka) were taken for each specimen. The above-obtained green fluorescence image and the red fluorescence image were merged to give a composite image, which was further processed, using image processing software in the fluorescence microscope, into a sharpened digital image in bit map format. Using thus obtained images, the following image analysis was performed.

Normal muscle emits green autofluorescence, and degenerated and/or necrotic muscle, into which EB is absorbed, emits red fluorescence. Connective tissue emits red fluorescence similar to that of degenerated and/or necrotic muscle due to EB penetrated and remaining therein. Using the fluorescence characteristics (J. Anat., 2002, 200, 69-79), each area of the muscles was determined by the procedure shown below.

A1 A binary image of a gastrocnemius section was obtained by extracting black background portions (black) and reversing the image (Image A).

A2 Red portions were extracted and divided into two, connective tissue portions and degenerated and/or necrotic muscle portions, based on the size, shape factor, and the like of each extracted portion. The two were each binarized (connective tissue: Image B, degenerated and/or necrotic muscle: Image C).

A3 An image obtained by subtracting Image B from Image A was set as the measurement field, and then the area of the measurement field and the area of Image C in the field were determined.

A4 The above A1 to A3 were performed for each of the three specimens of each animal. The degenerated and/or necrotic muscle area ratio is expressed as a percent by dividing the sum of the Image C areas of the three specimens divided by the sum of the measurement field areas of the three.

As a result of the above test, Compound 133 inhibited 40% of the degeneration and/or the necrosis of the diaphragm and 49% of the degeneration and/or the necrosis of the gastrocnemius muscle.

That is, it was shown that Compound (I) or a pharmaceutically acceptable salt thereof is useful as a therapeutic and/or preventive agent for a myodegenerative disease (such as muscular dystrophy or polymyositis); and the like.

From the above tests, Compound (I) or a pharmaceutically acceptable salt thereof is considered to be useful as a therapeutic and/or preventive agent for a disease involving H-PGDS, for example, a disease involving allergic

inflammation (such as asthma, allergic rhinitis, or atopic dermatitis); COPD; a myodegenerative disease (such as muscular dystrophy or polymyositis); and the like. More specifically, the compound or a pharmaceutically acceptable salt thereof is considered to be useful as a therapeutic and/or preventive agent for a disease involving allergic inflammation, such as asthma, allergic rhinitis, or atopic dermatitis (especially asthma or allergic rhinitis); COPD; a myodegenerative disease, such as muscular dystrophy or polymyositis (especially muscular dystrophy); and the like.

Compound (I) or a pharmaceutically acceptable salt thereof can be administered alone. However, Compound (I) or a pharmaceutically acceptable salt thereof is usually preferably provided as various pharmaceutical preparations. Further, such pharmaceutical preparations are to be used in animals or humans.

The pharmaceutical preparations according to the present invention may contain Compound (I) or a pharmaceutically acceptable salt thereof alone as an effective ingredient or a mixture thereof with an optional active ingredient for any other therapy. Further, these pharmaceutical preparations are prepared by mixing the active ingredient with one or more pharmaceutically acceptable carriers (such as a diluent, a solvent and an excipient) and then subjecting the mixture to any method well known in the technical field of pharmaceutics.

Regarding the administration route, it is preferable that the most effective route is selected for therapy. Examples of the administration route include oral administration and parenteral administration, such as intravenous administration. Examples of the dosage form include tablets, injections, and the like.

For example, tablets and the like suitable for oral administration may be prepared by use of excipients such as lactose, disintegrators such as starch, lubricants such as magnesium stearate, binders such as hydroxypropyl cellulose, and the like.

For example, the injection and the like suitable for parenteral administration can be produced with a diluent or a solvent such as a salt solution, a glucose solution, or a mixture of a salt solution and a glucose solution, or the like.

The doses and the frequencies of administration of Compound (I) or a pharmaceutically acceptable salt thereof may vary depending on the dosage form, age and body weight of a patient, nature or seriousness of the symptom to be treated, and the like. However, in oral administration, in general, a dose of 0.01 to 1000 mg, preferably, 0.05 to 100 mg is administered to an adult patient once to several times a day. In parenteral administration, such as intravenous administration, a dose of 0.001 to 1000 mg, preferably, 0.01 to 100 mg is administered to an adult patient once to several times a day. However, these doses and frequencies of administration vary depending on the various conditions described above.

Hereinafter, the present invention will be described more specifically with reference to Examples and Reference Examples. However, the scope of the present invention is not limited to the Examples.

A proton nuclear magnetic resonance spectrum ($^1$H-NMR) used in Examples and Reference Examples was determined at 270 MHz or 300 MHz, and exchangeable proton is not clearly observed in some cases depending on the compounds and measurement conditions. Further, for the descriptions of the multiplicity of signals, those generally applied are used, and the symbol "br" represents an apparent broad signal.

Each compound was named using ChemBioDraw Ver. 11.0 (CambridgeSoft Corporation).

Reference Example 1

Production of 7-(furan-2-yl)-2-phenyl-2H-[1,2,3]triazolo-[4,5-f]quinoline-9-carboxylic acid

Step 1

2-chloro-5-nitroquinoline-4-carboxylic acid methyl ester

The title compound was synthesized according to Step A and Step B of Example 319 of WO 2003/45313, page 95.

Step 2

2-(furan-2-yl)-6-nitroquinoline-4-carboxylic acid methyl ester

Under nitrogen atmosphere, the 2-chloro-6--nitroquinoline-4-carboxylic acid methyl ester obtained in Step 1 (1.5g, 5.64 mmol), 2-tributylstannylfuran (2.13 mL, 6.77 mmol), and a 1,1'-[bis(diphenylphosphino)ferrocene]-dichloropalladium(II) dichloromethane adduct (230 mg, 0.28 mmol) were dissolved in 1,2-dimethoxyethane, and the mixture was refluxed for 2 hours. After the mixture was cooled to room temperature, a saturated ammonium fluoride aqueous solution was added thereto. The precipitate was filtered and removed. Organic compounds were extracted with chloroform, and the organic layer was dried over anhydrous magnesium sulfate. After filtration, the solvent in the filtrate was removed in vacuo. The residue was purified by silica gel column chromatography (methanol/chloroform = 0/100 to 1/99),

and thus the title compound (883 mg, 53%) was obtained as a yellow solid substance.
$^1$H-NMR (DMSO-$d_6$) δ: 4.06 (3H, s), 6.83 (1H, dd, J =3.3, 1.8 Hz), 7.64 (1H, d, J =3.3 Hz), 8.08 (1H, d, J = 1.8 Hz), 8.26 (1H, d, J = 9.2 Hz), 8.49 (1H, s), 8.53 (1H, dd, J = 9.2, 2.6 Hz), 9.57 (1H, d, J = 2.6 Hz).
ESI-MS (m/z); 299 $[M+H]^+$.

Step 3

6-amino-2-(furan-2-yl)quinoline-4-carboxylic acid methyl ester

The 2-(furan-2-yl)-6-nitroquinoline-4-carboxylic acid methyl ester (880 mg, 3.0 mmol) obtained in Step 2 was dissolved in ethanol (15 mL) and acetic acid (15 mL), and iron (660 mg, 12 mmol) was added thereto at room temperature. The mixture was refluxed for 3 hours, and a saturated sodium hydrogencarbonate aqueous solution was added thereto. Insoluble compounds were filtered and removed, and organic compounds were extracted with chloroform. Then the organic layer was dried over anhydrous magnesium sulfate. After filtration, the solvent in the filtrate was removed in vacuo. The residue was purified by silica gel column chromatography (methanol/chloroform = 0/100 to 1/99), and thus the title compound (468 mg, 59%) was obtained as a yellow solid substance. $^1$H-NMR (DMSO-$d_6$) δ: 3.96 (3H, s), 6.03 (2H, s), 6.68 (1H, dd, J = 3.3, 2.1 Hz), 7.17 (1H, d, J = 3.3 Hz), 7.24 (1H, dd, J = 8.8, 2.0 Hz), 7.58 (1H, d, J = 2.1 Hz), 7.77 (1H, d, J = 8.8 Hz), 7.86 (1H, d, J = 2.0 Hz), 8.12 (1H, s).
ESI-MS (m/z); 269 $[M+H]^+$.

Step 4

7-(furan-2-yl)-2-phenyl-2H-[1,2,3]triazolo[4,5-f]quinoline-9-carboxylic acid methyl ester

Aniline (0.49 mL, 5.36 mmol) was dissolved in 3 mol/L hydrochloric acid (5 mL). To this, sodium nitrite (339 mg, 4.91 mmol) dissolved in water (5 mL) was added dropwise with ice cooling. After the mixture was stirred at 0˚C for 1 hour, sodium acetate (1.1 g, 13.4 mmol) dissolved in water (10 mL) was added thereto dropwise, and the mixture was stirred at 0˚C for 10 minutes. Further, the 6-amino-2-(furan-2-yl)quinoline-4-carboxylic acid methyl ester (1.2 g, 4.46 mmol) obtained according to Step 3 and suspended in ethanol (24 mL) was added thereto. The mixture was stirred at 0˚C for 2 hours, and the precipitate was filtered and collected, and thus a crude product of 6-amino-2-(furan-2-yl)-5-phenylazoquinoline-4-carboxylic acid methyl ester was obtained.

The crude product of 6-amino-2-(furan-2-yl)-5-phenylazoquinoline-4-carboxylic acid methyl ester obtained above was dissolved in pyridine (25 mL) and water (25 mL), and copper sulfate (2.85 g, 17.8 mmol) was added thereto at room temperature. After refluxed for 4 hours, the mixture was cooled to room temperature, and 1 mol/L hydrochloric acid was added thereto. Organic compounds were extracted with chloroform, and the organic layer was dried over anhydrous magnesium sulfate. After filtration, the solvent in the filtrate was removed in vacuo. The residue was purified by silica gel column chromatography (ethyl acetate/hexane = 1/9 to 3/7), and thus the title compound (567 mg, 34%) was obtained as a red-orange solid substance.
$^1$H-NMR ($CDCl_3$) δ: 4.20 (3H, s), 6.63 (1H, dd, J = 3.7, 1.8 Hz), 7.30 (1H, dd, J = 3.7, 0.7 Hz), 7.46 (1H, tt, J = 6.8, 1.3 Hz), 7.57 (2H, td, J = 8.6, 6.8 Hz), 7.65 (1H, dd, J = 1.8, 0.7 Hz), 8.01 (1H, s), 8.03 (1H, d, J = 9.2 Hz), 8.11 (1H, d, J = 9.5 Hz), 8.32 (2H, dd, J = 8.6, 1.3 Hz).
ESI-MS (m/z); 371 $[M+H]^+$.

Step 5

7-(furan-2-yl)-2-phenyl-2H-[1,2,3]triazolo[4,5-f]quinoline-9-carboxylic acid

7-(furan-2-yl)-2-phenyl-2H-[1,2,3]triazolo[4,5-f]quinoline-9-carboxylic acid methyl ester obtained according to Step 4 (790 mg, 2.14 mmol) was dissolved in tetrahydrofuran (21 mL), and a 5 mol/L sodium hydroxide aqueous solution (4.3 mL) was added thereto at room temperature. After refluxed for 12 hours, the mixture was cooled to room temperature, and 1 mol/L hydrochloric acid (25 mL) was added thereto. Organic compounds were extracted with ethyl acetate, and the organic layer was dried over anhydrous magnesium sulfate. After filtration, the solvent in the filtrate was removed in vacuo. The residue was reslurriedwith ethyl acetate/hexane (1/1), the precipitate was filtered and collected, and thus the title compound (610 mg, 80%) was obtained as a red-orange solid substance.
$^1$H-NMR (300 MHz, DMSO-$d_6$) δ: 6.77 (1H, dd, J = 3.3, 1.8 Hz), 7 .48 (1H, d, J = 3.3 Hz), 7.58 (1H, t, J = 7.5 Hz), 7.71 (2H, dd, J = 7.8, 7.5 Hz), 7.99 (1H, d, J = 1.8 Hz), 7.99 (1H, d, J = 9.6 Hz), 8.10 (1H, s), 8.28 (1H, d, J = 9.6 Hz), 8.30 (2H, d, J = 7.8 Hz).
ESI-MS (m/z); 357 $[M+H]^+$.

Example 1

Step 1

2-chloro-6-nitroquinoline-4-carboxylic acid methyl ester (Compound 1-1)

The title compound was synthesized according to WO 2003/45313, page 95, Example 319, Step A and Step B.

Step 2

2-(furan-2-yl)-6-nitroquinoline-4-carboxylic acid methyl ester (Compound 1-2)

Under nitrogen atmosphere, Compound 1-1 (1.5 g, 5.64 mmol) obtained in Step 1, 2-tributylstannylfuran (2.13 mL, 6.77 mmol), and a 1,1'-[bis(diphenylphosphino)ferrocene]palladium(II) chloride dichloromethane adduct (230 mg, 0.28 mmol) were dissolved in 1,2-dimethoxyethane, and the mixture was refluxed for 2 hours. After the mixture was cooled to room temperature, a saturated ammonium fluoride aqueous solution was added thereto. The precipitate was filtered and removed, and the filtrate was extracted with chloroform, dried over anhydrous magnesium sulfate. After filtration, the solvent in the filtrate was removed in vacuo. The residue was purified by silica gel column chromatography (methanol/chloroform = 0/100 to 1/99), and thus the title Compound 1-2 (883 mg, 53%) was obtained as a yellow solid substance.
$^1$H-NMR (DMSO-$d_6$) $\delta$: 9.57 (1H, d, J = 2.6 Hz), 8.53 (1H, dd, J = 9.2, 2.6 Hz), 8.49 (1H, s), 8.26 (1H, d, J = 9.2 Hz), 8.08 (1H, d, J = 1.8 Hz), 7.64 (1H, d, J = 3.3 Hz), 6.83 (1H, dd, J = 3.3, 1.8 Hz), 4.06 (3H, s).
ESI-MS (m/z) 299 $[M+H]^+$.

Step 3

6-amino-2-(furan-2-yl)quinoline-4-carboxylic acid methyl ester (Compound 1-3)

Compound 1-2 (880 mg, 3.0 mmol) obtained in Step 2 was dissolved in ethanol (15 mL) and acetic acid (15 mL), and iron (660 mg, 12 mmol) was added thereto at room temperature. The mixture was refluxed for 3 hours, and a saturated sodium hydrogencarbonate aqueous solution was added thereto. Insoluble compounds were filtered and removed, and the filtrate was extracted with chloroform, dried over anhydrous magnesium sulfate. After filtration, the solvent in the filtrate was removed in vacuo. The residue was purified by silica gel column chromatography (methanol/chloroform = 0/100 to 1/99), and thus the title Compound 1-3 (468 mg, 59%) was obtained as a yellow solid substance.
$^1$H-NMR (DMSO-$d_6$) $\delta$: 8.12 (1H, s), 7.86 (1H, d, J = 2.0 Hz), 7.77 (1H, d, J = 8.8 Hz), 7.58 (1H, d, J = 2.1 Hz), 7.24 (1H, dd, J = 8.8, 2.0 Hz), 7.17 (1H, d, J = 3.3 Hz), 6.68 (1H, dd, J = 3.3, 2.1 Hz), 6.03 (2H, s), 3.96 (3H, s).
ESI-MS (m/z) 269 $[M+H]^+$.

Step 4

7-(furan-2-yl)-2-phenyl-2H-[1,2,3]triazolo[4,5-f]quinoline-9-carboxylic acid methyl ester (Compound 1)

Aniline (0.49 mL, 5.36 mmol) was dissolved in 3 mol/L hydrochloric acid (5 mL). To this, sodium nitrite (339 mg, 4.91 mmol) dissolved in water (5 mL) was added dropwise with ice cooling. After the mixture was stirred at 0˚C for 1 hour, sodium acetate (1.1 g, 13.4 mmol) dissolved in water (10 mL) was added thereto dropwise, and the mixture was stirred at 0˚C for 10 minutes. To the mixture, Compound 1-3 (1.2 g, 4.46 mmol) obtained in Step 3 suspended in ethanol (24 mL) was added, and the mixture was stirred at 0˚C for 2 hours. The precipitate was filtered and collected, and thus a crude product of 6-amino-2-(furan-2-yl)-5-phenylazoquinoline-4-carboxylic acid methyl ester (Compound 1-4) was obtained.

The crude product of Compound 1-4 obtained above was dissolved in pyridine (25 mL) and water (25 mL), and copper sulfate (2.85 g, 17.8 mmol) was added thereto at room temperature. After the mixture was refluxed for 4 hours and cooled to room temperature, 1 mol/L hydrochloric acid was added thereto. Then the aqueous phase was extracted with chloroform, dried over anhydrous magnesium sulfate, and filtered, and the solvent in the filtrate was removed in vacuo. The residue was purified by silica gel column chromatography (ethyl acetate/hexane = 1/9 to 3/7), and thus the title Compound 1 (567 mg, 2 steps: 34%) was obtained as a red-orange solid substance.
$^1$H-NMR ($CDCl_3$) $\delta$: 8.32 (2H, dd, J = 8.6, 1.3 Hz), 8.11 (1H, d, J = 9.5 Hz), 8.03 (1H, d, J = 9.2 Hz), 8.01 (1H, s), 7.65 (1H, dd, J = 1.8, 0.7 Hz), 7.57 (2H, td, J = 8.5, 6.8 Hz), 7.46 (1H, tt, J = 6.8, 1.3 Hz), 7.30 (1H, dd, J = 3.7, 0.7 Hz), 6.63 (1H, dd, J = 3.7, 1.8 Hz), 4.20 (3H, s).

ESI-MS (m/z) 371 $[M+H]^+$.

Example 2

Step 1

7-(furan-2-yl)-2-phenyl-2H-[1,2,3]triazolo[4,5-f]quinoline-9-carboxylic acid (Compound 133)

Compound 1 (790 mg, 2.14 mmol) obtained in Step 4 of Example 1 was dissolved in THF (21 mL) and a 5 mol/L sodium hydroxide aqueous solution (4.3 mL, 21.4 mmol) was added thereto at room temperature. The mixture was refluxed for 12 hours and then cooled to room temperature. To the mixture, 1 mol/L hydrochloric acid (25 mL) was added, and the aqueous phase was extracted with ethyl acetate and dried over anhydrous magnesium sulfate. After filtration, the solvent in the filtrate was removed in vacuo. The residue was reslurried with a mixed solvent of ethyl acetate and hexane (volume ratio 1:1), the precipitate was filtered and collected, and thus the title Compound 133 (610 mg, 80%) was obtained as a red-orange solid substance.
$^1$H-NMR (300 MHz, DMSO-$d_6$) δ: 6.77 (1H, dd, J = 3.3, 1.8 Hz), 7.48 (1H, d, J = 3.3 Hz), 7.58 (1H, t, J = 7.5 Hz), 7.71 (2H, dd, J = 7.8, 7.5 Hz), 7.99 (1H, d, J = 1.8 Hz), 7.99 (1H, d, J = 9.6 Hz), 8.10 (1H, s), 8.28 (1H, d, J = 9.6 Hz), 8.30 (2H, d, J = 7.8 Hz).
ESI-MS (m/z) 357 $[M+H]^+$.

Step 2

7-(furan-2-yl)-2-phenyl-2H-[1,2,3]triazolo[4,5-f]quinoline-9-carboxylic acid methyl amide (Compound 2)

Compound 133 (45 mg, 0.13 mmol) obtained in Step 1 was dissolved in DMF (0.63 mL). To this were added a 2.0 mol/L methylamine-THF solution (0.095 mL, 0.19 mmol), 1-ethyl-3-(3-dimethylaminopropyl)carbodiimide hydrochloride (29 mg, 0.15 mmol) and 1-hydroxybenzotriazole monohydrate (5.0 mg, 0.038 mmol) at room temperature. Then, the mixture was stirred at room temperature for 3 hours. After addition of water, the precipitate was filtered and collected, and thus the title Compound 2 (19 mg, 41%) was obtained as a white solid substance.
$^1$H-NMR (DMSO-$d_6$) δ: 8.73 (1H, d, J = 4.8 Hz), 8.31-8.26 (2H, m), 8.02-7.99 (2H, m), 7.71 (2H, d, J = 7.9 Hz), 7.58 (1H, t, J = 7.3 Hz), 7.50 (1H, d, J = 3.3 Hz), 6.77 (1H, dd, J = 3.3, 1.8 Hz), 3.02 (3H, d, J = 4.8 Hz).
ESI-MS (m/z) 370 $[M+H]^+$.

Example 3

7-(furan-2-yl)-2-phenyl-2H-[1,2,3]triazolo[4,5-f]quinoline-9-carboxylic acid dimethyl amide (Compound 3)

In a similar manner to Step 2 of Example 2, from Compound 133 (45 mg, 0.13 mmol) obtained in Step 1 of Example 2 and a 50% dimethylamine-THF solution (17 mg, 0.19 mmol), the title Compound 3 (25 mg, 51%) was obtained as a white solid substance.
$^1$H-NMR (DMSO-$d_6$) δ: 8.32-8.24 (3H, m), 8.04-7.97 (3H, m), 7.71 (2H, t, J = 7.7 Hz), 7.59 (1H, d, J = 7.7 Hz), 7.50 (1H, d, J = 3.7 Hz), 6.77 (1H, dd, J = 3.7, 1.8 Hz), 3.28 (3H, s), 2.78 (3H, s).
ESI-MS (m/z)- 384 $[M+H]^+$.

Example 4

Step 1

6-amino-2-chloroquinoline-4-carboxylic acid methyl ester (Compound 4-1)

In a similar manner to Step 3 of Example 1, from Compound 1-1 (10 g, 37 mmol) obtained in Step 1 of Example 1, the title Compound 4-1 (6.9 g, 78%) was obtained as an orange solid substance.
$^1$H-NMR (DMSO-$d_6$) δ: 7. 71 (1H, d, J = 9.0 Hz), 7. 71 (1H, s), 7.53 (1H, d, J = 2.5 Hz), 7.27 (1H, dd, J = 9.0, 2.5 Hz), 6.09 (2H, s), 3.94 (3H, s).
ESI-MS (m/z) 237 $[M+H]^+$.

Step 2

7-chloro-2-phenyl-2H[1,2,3]triazolo[4,5-f]quinoline-9-carbo xylic acid methyl ester (Compound 4)

In a similar manner to Step 4 of Example 1, from Compound 4-1 (434 mg, 1.84 mmol) obtained in Step 1, the title Compound 4 (75 mg, 12%) was obtained as a white solid substance.
$^1$H-NMR ($CDCl_3$) δ: 8.31 (2H, dd, J = 7.9, 1.6 Hz), 8.17 (1H, d, J = 9.2 Hz), 7.95 (1H, d, J = 9.2 Hz), 7.60-7.48 (4H, m), 4.19 (3H, s).
ESI-MS (m/z) 339 $[M+H]^+$.

Example 5

7-(4-methanesulfonylaminophenyl)-2-phenyl-2H[1,2,3]triazolo [4,5-f]quinoline-9-carboxylic acid methyl ester (Compound 5)

Compound 4 (70 mg, 0.21 mmol) obtained in Step 2 of Example 4 was dissolved in 1,4-dioxane (1.6 mL) and water (0.4 mL). To the mixture, under nitrogen atmosphere, N-[4-(4,4,5,5-tetramethyl-1,3,2-dioxaborolan-2-yl)phenyl]-methanesulfonamide (74 mg, 0.25 mmol), cesium carbonate (202 mg, 0.62 mmol), and a 1,1'- [bis (diphenylphosphino) ferrocene] - palladium(II) chloride dichloromethane adduct (8.5 mg, 0.010 mmol) were added at room temperature. The mixture was then refluxed for 4 hours. The mixture was cooled to room temperature and filtered through a Celite pad. Water was added thereto, and the precipitate was filtered and collected. Thus the title Compound 5 (91 mg, 93%) was obtained as a white solid substance.
$^1$H-NMR (DMSO-$d_6$) δ: 10.14 (1H, s), 8.45 (1H, s), 8.35-8.26 (5H, m), 8.08 (1H, d, J = 9.2 Hz), 7.72 (2H, t, J = 7.9 Hz), 7.59 (1H, t, J = 7.9 Hz), 7.40 (2H, d, J = 8.8 Hz), 4.16 (3H, s), 3.11 (3H, s).
ESI-MS (m/z) 474 $[M+H]^+$.

Example 6

7-(4-methanesulfonylaminophenyl)-2-phenyl-2H[1,2,3]triazolo [4,5-f]quinoline-9-carboxylic acid (Compound 6)

Compound 5 (48 mg, 0.10 mmol) obtained in Example 5 was dissolved in acetonitrile (1 mL) and a 5 mol/L sodium hydroxide aqueous solution (0.2 mL, 1.0 mmol) was added thereto at room temperature. After refluxed for 3.5 hours, the mixture was cooled to room temperature, and 1 mol/L hydrochloric acid was added thereto. The precipitate was filtered and collected, and thus the title Compound 6 (46 mg, quantitative yield) was obtained as an orange solid substance.
$^1$H-NMR (DMSO-$d_6$) δ: 10.13 (1H, s), 8.36-8.30 (6H, m), 8.06 (1H, d, J = 9.2 Hz), 7.72 (2H, t, J = 7.5 Hz), 7.59 (1H, t, J = 7.5 Hz), 7.39 (2H, d, J = 8.6 Hz), 3.11 (3H, s).
ESI-MS (m/z) 460 $[M+H]^+$.

Example 7

7-(4-methanesulfonylmethylaminophenyl)-2-phenyl-2H[1,2,3]-triazolo[4,5-f]quinoline-9-carboxylic acid methyl ester (Compound 7)

Compound 5 (90 mg, 0.19 mmol) obtained in Example 5 and methyl iodide (0.060 mL, 0.95 mmol) were dissolved in DMF (1.9 mL). To this, 60% by weight sodium hydride (11 mg, 0.76 mmol) was added under ice-cooling. Then, the mixture was stirred at room temperature for 45 minutes. After addition of water, the precipitate was filtered and collected, and thus the title Compound 7 (90 mg, 98%) was obtained as a white solid substance.
ESI-MS (m/z) 488 $[M+H]^+$.

Example 8

7-(4-methanesulfonylmethylaminophenyl)-2-phenyl-2H[1,2,3]-triazolo[4,5-f]quinoline-9-carboxylic acid (Compound 8)

In a similar manner to Example 6, from Compound 7 (90 mg, 0.19 mmol) obtained in Example 7, the title Compound 8 (82 mg, 94%) was obtained as a white solid substance.
$^1$H-NMR (DMSO-$d_6$) δ: 8.43 (1H, s), 8.40 (2H, d, J = 8.8 Hz), 8.33-8.29 (3H, m), 8.07 (1H, d, J = 9.5 Hz), 7.71 (2H, t, J = 7.9 Hz), 7.62-7.56 (3H, m), 3.32 (3H, s), 3.02 (3H, s).

ESI-MS (m/z) 474 $[M+H]^+$.

Example 9

N-[7-(4-methanesulfonylaminophenyl)-2-phenyl-2H-[1,2,3]-triazolo[4,5-f]quinoline-9-carbonyl]methanesulfonamide (Compound 9)

Compound 6 (20 mg, 0.044 mmol) obtained in Example 6 was dissolved in DME (0.45 mL). To this, 1,1'-carbonyldiimidazole (28 mg, 0.174 mmol) was added, and then the mixture was stirred at room temperature for 1 hour. To the mixture, DBU (0.032 mL, 0.22 mmol) and methanesulfonamide (21 mg, 0.22 mmol) were added, and the mixture was further stirred at 60˚C for 1 hour. Then, the mixture was cooled to room temperature, and water was added thereto. The precipitate was filtered, collected and dried, and thus the title Compound 9 (22 mg, 94%) was obtained as a yellow solid substance.
$^1$H-NMR (DMSO-$d_6$) δ: 12.66 (1H, s), 10.13 (1H, s), 8.39-8.34 (6H, m), 8.08 (1H, d, J = 9.5 Hz), 7.67 (2H, t, J = 7.7 Hz), 7.57 (1H, t, J = 7.1 Hz), 7.41 (2H, d, J = 8.4 Hz), 3.59 (3H, s), 3.11 (3H, s).
ESI-MS (m/z) 537 $[M+H]^+$.

Example 10

Step 1

N-methyl-N-[2-(4,4,5,5-tetramethyl-1,3,2-dioxaborolan-2-yl) phenyl]methanesulfonamide (Compound 10-1)

N-[2-(4,4,5,5-tetramethyl-1,3,2-dioxaborolan-2-yl)-phenyl] methanesulfonamide (215 mg, 1.0 mmol) and methyl iodide (0.31 mL, 5.0 mmol) were dissolved in DMF (3.3 mL). To this, 60% by weight sodium hydride (60 mg, 1.5 mmol) was added under ice-cooling. The mixture was stirred at room temperature for 2 hours, and water was added thereto. The aqueous phase was extracted with ethyl acetate, dried over anhydrous magnesium sulfate. After filtration, the solvent in the filtrate was removed in vacuo. The residue was purified by silica gel column chromatography (ethyl acetate/hexane = 1/9 to 7/3), and thus the title Compound 10-1 (147 mg, 64%) was obtained.
ESI-MS (m/z) 228 $[M-H-C_6H_{10}]^-$.

Step 2

7-(2-methanesulfonylmethylaminophenyl)-2-phenyl-2H[1,2,3]-triazolo[4,5-f]quinoline-9-carboxylic acid methyl ester (Compound 10)

In a similar manner to Example 5, from Compound 4 (17 mg, 0.049 mmol) obtained in Step 2 of Example 4 and Compound 10-1 (20 mg, 0.087 mmol) obtained in Step 1, the title Compound 10 (19 mg, 80%) was obtained.
$^1$H-NMR ($CDCl_3$) δ: 8.33 (2H, d, J = 7.7 Hz), 8.14 (1H, d, J = 9.2 Hz), 8:04 (1H, d, J = 9.2 Hz), 7.92 (1H, s), 7.78-7.75 (1H, m), 7.63-7.43 (7H, m), 4.20 (3H, s), 3.24 (3H, s), 2.79 (3H, s).
ESI-MS (m/z) 488 $[M+H]^+$.

Example 11

7-(2-methanesulfonylmethylaminophenyl)-2-phenyl-2H[1,2,3]-triazolo[4,5-f]quinoline-9-carboxylic acid (Compound 11)

In a similar manner to Example 6, from Compound 10 (19 mg, 0.039 mmol) obtained in Step 2 of Example 10, the title Compound 11 (17 mg, 64%) was obtained.
$^1$H-NMR (DMSO-$d_6$) δ: 8.33-8.27 (3H, m), 8.06 (1H, d, J = 9.5 Hz), 7.99 (1H, s), 7.84 (1H, dd, J = 7.0, 2.2 Hz), 7.77-7.52 (6H, m), 3.26 (3H, s), 2.93 (3H, s).
ESI-MS (m/z) 472 $[M-H]^-$.

Example 12

Step 1

N-[4-(4,4,5,5-tetramethyl-1,3,2-dioxaborolan-2-yl)benzoyl]-methanesulfonamide (Compound 12-1)

In a similar manner to Example 9, from 4-(4,4,5,5-tetramethyl-1,3,2-dioxaborolan-2-yl)benzoic acid (200 mg, 0. 81 mmol), the title Compound 12-1 (121 mg, 46%) was obtained.
$^1$H-NMR ($CDCl_3$) δ: 8.54 (1H, s), 7.93 (2H, d, J = 8.3 Hz), 7.81 (2H, d, J = 8.3 Hz), 3.44 (3H, s), 1.36 (12H, s).
ESI-MS (m/z) 326 $[M+H]^+$.

Step 2

7-(4-methanesulfonylaminocarbonylphenyl)-2-phenyl-2H[1,2,3] triazolo[4,5-f]quinoline-9-carboxylic acid methyl ester (Compound 12)

In a similar manner to Example 4, from Compound 4 (16 mg, 0.047 mmol) obtained in Step 2 of Example 4 and Compound 12-1 (18 mg, 0.057 mmol) obtained in Step 1, the title Compound 12 (9.0 mg, 39%) was obtained.
$^1$H-NMR (DMSO-$d_6$) δ: 8.85-8.89 (1H, m), 8.66 (1H, d, J = 7.9 Hz), 8.59 (1H, s), 8.37 (1H, d, J = 9.6 Hz), 8.29 (2H, d, J = 7.9 Hz), 8.15 (1H, d, J = 9.6 Hz), 8.10 (1H, d, J=8.6Hz), 7.67-7.81 (3H, m), 7.61 (1H, d, J = 7.3 Hz), 5.32 (1H, s), 3.43 (3H, s), 3.31 (3H, s).

Example 13

7-(4-methanesulfonylaminocarbonylphenyl)-2-phenyl-2H[1,2,3] triazolo[4,5-f]quinoline-9-carboxylic acid (Compound 13)

In a similar manner to Example 6, from Compound 12 (9.0 mg, 0.018 mmol) obtained in Step 2 of Example 12, the title Compound 13 (6.8 mg, 78%) was obtained.
$^1$H-NMR (DMSO-$d_6$) δ: 8.57-8.50 (3H, m), 8.38-8.28 (3H, m), 8.20-8.09 (3H, m), 7.73 (2H, t, J = 7.5 Hz), 7.60 (1H, t, J = 7.5 Hz), 3.42 (3H, s).
ESI-MS (m/z) 486 $[M-H]^-$.

Example 14

Step 1

N-[4-(4,4,5,5-tetramethyl-1,3,2-dioxaborolan-2-yl)phenyl]-benzenesulfonamide (Compound 14-1)

4-(4,4,5,5-tetramethyl-1,3,2-dioxaborolan-2-yl)-aniline (440 mg, 2.0 mmol) and triethylamine (0.42 mL, 3.0 mmol) were dissolved in DMF (4.0 mL). To this, benzenesulfonamide (0.31 mL, 2.4 mmol) was added at room temperature. Then, the mixture was stirred for 1 hour. After addition of water, the precipitate was filtered and collected, and thus the title Compound 14-1 (520 mg, 72%) was obtained as a white solid substance.
$^1$H-NMR (DMSO-$d_6$) δ: 10.54 (1H, s), 7.78 (2H, dd, J = 6.8, 1.5 Hz), 7.48-7.63 (5H, m), 7.12 (2H, d, J = 8.6 Hz), 1.24 (12H, s) .

Step 2

7-(4-benzenesulfonylaminophenyl)-2-phenyl-2H[1,2,3]triazolo [4,5-f]quinoline-9-carboxylic acid methyl ester (Compound 14)

In a similar manner to Example 5, from Compound 4 (24 mg, 0.071 mmol) obtained in Step 2 of Example 4 and Compound 14-1 (31 mg, 0.086 mmol) obtained in Step 1, the title Compound 14 (30 mg, 79%) was obtained.
ESI-MS (m/z) 536 $[M+H]^+$.

Example 15

7-(4-benzenesulfonylaminophenyl)-2-phenyl-2H[1,2,3]triazolo [4,5-f]quinoline-9-carboxylic acid (Compound 15)

In a similar manner to Example 6, from Compound 14 (30 mg, 0.056 mmol) obtained in Step 2 of Example 14, the title Compound 15 (34 mg, quantitative yield) was obtained.
$^1$H-NMR (DMSO-$d_6$) δ: 10.67 (1H, s), 8.20-8.34 (5H, m), 8.02 (1H, d, J = 9.2 Hz), 7.85 (2H, dd, J = 8.1, 1.5 Hz), 7.71 (2H, dd, J = 7.7, 7.7 Hz), 7.54-7.65 (5H, m), 7.30 (2H, d, J = 8.8 Hz).
ESI-MS (m/z) 522 $[M+H]^+$.

Example 16

Step 1

N-[3-(4,4,5,5-tetramethyl-1,3,2-dioxaborolan-2-yl)benzoyl]-methanesulfonamide (Compound 16-1)

In a similar manner to Example 9, from 3-(4,4,5,5-tetramethyl-1,3,2-dioxaborolan-2-yl)benzoic acid (200 mg, 0.81 mmol), the title Compound 16-1 (129 mg, 50%) was obtained. $^1$H-NMR (DMSO-$d_6$) δ: 8.56 (1H, s), 8.15 (1H, s), 7.99-8.08 (2H, m), 7.53 (1H, dd, J = 7.7 Hz), 3.44 (3H, s), 1.37 (12H, s).

Step 2

7-(3-methanesulfonylaminocarbonylphenyl)-2-phenyl-2H[1,2,3] triazolo[4,5-f]quinoline-9-carboxylic acid methyl ester (Compound 16)

In a similar manner to Example 5, from Compound 4 (33 mg, 0.098 mmol) obtained in Step 2 of Example 4 and Compound 16-1 (38 mg, 0.12 mmol) obtained in Step 1, the title Compound 16 (36 mg, 71%) was obtained.
$^1$H-NMR ($CDCl_3$) δ: 8.33 (2H, d, J = 8.6 Hz), 8.02-8.19 (5H, m), 7.54-7.64 (5H, m), 7.43-7.52 (3H, m), 7.31 (2H, d, J = 8.6 Hz), 4.22 (3H, s), 3.26 (3H, s).

Example 17

7-(3-methanesulfonylaminocarbonylphenyl)-2-phenyl-2H[1,2,3] triazolo[4,5-f]quinoline-9-carboxylic acid (Compound 17)

In a similar manner to Example 6, from Compound 16 (16 mg, 0.031 mmol) obtained in Step 2 of Example 16, the title Compound 17 (15 mg, quantitative yield) was obtained.
$^1$H-NMR (DMSO-$d_6$) δ: 8.86 (1H, s), 8.66 (1H, d, J = 7.7 Hz), 8.54 (1H, s), 8.29-8.37 (3H, m), 8.13 (1H, d, J = 9.5 Hz), 8.09 (1H, d, J = 7.3 Hz), 7.76 (1H, t, J = 7.9 Hz), 7.73 (2H, dd, J = 7.7, 7.7 Hz), 7.60 (1H, t, J = 7.1 Hz), 3.44 (3H, s).
ESI-MS (m/z) 488 $[M+H]^+$.

Example 18

7-ethoxy-2-phenyl-2H[1,2,3]triazolo[4,5-f]quinoline-9-carboxylic acid (Compound 18)

Compound 4 (52 mg, 0.015 mmol) obtained in Step 2 of Example 4 was dissolved in ethanol (1.5 mL) and a 5 mol/L sodium hydroxide aqueous solution (0.30 mL, 1.5 mmol) was added thereto at room temperature. After refluxed for 4 hours, the mixture was cooled to room temperature, 1 mol/L hydrochloric acid was added thereto, and the precipitate was filtered and collected. The obtained solid substance was purified by preparative thin-layer chromatography (methanol/chloroform = 1/4), and thus the title Compound 18 (15 mg, 29%) was obtained.
$^1$H-NMR (DMSO-$d_6$) δ: 14.02 (1H, s), 8.29 (1H, d, J = 7.7 Hz), 8.23 (1H, d, J = 9.2 Hz), 7.82 (1H, d, J = 9.2 Hz), 7.70 (2H, dd, J = 7.7, 7.7 Hz), 7.57 (1H, t, J = 7.7 Hz), 7.23 (1H, s), 4.54 (2H, q, J = 7.0 Hz), 1.42 (3H, t, J = 7.0 Hz).
ESI-MS (m/z) 335 $[M+H]^+$.

Example 19

7-chloro-2-phenyl-2H[1,2,3]triazolo[4,5-f]quinoline-9-carboxylic acid (Compound 19)

Compound 4 (16 mg, 0.047 mmol) obtained in Step 2 of Example 4 was dissolved in acetonitrile (0.34 mL) and a 5.0 mol/L sodium hydroxide aqueous solution (0.34 mL, 1. 7 mmol) was added thereto at room temperature. After refluxed for 6.5 hours, the mixture was cooled to room temperature, and 1 mol/L hydrochloric acid was added thereto. The precipitate was filtered and collected, and thus the title Compound 19 (11 mg, 73%) was obtained.
$^1$H-NMR (DMSO-$d_6$) δ: 10.54 (1H, s), 7.74-7.84 (2H, m), 7.48-7.63 (4H, m), 7.12 (2H, d, J = 8.6 Hz).

Example 20

7-(4-methanesulfonylmethylaminocarbonylphenyl)-2-phenyl-2H[1,2,3]triazolo[4,5-f]quinoline-9-carboxylic acid (Compound 20)

In a similar manner to Example 5, from Compound 19 (11 mg, 0.034 mmol) obtained in Example 19 and N-methyl-N-methanesulfonyl-4-(4,4,5,5-tetramethyl-1,3,2-dioxaborolan-2-yl)benzamide (14 mg, 0.041 mmol), the title Compound 20 (21 mg, 90%) was obtained.
$^1$H-NMR (DMSO-$d_6$) δ: 8.52 (1H, s), 8.51 (2H, d, J = 8.3 Hz), 8.27-8.40 (3H, m), 8.11 (1H, d, J = 9.3 Hz), 7.86 (2H, d, J = 8.6 Hz), 7.73 (2H, dd, J = 8.6, 7.4 Hz), 7.60 (1H, t, J = 7.4 Hz), 3.49 (3H, s), 3.25 (3H, s).
ESI-MS (m/z) 502 $[M+H]^+$.

Example 21

7-(4-benzenesulfonylmethylaminophenyl)-2-phenyl-2H[1,2,3]-triazolo[4,5-f]quinoline-9-carboxylic acid methyl ester (Compound 21)

In a similar manner to Example 5, from Compound 4 (30 mg, 0.088 mmol) obtained in Step 2 of Example 4 and N-methyl-N-[4-(4,4,5,5-tetramethyl-1,3,2-dioxaborolan-2-yl) phenyl]benzenesulfonamide (63 mg, 0.11 mmol), a crude product of the title Compound 21 (80 mg) was obtained.
ESI-MS (m/z) 384 $[M+H]^+$.

Example 22

7-(4-benzenesulfonylmethylaminophenyl)-2-phenyl-2H[1,2,3]-triazolo[4,5-f]quinoline-9-carboxylic acid (Compound 22)

In a similar manner to Example 6, from a crude product of Compound 21 (80 mg) obtained in Example 21, the title Compound 22 (41 mg, 2 steps: 86%) was obtained.
$^1$H-NMR (DMSO-$d_6$) δ: 8.25-8.44 (6H, m), 8.07 (1H, d, J = 9.5 Hz), 7.67-7.78 (3H, m), 7.54-7.66 (5H, m), 7.34 (2H, d, J = 8.8 Hz), 3.23 (3H, s).
ESI-MS (m/z) 536 $[M+H]^+$.

Example 23

7-(3-methanesulfonylmethylaminocarbonylphenyl)-2-phenyl-2H[1,2,3]triazolo[4,5-f]quinoline-9-carboxylic acid (Compound 23)

In a similar manner to Example 5, from Compound 19 (30 mg, 0.092 mmol) obtained in Example 19 and N-methyl-N-methanesulfonyl-3-(4,4,5,5-tetramethyl-1,3,2-dioxaborolan -2-yl)benzamide (63 mg, 0.11 mmol), the title Compound 23 (28 mg, 61%) was obtained.
$^1$H-NMR (DMSO-$d_6$) δ: 8.55-8.65 (3H, m), 8.34 (1H, d, J = 9.5 Hz), 8.32 (2H, d, J = 7.7 Hz), 8.12 (1H, d, J = 9.5 Hz), 7.68-7.83 (4H, m), 7.60 (1H, t, J = 7.1 Hz), 3.51 (3H, s), 3.27 (3H, s).
ESI-MS (m/z) 502 $[M+H]^+$.

Example 24

7-(3-benzenesulfonylmethylaminocarbonylphenyl)-2-phenyl-2H[1,2,3]triazolo[4,5-f]quinoline-9-carboxylic acid (Compound 24)

In a similar manner to Example 5, from Compound 19 (30 mg, 0.092 mmol) obtained in Example 19 and N-methyl-N-benzenesulfonyl-3-(4,4,5,5-tetramethyl-1,3,2-dioxaborolan -2-yl)benzamide (111 mg, 0.11 mmol), the title Compound 24 (18 mg, 35%) was obtained.
$^1$H-NMR (DMSO-$d_6$) δ: 8.55-8.63 (1H, m), 8.46-8.52 (2H, m), 8.33 (1H, d, J = 9.3 Hz), 8.32 (2H, d, J = 7.6 Hz), 8.10 (1H, d, J = 9.3 Hz), 7.99-8.06 (2H, m), 7.63-7.80 (7H, m), 7.60 (1H, t, J = 7.3 Hz), 3.37 (3H, s).
ESI-MS (m/z) 564 $[M+H]^+$.

Example 25

7-(3-benzenesulfonylaminocarbonylphenyl)-2-phenyl-2H[1,2,3] triazolo[4,5-f]quinoline-9-carboxylic acid methyl ester (Compound 25)

In a similar manner to Example 5, from Compound 4 (50 mg, 0.15 mmol) obtained in Step 2 of Example 4 and N-benzenesulfonyl-3-(4,4,5,5-tetramethyl-1,3,2-dioxaborolan -2-yl)benzamide (69 mg, 0.18 mmol), the title Compound 25 (62 mg, 74%) was obtained.
$^1$H-NMR ($CDCl_3$) δ: 12.38 (1H, s), 8.82 (1H, s), 8.58 (1H, s), 8.33 (2H, d, J = 7.3 Hz), 8.32 (1H, s), 8.12-8.19 (3H, m), 8.06-8.11 (2H, m), 7.20-7.70 (7H, m), 4.23 (3H, s).

Example 26

7-(3-benzenesulfonylaminocarbonylphenyl)-2-phenyl-2H[1,2,3] triazolo[4,5-f]quinoline-9-carboxylic acid (Compound 26)

In a similar manner to Example 6, from Compound 25 (60 mg, 0.11 mmol) obtained in Example 25, the title Compound 26 (58 mg, quantitative yield) was obtained.
$^1$H-NMR (DMSO-$d_6$) δ: 8.82 (1H, s), 8.63 (1H, d, J = 7.7 Hz), 8.54 (1H, s), 8.34 (1H, d, J = 9.2 Hz), 8.32 (2H, d, J = 8.4 Hz), 8.12 (1H, d, J = 9.2 Hz), 8.07 (2H, d, J = 8.1 Hz), 8.00 (1H, d, J = 7.3 Hz), 7.55-7.79 (8H, m).
ESI-MS (m/z) 550 $[M+H]^+$.

Example 27

7-(4-benzenesulfonylaminocarbonylphenyl)-2-phenyl-2H[1,2,3] triazolo[4,5-f]quinoline-9-carboxylic acid methyl ester (Compound 27)

In a similar manner to Example 5, from Compound 4 (50 mg, 0.15 mmol) obtained in Step 2 of Example 4 and N-benzenesulfonyl-4-(4,4,5,5-tetramethyl-1,3,2-dioxaborolan -2-yl)benzamide (69 mg, 0.18 mmol), the title Compound 27 (56 mg, 67%) was obtained.
$^1$H-NMR (DMSO-$d_6$) δ: 12.27 (1H, s), 8.28-8.38 (4H, m), 8.10-8.19 (6H, m), 8.08 (1H, d, J = 9.5 Hz), 7.43-7.70 (6H, m), 4.21 (3H, s).

Example 28

7-(4-benzenesulfonylaminocarbonylphenyl)-2-phenyl-2H[1,2,3] triazolo[4,5-f]quinoline-9-carboxylic acid (Compound 28)

In a similar manner to Example 6, from Compound 27 (55 mg, 0.098 mmol) obtained in Example 27, the title Compound 28 (55 mg, quantitative yield) was obtained.
$^1$H-NMR (DMSO-$d_6$) δ: 8.52 (1H, s), 8.50 (2H, d, J = 8.4 Hz), 8.34 (1H, d, J = 9.2 Hz), 8.31 (2H, d, J = 7.7 Hz), 8.22 (1H, d, J = 9.2 Hz), 8.08 (2H, d, J = 8.4 Hz), 8.05 (2H, d, J = 7.7 Hz), 7.56-7.77 (6H, m).

Example 29

7-(4-benzenesulfonylmethylaminocarbonylphenyl)-2-phenyl-2H[ 1,2,3]triazolo[4,5-f]quinoline-9-carboxylic acid (Compound 29)

In a similar manner to Example 5, from Compound 19 (29 mg, 0.089 mmol) obtained in Example 19 and N-methyl-N-benzenesulfonyl-4-(4,4,5,5-tetramethyl-1,3,2-dioxaborolan-2-yl)benzamide (72 mg, 0.18 mmol), the title Compound 29 (13 mg, 26%) was obtained.
$^1$H-NMR (DMSO-$d_6$) δ: 8.52 (1H, s), 8.48 (2H, d, J = 8.2 Hz), 8.35 (1H, d, J = 9.5 Hz), 8.32 (2H, d, J = 7.9 Hz), 8.11 (1H, d, J = 9.5 Hz), 8.03 (2H, d, J = 8.2 Hz), 7.70-7.84 (7H, m), 7.60 (1H, t, J = 7.2 Hz), 3.35 (3H, s).

Example 30

7-[1-(tert-butoxycarbonyl)-1H-pyrrol-2-yl]-2-phenyl-2H[1,2, 3]triazolo[4,5-f]quinoline-9-carboxylic acid methyl ester (Compound 30)

In a similar manner to Example 5, from Compound 4 (40 mg, 0.12 mmol) obtained in Step 2 of Example 4 and 1-(tert-butoxycarbonyl)-1H-pyrrol-2-ylboronic acid (30 mg, 0.14 mmol), the title Compound 30 (55 mg, quantitative yield) was obtained.
$^1$H-NMR ($CDCl_3$) δ: 8.33 (2H, d, J = 8.6 Hz), 8.11 (1H, d, J = 9.5 Hz), 8.00 (1H, d, J = 9.5 Hz), 7.72 (1H, s), 7.57 (2H, dd, J = 8.6, 7.5 Hz), 7.45-7.50 (2H, m), 6.65 (1H, dd, J = 3.5, 1.6 Hz), 6.33 (1H, d, J = 3.5 Hz), 4.19 (3H, s), 1.31 (9H, s).

Example 31

2-phenyl-7-(1H-pyrrol-2-yl)-2H[1,2,3]triazolo[4,5-f]-quinoline-9-carboxylic acid methyl ester (Compound 31)

Compound 30 (56 mg, 0.012 mmol) obtained in Example 30 was dissolved in dichloromethane (1.0 mL), and then trifluoroacetic acid (0.1 mL) was added thereto at room temperature. After the mixture was stirred for 4.5 hours, the solvent was removed in vacuo. The residue was purified by silica gel column chromatography (chloroform), and thus the title Compound 31 (34 mg, 77%) was obtained.
$^1$H-NMR ($CDCl_3$) δ: 9.81 (1H, s), 8.30 (2H, dd, J = 7.9, 1.5 Hz), 8.30 (1H, d, J = 9.3.Hz), 8.06 (1H, d, J = 9.3 Hz), 7.83 (1H, s), 7.56 (1H, dd, J = 7.9, 7.3 Hz), 7.45 (1H, tt; J = 7.3, 1.5 Hz), 7.00-7.03 (1H, m), 6.88-6.93 (1H, m), 6.36 (1H, dd, J = 6.2, 2.7 Hz), 4.19 (3H, s).

Example 32

2-phenyl-7-(1H-pyrrol-2-yl)-2H[1,2,3] triazolo[4,5-f]-quinoline-9-carboxylic acid (Compound 32)

In a similar manner to Example 6, from Compound 31 (34 mg, 0.091 mmol) obtained in Example 31, the title Compound 32 (30 mg, 92%) was obtained.
$^1$H-NMR (DMSO-$d_6$) δ: 11.74 (1H, s), 8.29 (2H, d, J = 7.7 Hz), 8.25 (1H, d, J = 9.5 Hz), 8.10 (1H, s), 7. 95 (1H, d, J = 9.5 Hz), 7.71 (2H, dd, J = 7.7, 7.3 Hz), 7.58 (1H, t, J = 7.3 Hz), 7.10-7.16 (1H, m), 7.01-7.07 (1H, m), 6.22-6.28 (1H, m).

Example 33

2-phenyl-7-(1-methyl-1H-pyrrol-2-yl)-2H[1,2,3]triazolo[4,5-f]quinoline-9-carboxylic acid methyl ester (Compound 33)

Compound 31 (26 mg, 0.070 mmol) obtained in Example 31 and methyl iodide (0.022 mL, 0.35 mmol) were dissolved in DMF (0.35 mL), and sodium hydride (60% by weight, 5.6 mg, 0.14 mmol) was added thereto. The mixture was stirred at room temperature for 3 hours, and water was added thereto. The aqueous phase was extracted with chloroform, dried over anhydrous magnesium sulfate. After filtration, the solvent in the filtrate was removed in vacuo. The residue was purified by silica gel column chromatography (chloroform), and thus the title Compound 33 (19 mg, 69%) was obtained as a white solid substance.
$^1$H-NMR (DMSO-$d_6$) δ: 8.20-8.33 (3H, m), 8.08 (1H, s), 7.97 (1H, d, J = 9.6 Hz), 7.71 (2H, dd, J = 7.8 Hz), 7.58 (1H, t, J = 7.8 Hz), 7.04-7.10 (2H, m), 6.18 (1H, dd, J = 4.0, 2.6 Hz), 4.18 (3H, s).

Example 34

2-phenyl-7-(1-methyl-1H-pyrrol-2-yl)-2H[1,2,3]triazolo[4,5-f]quinoline-9-carboxylic acid (Compound 34)

In a similar manner to Example 6, from Compound 33 (18 mg, 0.047 mmol) obtained in Example 33, the title Compound 34 (17 mg, 96%) was obtained.
$^1$H-NMR (DMSO-$d_6$) δ: 8.30 (2H, d, J = 8.6 Hz), 8.25 (1H, d, J = 9.3 Hz), 8.08 (1H, s), 7.97 (1H, d, J = 9.3 Hz), 7.71 (2H, dd, J = 8.6, 7.3 Hz), 7.57 (1H, t, J = 7.3 Hz), 7.04-7.10 (2H, m), 6.18 (1H, dd, J = 4.0, 2.6 Hz), 4.18 (3H, s).

Example 35

Step 1

6-amino-5-bromo-2-chloroquinoline-4-carboxylic acid methyl ester (Compound 35-1)

Compound 4-1 (6.93 g, 29.3 mmol) obtained in Step 1 of Example 4 and tetrabutylammonium bromide (5.66 g, 17.6 mmol) were dissolved in DMF (300 mL), and N-bromosuccinimide (5.47 g, 30.8 mmol) was added thereto at room temperature. The mixture was stirred for 6 hours, and insoluble compounds were filtered and removed. The filtrate was extracted with chloroform, dried over anhydrous magnesium sulfate. After filtration, the solvent in the filtrate was removed in vacuo. The residue was purified by silica gel column chromatography (ethyl acetate/hexane = 1/9 to 9/1), and thus the title Compound 35-1 (5.29 g, 57%) was obtained as a brown solid substance.
$^1$H-NMR ($CDCl_3$) δ: 7.77 (1H, d, J = 8.9 Hz), 7.61 (1H, s), 7.47 (1H, d, J = 8.9 Hz), 6.33 (2H, s), 3.92 (3H, s).

Step 2

6-benzamido-5-bromo-2-chloroquinoline-4-carboxylic acid methyl ester (Compound 35-2)

Compound 35-1 (400 mg, 1.27 mmol) obtained in Step 1, triethylamine (0.71 mL, 5.09 mmol), and 4-dimethylaminopyridine (12 mg, 0.098 mmol) were dissolved in dichloromethane (6.4 mL), and benzoyl chloride (0.37 mL, 3.19 mmol) was added thereto. The mixture was stirred for 15 hours, and aqueous ammonia was added thereto. The aqueous phase was extracted with chloroform, dried over anhydrous magnesium sulfate. After filtration, the solvent in the filtrate was removed in vacuo. The residue was purified by silica gel column chromatography (ethyl acetate/hexane = 1/9 to 1/1), and thus the title Compound 35-2 (541 mg, quantitative yield) was obtained.
$^1$H-NMR ($CDCl_3$) δ: 9.03 (1H, d, J = 9.3 Hz), 8.75 (1H, s), 8.13 (1H, d, J = 9.3 Hz), 7.98 (2H, dd, J = 8.3, 1.3 Hz), 7.36-7.68 (4H, m), 4.03 (3H, s).

Step 3

7-chloro-2-phenyloxazolo[5,4-f]quinoline-9-carboxylic acid (Compound 35)

Compound 35-2 (400 mg, 0.95 mmol) obtained in Step 2 was dissolved in acetonitrile (200 mL), a sodium hydroxide aqueous solution (2.0 mol/L, 14 mL, 28 mmol) was added thereto, and the mixture was irradiated by a high pressure mercury lamp (435 W) for 3.5 hours. After the completion of the reaction, hydrochloric acid was added to the mixture for adjusting the pH to 1, and the solvent was removed. The precipitate was filtered and collected, and thus the title Compound 35 (187 mg, 61%) was obtained as a white solid substance.
$^1$H-NMR (DMSO-$d_6$) δ: 8.33 (1H, d, J = 9.2 Hz), 8.22-8.29 (2H, m), 8.07 (1H, d, J = 9.2 Hz), 7.99 (1H, s), 7.67-7.73 (3H, s). ESI-MS (m/z) 325 $[M+H]^+$.

Example 36

7-chloro-2-phenyloxazolo[5,4-f]quinoline-9-carboxylic acid methyl ester (Compound 36)

Compound 35-2 (85 mg, 0.20 mmol) obtained in Step 2 of Example 35 was dissolved in acetonitrile (40 mL), a sodium hydroxide aqueous solution (2.0 mol/L, 0.40 mL, 0.80 mmol) was added thereto, and the mixture was irradiated by a high pressure mercury lamp (435 W) for 4.5 hours. Hydrochloric acid was added to the mixture for adjusting the pH to 1, and the solvent was removed. The precipitate was filtered and collected, and thus the title Compound 36 (50 mg, 74%) was obtained as a white solid substance.
$^1$H-NMR ($CDCl_3$) δ: 8.23-8.29 (2H, m), 8.18 (1H, d, J = 8.8 Hz), 8.08 (1H, d, J = 8.8 Hz), 7.73 (1H, s), 7.55-7.61 (3H, m), 4.20 (3H, s).

Example 37

7-(furan-2-yl)-2-phenyloxazolo[5,4-f]quinoline-9-carboxylic acid methyl ester (Compound 37)

In a similar manner to Step 2 of Example 1, from Compound 36 (20 mg, 0.059 mmol) obtained in Example 36 and 2-tributylstannylfuran (0.023 mL, 0.071 mmol), the title Compound 37 (19 mg, 89%) was obtained.
$^1$H-NMR ($CDCl_3$) δ: 8.20-8.31 (2H, m), 8.07-8.20 (3H, m), 7.65 (1H, s), 7.51-7.61 (3H, m), 7.29 (1H, d, J = 3.3 Hz), 6.61 (1H, dd, J = 3.3, 1.5 Hz), 4.20 (3H, s).

Example 38

7-(furan-2-yl)-2-phenyloxazolo[5,4-f]quinoline-9-carboxylic acid (Compound 38)

In a similar manner to Example 6, from Compound 37 (19 mg, 0.051 mmol) obtained in Example 37, the title Compound 38 (17 mg, 91%) was obtained.
$^1$H-NMR ($CDCl_3$) δ: 8.51 (1H, d, J = 9.2 Hz), 8.30-8.38 (2H, m), 8.25 (1H, s), 8.18 (1H, d, J = 9.2 Hz), 7.81 (1H, s), 7.73 (1H, d, J = 1.8 Hz), 7.40-7.52 (3H, m), 6.69 (1H, dd, J = 3.7, 1.8 Hz).
ESI-MS (m/z) 357 $[M+H]^+$.

Example 39

2,7-diphenyloxazolo[5,4-f]quinoline-9-carboxylic acid methyl ester (Compound 39)

In a similar manner to Example 5, from Compound 36 (23 mg, 0.068 mmol) obtained in Example 36 and phenylboronic acid (10 mg, 0.082 mmol), the title Compound 39 (19 mg, 75%) was obtained.
$^1$H-NMR ($CDCl_3$) δ: 8.18-8.30 (6H, m), 8.14 (1H, d, J = 9.2 Hz), 7.47-7.61 (3H, m), 7.44 (2H, dd, J = 7.7, 7.3 Hz), 7.34 (1H, tt, J = 7.3, 1.6 Hz), 4.22 (3H, s).

Example 40

2,7-diphenyloxazolo[5,4-f]quinoline-9-carboxylic acid (Compound 40)

In a similar manner to Example 6, from Compound 39 (19 mg, 0.051 mmol) obtained in Example 39, the title Compound 40 (13 mg, '69%) was obtained.
$^1$H-NMR (DMSO-$d_6$) δ: 8.47 (1H, s), 8.38 (2H, d, J = 8.1 Hz), 8.23-8.31 (3H, m), 8.19 (1H, d, J = 8.8 Hz), 7.65-7.73 (3H, m), 7.52-7.63 (3H, m).
ESI-MS (m/z) 367 $[M+H]^+$.

Example 41

2-phenyl-7-(thiophen-2-yl)oxazolo[5,4-f]quinoline-9-carboxylic acid methyl ester (Compound 41)

In a similar manner to Step 2 of Example 1, from Compound 36 (25 mg, 0.074 mmol) obtained in Example 36 and 2- (tributylstannyl) thiophene (0.030 mL, 0. 096 mmol), the title Compound 41 (29 mg, quantitative yield) was obtained.
ESI-MS (m/z) 387 $[M+H]^+$.

Example 42

2-phenyl-7-(thiophen-2-yl)oxazolo[5,4-f]quinoline-9-carboxylic acid (Compound 42)

In a similar manner to Example 6, from Compound 41 (29 mg, 0.075 mmol) obtained in Example 41, the title Compound 42 (16 mg, 57%) was obtained.
$^1$H-NMR (DMSO-$d_6$) δ: 8.16-8.28 (3H, m), 8.03 (2H, d, J = 9.2 Hz), 7.79 (1H, d, J = 5.1 Hz), 7.59-7.72 (4H, m), 7.26 (1H, dd, J = 5.1, 3.7 Hz).
ESI-MS (m/z) 373 $[M+H]^+$.

Example 43

7-(3-methanesulfonylaminophenyl)-2-phenyloxazolo[5,4-f]-quinoline-9-carboxylic acid (Compound 43)

In a similar manner to Example 5, from Compound 35 (28 mg, 0.086 mmol) obtained in Step 3 of Example 35 and 3-(methanesulfonamide)phenylboronic acid (22 mg, 0.10 mmol), the title Compound 43 (37 mg, 93%) was obtained.
$^1$H-NMR (DMSO-$d_6$) δ: 9.95 (1H, s), 8.39 (1H, s), 8.12-8.37 (5H, m), 8.08 (1H, d, J = 7.9 Hz), 7.66-7.73 (3H, m), 7.56 (1H, t, J = 7.8 Hz), 7.42 (1H, d, J = 7.6 Hz), 3.08 (3H, s).
ESI-MS (m/z) 460 $[M+H]^+$.

Example 44

7-(oxazol-2-yl)-2-phenyloxazolo[5,4-f]quinoline-9--carboxylic acid methyl ester (Compound 44)

In a similar manner to Step 2 of Example 1, from Compound 36 (7.1 mg, 0.021 mmol) obtained in Example 36 and 2-(tributylstannyl)oxazole (0.005 mL, 0.025 mmol), the title Compound 44 (3.6 mg, 46%) was obtained.
$^1$H-NMR ($CDCl_3$) δ: 8.61 (1H, s), 8.33 (1H, d, J = 9.2Hz), 8.25-8.32 (2H, m), 8.21 (1H, d, J = 9.2Hz), 7.93 (1H, s), 7.55-7.62 (3H, m), 7.42 (1H, s), 4.22 (3H, s).

Example 45

7-(oxazol-2-yl)-2-phenyloxazolo[5,4-f]quinoline-9--carboxylic acid (Compound 45)

In a similar manner to Example 6, from Compound 44 (3.6 mg, 0.0097 mmol) obtained in Example 44, the title Compound 45 (1.9 mg, 32%) was obtained.
$^1$H-NMR (DMSO-$d_6$) δ: 8.43-8.50 (2H, m), 8.31-8.38 (1H, m), 8.20-8.31 (3H, m), 7.64-7.75 (3H, m), 7.58-7.63 (1H, m).
ESI-MS (m/z) 358 $[M+H]^+$.

Example 46

7-(4-methanesulfonylaminophenyl)-2-phenyloxazolo[5,4-f]-quinoline-9-carboxylic acid (Compound 46)

In a similar manner to Example 5, from Compound 35 (30 mg, 0.092 mmol) obtained in Step 3 of Example 35 and N-[4-(4,4,5,5-tetramethyl-1,3,2-dioxaborolan-2-yl)phenyl]-methanesulfonamide (33 mg, 0.11 mmol), the title Compound 46 (38 mg, 89%) was obtained as an orange solid substance.
$^1$H-NMR (DMSO-$d_6$) δ: 10.13 (1H, s), 8.42 (1H, s), 8.36 (2H, d, J=8.8Hz), 8.21-8.30 (3H, m), 8.15 (1H, d, J=9.2Hz), 7.65-7.74 (3H, m), 7.40 (2H, d, J = 8.8 Hz), 3.11 (3H, s).
ESI-MS (m/z) 460 $[M+H]^+$.

Example 47

7-(2-methanesulfonylaminophenyl)-2-phenyloxazolo[5,4-f]-quinoline-9-carboxylic acid (Compound 47)

In a similar manner to Example 5, from Compound 35 (30 mg, 0.092 mmol) obtained in Step 3 of Example 35 and 2-(methanesulfonamide)phenylboronic acid (24 mg, 0.11 mmol), the title Compound 47 (21 mg, 50%) was obtained as a pale yellow solid substance.
$^1$H-NMR (DMSO-$d_6$) δ: 12.13 (1H, s), 8.49 (1H, s), 8.37 (1H, d, J = 9.2 Hz), 8.22-8.31 (2H, m), 8.21 (1H, d, J = 7.3 Hz), 8.10 (1H, d, J = 9.2 Hz), 7. 63-7.75 (4H, m), 7. 57 (1H, dd, J = 8.1, 7.0 Hz), 7.36 (1H, dd, J = 8.1, 7.3 Hz), 3.11 (3H, s).
ESI-MS (m/z) 458 $[M-H]^-$.

Example 48

7-{3-[methoxy(methyl)carbamoyl]phenyl}-2-phenyloxazolo[5,4-f]quinoline-9-carboxylic acid (Compound 48)

In a similar manner to Example 5, from Compound 35 (18 mg, 0.055 mmol) obtained in Step 3 of Example 35 and N-methoxy-N-methyl-3-(4,4,5,5-tetramethyl-1,3,2-dioxaborola n-2-yl)benzamide (19 mg, 0.066 mmol), the title Compound 48 (15 mg, 60%) was obtained as an ocher solid substance.
$^1$H-NMR (DMSO-$d_6$) δ: 8.51-8.62 (2H, m), 8.48 (1H, dd, J = 7.7, 1.1 Hz), 8.23-8.32 (3H, m), 8.21 (1H, d, J = 8.8 Hz),

7.63-7.77 (4H, m), 7.44-7.62 (1H, m), 3.62 (3H, s), 3.34 (3H, s).
ESI-MS (m/z) 452 [M-H]$^-$.

Example 49

7-{4-[methoxy(methyl)carbamoyl]phenyl}-2-phenyloxazolo[5,4-f]quinoline-9-carboxylic acid (Compound 49)

In a similar manner to Example 5, from Compound 35 (30 mg, 0.092 mmol) obtained in Step 3 of Example 35 and N-methoxy-N-methyl-4-(4,4,5,5-tetramethyl-1,3,2--dioxaborolan-2-yl)benzamide (32 mg, 0.11 mmol), the title Compound 49 (35 mg, 72%) was obtained as an ocher solid substance.
$^1$H-NMR (DMSO-$d_6$) δ: 8.52 (1H, s), 8.44 (2H, d, J = 8.1 Hz), 8.23-8.34 (3H, m), 8.21 (1H, d, J = 8.8 Hz), 7.80 (2H, d, J = 8.4 Hz), 7.63-7.74 (3H, m), 3.61 (3H, s), 3.32 (3H, s).
ESI-MS (m/z) 454 [M+H]$^+$.

Example 50

7-(4-carboxyphenyl)-2-phenyloxazolo[5,4-f]quinoline-9--carboxylic acid (Compound 50)

In a similar manner to Example 5, from Compound 35 obtained in Step 3 of Example 35 and 4-(4,4,5,5--tetramethyl-1,3,2-dioxaborolan-2-yl)benzoic acid (18 mg, 0.074 mmol), the title Compound 50 (12 mg, 49%) was obtained.
$^1$H-NMR (DMSO-$d_6$) δ: 8.55 (1H, s), 8.50 (2H, d, J = 8.4 Hz), 8.31 (1H, d, J = 9.2 Hz), 8.23-8.30 (2H, m), 8.22 (1H, d, J = 9.2 Hz), 8.14 (2H, d, J = 8.4 Hz), 7.66-7.73 (3H, m).

Example 51

7-[2-(3-ethylureido)phenyl]-2-phenyloxazolo[5,4-f]quinoline -9-carboxylic acid (Compound 51)

In a similar manner to Example 5, from Compound 35 (40 mg, 0.12 mmol) obtained in Step 3 of Example 35 and 1-ethyl-3-[2-(4,4,5,5-tetramethyl-1,3,2-dioxaborolan-2-yl)p henyl]urea (43 mg, 0.15 mmol), the title Compound 51 (26 mg, 47%) was obtained as a brown solid substance.
$^1$H-NMR (DMSO-$d_6$) δ: 10.47 (1H, s), 8.44 (1H, d, J = 9.2 Hz), 8.34 (1H, d, J = 9.2 Hz), 8.24-8.31 (3H, m), 8.18 (1H, dd, J = 8.4, 0.7 Hz), 7. 92 (1H, dd, J = 8. 1, 1.5 Hz), 7.65-7.74 (3H, m), 7.45-7.60 (1H, m), 7.43 (1H, ddd, J = 8.4, 7.5, 1.5 Hz), 7.14 (1H, ddd, J = 8.1, 7.5, 0.7 Hz), 3.12 (2H, q, J = 7.1 Hz), 1.06 (3H, t, J = 7.1 Hz).
ESI-MS (m/z) 453 [M+H]$^+$.

Example 52

7-(3-carboxyphenyl)-2-phenyloxazolo[5,4-f]quinoline-9--carboxylic acid (Compound 52)

In a similar manner to Example 5, from Compound 35 (40 mg, 0.12 mmol) obtained in Step 3 of Example 35 and 3-(4,4,5,5-tetramethyl-1,3,2-dioxaborolan-2-yl)benzoic acid (36 mg, 0.15 mmol), the title Compound 52 (23 mg, 46%) was obtained as a brown solid substance.
ESI-MS (m/z) 411 [M+H]$^+$.

Example 53

7-(4-benzenesulfonylaminophenyl)-2-phenyloxazolo[5,4-f]-quinoline-9-carboxylic acid (Compound 53)

In a similar manner to Example 5, from Compound 35 (40 mg, 0.12 mmol) obtained in Step 3 of Example 35 and N-[3-(4,4,5,5-tetramethyl-1,3,2-dioxaborolan-2-yl)phenyl]-benzenesulfonamide (53 mg, 0.15 mmol), the title Compound 53 (50 mg, 78%) was obtained as an ocher solid substance.
$^1$H-NMR (DMSO-$d_6$) δ: 10.58 (1H, s), 8.19-8.24 (2H, m), 8.15 (2H, d, J = 8.8 Hz), 8.07 (1H, d, J = 8.8 Hz), 7.96 (1H, d, J = 8.8 Hz), 7.79-7.87 (3H, m), 7.53-7.67 (6H, m), 7.27 (2H, d, J = 8.8 Hz).
ESI-MS (m/z) 520 [M-H]$^-$.

Example 54

Step 1

5-bromo-2-chloro-6-(4-fluorobenzamido)quinoline-4--carboxylic acid methyl ester (Compound 54-1)

In a similar manner to Step 2 of Example 35, from Compound 35-1 (100 mg, 0.32 mmol) obtained in Step 1 of Example 35 and 4-fluorobenzoyl chloride (0.10 mL, 0.79 mmol), the title Compound 54-1 (102 mg, 73%) was obtained.
$^1$H-NMR (DMSO-$d_6$) δ: 10.48 (1H, s), 8.10-8.18 (3H, m), 8.08 (1H, d, J = 9.2 Hz), 7.93 (1H, s), 7.43 (2H, dd, J = 8.8, 8.8 Hz), 4.00 (3H, s).
ESI-MS (m/z) 437 $[M+H]^+$.

Step 2

7-chloro-2-(4-fluorophenyl)oxazolo[5,4-f]quinoline-9--carboxylic acid (Compound 54)

In a similar manner to Step 3 of Example 35, from Compound 54-1 (100 mg, 0.23 mmol) obtained in Step 1, the title Compound 54 (50 mg, 64%) was obtained.
$^1$H-NMR (DMSO-$d_6$) δ: 8.18-8.34 (3H, m), 8.05 (1H, d, J = 8.9 Hz), 7.98 (1H, s), 7.48-7.58 (2H, dd, J = 8.6, 8.6 Hz).
ESI-MS (m/z) 341 $[M-H]^-$.

Example 55

2-(4-fluorophenyl)-7-[4-(methanesulfonamide)phenyl]oxazolo[ 5,4-f]quinoline-9-carboxylic acid (Compound 55)

In a similar manner to Example 5, from Compound 54 (48 mg, 0.14 mmol) obtained in Step 2 of Example 54 and N-[4-(4,4,5,5-tetramethyl-1,3,2-dioxaborolan-2-yl)phenyl]-methanesulfonamide (42 mg, 0.17 mmol), the title Compound 55 (62 mg, 93%) was obtained.
$^1$H-NMR (DMSO-$d_6$) δ: 10.12 (1H, s), 8.42 (1H, s), 8.35 (2H, d, J = 8.8 Hz), 8.22-8.33 (3H, m), 8.15 (1H, d, J = 8.8 Hz), 7.56 (2H, dd, J = 9.2, 9.0 Hz), 7.40 (2H, d, J = 8.8 Hz), 3.11 (3H, s).
ESI-MS (m/z) 478 $[M+H]^+$.

Example 56

Step 1

5-bromo-2-chloro-6-(2-fluorobenzamido)quinoline-4--carboxylic acid methyl ester (Compound 56-1)

In a similar manner to Step 2 of Example 35, from Compound 35-1 (100 mg, 0.32 mmol) obtained in Step 1 of Example 35 and 2-fluorobenzoyl chloride (0.10 mL, 0.79 mmol), the title Compound 56-1 (140 mg, quantitative yield) was obtained.
$^1$H-NMR (DMSO-$d_6$) δ: 10.38 (1H, s), 8.22 (1H, d, J = 9.3 Hz), 8.13 (1H, d, J = 9.3 Hz), 7.93 (1H, s), 7.85 (1H, td, J = 7.7, 1.8 Hz), 7.49-7.61 (1H, m), 7.47-7.55 (1H, m), 7.35-7.44 (1H, m), 3.98 (3H, s).

Step 2

7-chloro-2-(2-fluorophenyl)oxazolo[5,4-f]quinoline-9--carboxylic acid (Compound 56)

In a similar manner to Step 3 of Example 35, from Compound 56-1 (140 mg, 0.32 mmol) obtained in Step 1, the title Compound 56 (38 mg, 35%) was obtained.
$^1$H-NMR (DMSO-$d_6$) δ: 8.36 (1H, d, J = 9.2 Hz), 8.24 (1H, td, J = 7.5, 1.3 Hz), 8.08 (1H, d, J = 9.2 Hz), 7.99 (1H, s), 7.69-7.79 (1H, m), 7.47-7.58 (2H, m).
ESI-MS (m/z) 343 $[M+H]^+$.

Example 57

2-(2-fluorophenyl)-7-[4-(methanesulfonamide)phenyl]oxazolo[ 5,4-f]quinoline-9-carboxylic acid (Compound 57)

In a similar manner to Example 5, from Compound 56 (37 mg, 0.11 mmol) obtained in Step 2 of Example 56 and N-[4-(4,4,5,5-tetramethyl-1,3,2-dioxaborolan-2-yl)phenyl]me thanesulfonamide (32 mg, 0.13 mmol), the title Compound 57 (46 mg, 89%) was obtained.
$^1$H-NMR (DMSO-$d_6$) δ: 10.12 (1H, s), 8.42 (1H, s), 8.36 (2H, d, J = 8.8 Hz), 8.30 (1H, d, J = 9.2 Hz), 8.26 (1H, td, J = 7.5, 1.8 Hz), 8.17 (1H, d, J = 9.2 Hz), 7.69-7.78 (1H, m), 7.45-7.64 (2H, m), 7.40 (2H, d, J = 8.8 Hz), 3.11 (3H, s).
ESI-MS (m/z) 478 $[M+H]^+$.

Example 58

Step 1

5-bromo-2-chloro-6-(3-fluorobenzamido)quinoline-4--carboxylic acid methyl ester (Compound 58-1)

In a similar manner to Step 2 of Example 35, from Compound 35-1 (100 mg, 0.32 mmol) obtained in Step 1 of Example 35 and 3-fluorobenzoyl chloride (0.10 mL, 0.79 mmol), the title Compound 58-1 (140 mg, quantitative yield) was obtained.
ESI-MS (m/z) 437 $[M+H]^+$.

Step 2

7-chloro-2-(3-fluorophenyl)oxazolo[5,4-f]quinoline-9--carboxylic acid (Compound 58)

In a similar manner to Step 3 of Example 35, from Compound 58-1 (140 mg, 0.32 mmol) obtained in Step 1, the title Compound 58 (65 mg, 59%) was obtained.
ESI-MS (m/z) 343 $[M+H]^+$.

Example 59

2-(3-fluorophenyl)-7-[4-(methanesulfonamide)phenyl]oxazolo[ 5,4-f]quinoline-9-carboxylic acid (Compound 59)

In a similar manner to Example 5, from Compound 58 (62 mg, 0.18 mmol) obtained in Step 2 of Example 58 and N-[4-(4,4,5,5-tetramethyl-1,3,2-dioxaborolan-2-yl)phenyl]me thanesulfonamide (65 mg, 0.22 mmol), the title Compound 59 (84 mg, 98%) was obtained.
$^1$H-NMR (DMSO-$d_6$) δ: 10.13 (1H, s), 8.42 (1H, s), 8.35 (2H, d, J = 8.8 Hz), 8.25 (1H, d, J = 9.2 Hz), 8.14 (1H, d, J = 9.2 Hz), 8.09 (1H, d, J = 7.7 Hz), 7.92-7.98 (1H, m), 7.73 (1H, ddd, J = 8.1, 7.7, 5.7 Hz), 7.54 (1H, ddd, J = 8.1, 7.7, 2.0 Hz), 7.40 (2H, d, J = 8.8 Hz), 3.11 (3H, s).
ESI-MS (m/z) 478 $[M+H]^+$.

Example 60

Step 1

5-bromo-2-chloro-6-(3-methoxybenzamido)quinoline-4--carboxylic acid methyl ester (Compound 60-1)

In a similar manner to Step 2 of Example 35, from Compound 35-1 (100 mg, 0.32 mmol) obtained in Step 1 of Example 35 and 3-methoxybenzoyl chloride (0.11 mL, 0.79 mmol), the title Compound 60-1 (51 mg, 36%) was obtained.
$^1$H-NMR ($CDCl_3$) δ: 9.02 (1H, d, J = 9.2 Hz), 8.74 (1H, s), 8.13 (1H, d, J = 9.2 Hz), 7.29-7.56 (4H, m), 7.03-7.18 (1H, m), 4.03 (3H, s), 3.91 (3H, s).

Step 2

7-chloro-2-(3-methoxyphenyl)oxazolo[5,4-f]quinoline-9--carboxylic acid methyl ester (Compound 60)

In a similar manner to Step 3 of Example 35, from Compound 60-1 (50 mg, 0.11 mmol) obtained in Step 1, the

title Compound 60 (19 mg, 49%) was obtained.
ESI-MS (m/z) 369 $[M+H]^+$.

Example 61

2-(3-methoxyphenyl)-7-[4-(methanesulfonamide)phenyl]oxazolo [5,4-f]quinoline-9-carboxylic acid methyl ester (Compound 61)

In a similar manner to Example 5, from Compound 60 (18 mg, 0.051 mmol) obtained in Step 2 of Example 60 and N-[4-(4,4,5,5-tetramethyl-1,3,2-dioxaborolan-2-yl)phenyl]me thanesulfonamide (18 mg, 0.061 mmol), the title Compound 61 (8 mg, 32%) was obtained.
ESI-MS (m/z) 504 $[M+H]^+$.

Example 62

2-(3-methoxyphenyl)-7-[4-(methanesulfonamide)phenyl]oxazolo [5,4-f]quinoline-9-carboxylic acid (Compound 62)

In a similar manner to Step 1 of Example 2, from Compound 61 (8 mg, 0.016 mmol) obtained in Example 61, the title Compound 62 (4 mg, 53%) was obtained.
$^1$H-NMR (DMSO-$d_6$) δ: 10.05 (1H, s), 8.27 (2H, d, J = 8.4 Hz), 8.10 (1H, d, J = 9.2 Hz), 8.01 (1H, d, J = 9.2 Hz), 7.95 (1H, s), 7.81 (1H, d, J = 7.7 Hz), 7.72-7.76 (1H, m), 7.55 (1H, dd, J = 8.1, 7.7 Hz), 7.37 (2H, d, J = 8.4 Hz), 7.22 (1H, dd, J = 8.1, 2.6 Hz), 3.89 (3H, s), 3.09 (3H, s).
ESI-MS (m/z) 490 $[M+H]^+$.

Example 63

Step 1

5-bromo-2-chloro-6-(3-chlorobenzamido)quinoline-4--carboxylic acid methyl ester (Compound 63-1)

In a similar manner to Step 2 of Example 35, from 6-amino-2-chloro-5-bromoquinoline-4-carboxylic acid methyl ester (130 mg, 0.41 mmol) and 3-chlorobenzoyl chloride (180 mg, 1.03 mmol), the title Compound 63-1 (180 mg, 96%) was obtained.
$^1$H-NMR ($CDCl_3$) δ: 8.96 (1H, d, J = 9.2 Hz), 8.66 (1H, s), 8.14 (1H, d, J = 9.2 Hz), 7.99-7.95 (1H, m), 7.87-7.81 (1H, m), 7.64-7.58 (1H, m), 7.50 (1H, t, J = 7.7 Hz), 7.48 (1H, s), 4.04 (3H, s).

Step 2

7-chloro-2-(3-chlorophenyl)oxazolo[5,4-f]quinoline-9--carboxylic acid (Compound 63)

In a similar manner to Step 3 of Example 35, from Compound 63-1 (180 mg, 0.40 mmol) obtained in Step 1, the title Compound 63 (93 mg, 65%) was obtained.
$^1$H-NMR (DMSO-$D_6$) δ: 8.34 (1H, d, J = 9.2 Hz), 8.24-8.17 (2H, m), 8.09 (1H, d, J = 8.8 Hz), 8.01 (1H, s), 7.82-7.67 (2H, m).
ESI-MS (m/z) 357 $[M-H]^-$.

Example 64

7-chloro-2-(3-fluorophenyl)-2H-[1,2,3]triazolo[4,5-f]-quinoline-9-carboxylic acid methyl ester (Compound 64)

In a similar manner to Step 4 of Example 1, from 3-fluoroaniline (0.244 mL, 2.54 mmol) and Compound 4-1 (500 mg, 2.11 mmol) obtained in Step 1 of Example 4, the title Compound 64 (123 mg, 16%) was obtained.
$^1$H-NMR ($CDCl_3$) δ: 8.17-8.09 (2H, m), 8.04 (1H, ddd, J = 9.5, 2.2, 2.2 Hz), 7.96 (1H, d, J = 9.5 Hz), 7.63 (1H, s), 7.55 (1H, ddd, J = 8.2, 8.2, 5.9 Hz), 7.19 (1H, ddd, J = 8.2, 2.2, 0.9 Hz), 4.19 (3H, s).
ESI-MS (m/z) 357 $[M+H]^+$.

Example 65

7-(furan-2-yl)-2-(3-fluorophenyl)-2H-[1,2,3]triazolo[4,5-f] quinoline-9-carboxylic acid methyl ester (Compound 65)

In a similar manner to Step 2 of Example 1, from Compound 64 (120 mg, 0.34 mmol) obtained in Example 64 and 2-tributylstannylfuran (0.13 mL, 0.40 mmol), the title Compound 65 (55 mg, 42%) was obtained.
$^1$H-NMR ($CDCl_3$) δ: 8.15-8.00 (5H, m), 7.65 (1H, dd, J = 1.8, 0.7 Hz), 7.53 (1H, ddd, J = 8.2, 8.2, 5.9 Hz), 7.30 (1H, dd, J = 3.3, 0.7 Hz), 7.16 (1H, ddd, J = 8.2, 2.5, 0.7 Hz), 6.63 (1H, dd, J = 3.3, 1.8 Hz), 4.20 (3H, s).

Example 66

7-(furan-2-yl)-2-(3-fluorophenyl)-2H-[1,2,3]triazolo[4,5-f] quinoline-9-carboxylic acid (Compound 66)

In a similar manner to Example 6, from Compound 65 (55 mg, 0.14 mmol) obtained in Example 65, the title Compound 66 (52 mg, 98%) was obtained.
$^1$H-NMR (DMSO-$d_6$) δ: 14.18 (1H, s), 8.29 (1H, d, J = 9.3 Hz), 8.20-8.12 (2H, m), 8.09 (1H, ddd, J=9.8, 2.3, 2.3 Hz), 7.98-8.05 (2H, m), 7.77 (1H, ddd, J = 8.3, 8.3, 6.3 Hz), 7.54-7.40 (2H, m), 6.78 (1H, dd, J = 3.5, 1.8 Hz).
ESI-MS (m/z) 375 $[M+H]^+$.

Example 67

7-(furan-3-yl)-2-(3-fluorophenyl)-2H-[1,2,3]triazolo[4,5-f] quinoline-9-carboxylic acid methyl ester (Compound 67)

In a similar manner to Example 5, from Compound 64 (37 mg, 0.10 mmol) obtained in Example 64 and (furan-3-yl)boronic acid (14 mg, 0.12 mmol), the title Compound 67 (36 mg, 90%) was obtained.
$^1$H-NMR ($CDCl_3$) δ: 8.21 (1H, dd, J = 1.5, 0.7 Hz), 8.16-7.97 (4H, m), 7.77 (1H, s), 7.59-7.49 (2H, m), 7.21-7.12 (1H, m), 7.10 (1H, dd, J = 2.0, 0.7 Hz), 4.20 (3H, s).
ESI-MS (m/z) 389 $[M+H]^+$.

Example 68

7-(furan-3-yl)-2-(3-fluorophenyl)-2H-[1,2,3]triazolo[4,5-f] quinoline-9-carboxylic acid (Compound 68)

In a similar manner to Example 6, from Compound 67 (36 mg, 0.093 mmol) obtained in Example 67, the title Compound 68 (33 mg, 94%) was obtained.
$^1$H-NMR (DMSO-$d_6$) δ: 8.67 (1H, s), 8.27 (1H, d, J = 9.5 Hz), 8.21 (1H, s), 8.16 (1H, dd, J = 8.2, 1.5 Hz), 8.09 (1H, ddd, J = 9.8, 2.2, 2.2 Hz), 8.00 (1H, d, J = 9.5 Hz), 7.88 (1H, dd, J = 1.5, 1.5 Hz), 7.77 (1H, ddd, J = 8.2, 8.2, 6.1 Hz), 7.45 (1H, ddd, J = 8.2, 8.2, 2.2 Hz), 7.29 (1H, d, J = 1.5 Hz).
ESI-MS (m/z) 375 $[M+H]^+$.

Example 69

7-{1-(tert-butoxycarbonyl)-1H-pyrrol-3-yl}-2-(3--fluorophenyl)-2H-[1,2,3]triazolo[4,5-f]quinoline-9--carboxylic acid methyl ester (Compound 69)

In a similar manner to Example 6, from Compound 64 (65 mg, 0.18 mmol) obtained in Example 64 and N-(tert-butoxycarbonyl)-3-(4,4,5,5-tetramethyl-1,3,2--dioxaborolan-2-yl)-1H-pyrrole (64 mg, 0.22 mmol), the title Compound 69 (52 mg, 59%) was obtained.
$^1$H-NMR ($CDCl_3$) δ: 8.17-7.98 (4H, m), 7.82 (1H, s), 7.53 (1H, ddd, J = 8.3, 8.3, 5.9 Hz), 7.38 (1H, dd, J = 3.3, 2.5 Hz), 7.16 (1H, ddd, J = 8.3, 2.5, 0.9 Hz), 6.95 (1H, dd, J = 3.3, 2.0 Hz), 4.20 (3H, s), 1.66 (10H, s).
ESI-MS (m/z) 488 $[M+H]^+$.

Example 70

2-(3-fluorophenyl)-7-(1H-pyrrol-3-yl)-2H-[1,2,3]triazolo[4, 5-f]quinoline-9-carboxylic acid methyl ester (Compound 70)

In a similar manner to Example 31, from Compound 69 (51 mg, 0.11 mmol) obtained in Example 69, the title Compound 70 (33 mg, 82%) was obtained.

$^1$H-NMR (DMSO-$d_6$) δ: 11.35 (1H, s), 8.22 (1H, d, J = 9.2 Hz), 8.16-8.09 (2H, m), 8.02 (1H, dt, J = 9.9, 2.3, 2.3 Hz), 7.96 (1H, d, J = 9.2 Hz), 7.81-7.71 (2H, m), 7.44 (1H, ddd, J = 8.3, 8.3, 2.3 Hz), 6.94-6.86 (2H, m), 4.12 (3H, s).
ESI-MS (m/z) 388 $[M+H]^+$.

Example 71

2-(3-fluorophenyl)-7-(1H-pyrrol-3-yl)-2H-[1,2,3]triazolo[4, 5-f]quinoline-9-carboxylic acid (Compound 71)

In a similar manner to Example 6, from Compound 70 (33 mg, 0.085 mmol) obtained in Example 70, the title Compound 71 (21 mg, 67%) was obtained.
$^1$H-NMR (DMSO-$d_6$) δ: 11.36 (1H, s), 8.22 (1H, d, J = 9.5 Hz), 8.15 (1H, dd, J = 7.9, 1.6 Hz), 8.11-8.05 (2H, m), 7.97 (1H, d, J = 9.5 Hz), 7.84-7.80 (1H, m), 7.76 (1H, ddd, J = 8.2, 8.2, 6.2 Hz), 7.44 (1H, ddd, J = 8.2, 8.2, 2.7 Hz), 6.94-6.88 (2H, m).
ESI-MS (m/z) 374 $[M+H]^+$.

Example 72

7-{1-(tert-butoxycarbonyl)-1H-pyrrol-2-yl}-2-(3--fluorophenyl)-2H-[1,2,3]triazolo[4,5-f]quinoline-9--carboxylic acid methyl ester (Compound 72)

In a similar manner to Example 5, from Compound 64 (62 mg, 0.17 mmol) obtained in Example 64 and 1-(tert--butoxycarbonyl)-1H-pyrrol-2-ylboronic acid (44 mg, 0.21 mmol), the title Compound 72 (57 mg, 67%) was obtained.
$^1$H-NMR ($CDCl_3$) δ: 8.17-7.98 (4H, m), 7.72 (1H, s), 7.54 (1H, ddd, J = 8.3, 8.3, 5.9 Hz), 7.47 (1H, dd, J = 3.1, 1.5 Hz), 7. 17 (1H, ddd, J = 8.0, 8.0, 2.3 Hz), 6.65 (1H, dd, J = 3.1, 1.5 Hz), 6.33 (1H, dd, J = 3.1, 3.1 Hz), 4.19 (3H, s), 1.32 (9H, s).
ESI-MS (m/z) 488 $[M+H]^+$.

Example 73

2-(3-fluorophenyl)-7-(1H-pyrrol-2-yl)-2H-[1,2,3]triazolo[4, 5-f]quinoline-9-carboxylic acid methyl ester (Compound 73)

In a similar manner to Example 31, from Compound 72 (56 mg, 0.12 mmol) obtained in Example 72, the title Compound 73 (41 mg, 93%) was obtained.
$^1$H-NMR ($CDCl_3$) δ: 9.79 (1H, s), 8.12 (1H, dd, J = 8.2, 1.3 Hz), 8.07-8.00 (2H, m), 7.91 (1H, d, J = 9.5 Hz), 7.83 (1H, s), 7.53 (1H, ddd, J = 8.3, 8.3, 6.0 Hz), 7.20-7.11 (1H, m), 7.06-7.01 (1H, m), 6.93-6.89 (1H, m), 6.40-6.34 (1H, m), 4.20 (3H, s).
ESI-MS (m/z) 388 $[M+H]^+$.

Example 74

2-(3-fluorophenyl)-7-(1H-pyrrol-2-yl)-2H-[1,2,3]triazolo[4, 5-f]quinoline-9-carboxylic acid (Compound 74)

In a similar manner to Example 6, from Compound 73 (41 mg, 0.11 mmol) obtained in Example 73, the title Compound 74 (39 mg, 99%) was obtained.
$^1$H-NMR (DMSO-$d_6$) δ: 14.05 (1H, s), 11.74 (1H, s), 8.24 (1H, d, J = 9.5 Hz), 8.15 (1H, dd, J = 8.2, 1.3 Hz), 8.12-8.04 (2H, m), 7.95 (1H, d, J = 9.5 Hz), 7.76 (1H, ddd, J = 8.2, 8.2, 6.1 Hz), 7.44 (1H, ddd, J = 8.2, 8.2, 2.4 Hz), 7.16-7.11 (1H, m), 7.05-7.02 (1H, m), 6.28-6.22 (1H, m).
ESI-MS (m/z) 374 $[M+H]^+$.

Example 75

7-chloro-2-(3-fluorophenyl)-2H-[1,2,3]triazolo[4,5-f]-quinoline-9-carboxylic acid (Compound 75)

In a similar manner to Example 19, from Compound 64 obtained in Example 64, the title Compound 75 (68 mg, quantitative yield) was obtained.
$^1$H-NMR (DMSO-$d_6$) δ: 8.37 (1H, d, J = 9.5 Hz), 8.16 (1H, dd, J = 8.2, 1.6 Hz), 8.09 (1H, ddd, J = 9.5, 2.4, 1.6 Hz), 8.01 (1H, s), 7.98 (1H, d, J = 9.5 Hz), 7.77 (1H, ddd, J = 8.2, 8.2, 6.2 Hz), 7.48 (1H, ddd, J = 8.4, 2.4, 9.5 Hz).
ESI-MS (m/z) 343 $[M+H]^+$.

Example 76

7-(5-carboxy-1H-pyrrol-3-yl)-2-(3-fluorophenyl)-2H-[1,2,3]-triazolo[4,5-f]quinoline-9-carboxylic acid (Compound 76)

Compound 75 (60 mg, 0.18 mmol) obtained in Example 75, 1-(tert-butoxycarbonyl)-2-(methoxycarbonyl)pyrrole-4-boroni c acid pinacol ester (92 mg, 0.26 mmol), and cesium carbonate (171 mg, 0.53 mmol) were dissolved in a mixed solvent of DME and water (4:1, 1.75 mL). To this, a 1,1'-[bis(diphenylphosphino)ferrocene]palladium(II) chloride dichloromethane adduct (13 mg, 0.018 mmol) was added, and the mixture was refluxed for 16 hours. After the mixture was cooled to room temperature, insoluble compounds were filtered and removed, and 4 mol/L hydrochloric acid was added to the filtrate. The precipitate was filtered and collected, and thus the title Compound 76 (35 mg, 48%) was obtained.
$^1$H-NMR (DMSO-$d_6$) δ: 12.27 (1H, s), 8.28-8.21 (2H, m), 8.16 (1H, dd, J = 8.2, 1.6 Hz), 8.08 (1H, ddd, J = 9.9, 2.2, 2.2 Hz), 8.06-7.98 (2H, m), 7.77 (1H, ddd, J = 8.2, 8.2, 3.2 Hz), 7.58 (1H, dd, J = 2.2, 1.6 Hz), 7.45 (1H, ddd, J = 8.2, 8.2, 2.7 Hz).
ESI-MS (m/z) 418 $[M+H]^+$.

Example 77

7-(2-methylfuran-3-yl)-2-(3-fluorophenyl)-2H-[1,2,3]-triazolo[4,5-f]quinoline-9-carboxylic acid methyl ester (Compound 77)

In a similar manner to Example 5, from Compound 64 (49 mg, 0.14 mmol) obtained in Example 64 and 4,4,5,5-tetramethyl-2-(2-methylfuran-3-yl)-1,3,2--dioxaborolane (34 mg, 0.17 mmol), the title Compound 77 (53 mg, 97%) was obtained.
$^1$H-NMR ($CDCl_3$) δ: 8.16-7.97 (4H, m), 7.73 (1H, s), 7.54 (1H, ddd, J = 8.3, 8.3, 5.9 Hz), 7.40 (1H, d, J = 2.0 Hz), 7.16 (1H, ddd, J = 8.3, 8.3, 2.5 Hz), 6.88 (1H, d, J = 2.0 Hz), 4.20 (3H, s), 2.82 (3H, s).
ESI-MS (m/z) 403 $[M+H]^+$.

Example 78

7-(2-methylfuran-3-yl)-2-(3-fluorophenyl)-2H-[1,2,3]-triazolo[4,5-f]quinoline-9-carboxylic acid (Compound 78)

In a similar manner to Example 6, from Compound 77 (51 mg, 0.13 mmol) obtained in Example 77, the title Compound 78 (40 mg, 81%) was obtained.
$^1$H-NMR (DMSO-$d_6$) δ: 14.10 (1H, s), 8.26 (1H, d, J = 9.5 Hz), 8.16 (1H, dd, J = 8.2, 1.8 Hz), 8.09 (1H, ddd, J = 9.9, 2.3, 2.3 Hz), 8.06 (1H, s), 8.00 (1H, d, J = 9.5 Hz), 7.77 (1H, ddd, J = 8.2, 8.2, 6.4 Hz), 7.69 (1H, d, J = 2.2 Hz), 7.45 (1H, ddd, J = 8.2, 8.2, 2.2 Hz), 7.25 (1H, d, J = 2.2 Hz), 2.83 (3H, s).
ESI-MS (m/z) 389 $[M+H]^+$.

Example 79

7-cyano-2-(3-fluorophenyl)-2H-[1,2,3]triazolo[4,5-f]-quinoline-9-carboxylic acid methyl ester (Compound 79)

Compound 64 (52 mg, 0.15 mmol) obtained in Example 64, zinc (2.4 mg, 0.036 mmol), zinc cyanide (26 mg, 0.22 mmol), bis(diphenylphosphino)ferrocene (12 mg, 0.022 mmol), and tris(dibenzylidene acetone)dipalladium(0) (13 mg, 0.015 mmol) were suspended in N,N-dimethylacetamide (1.5 mL), and the mixture was stirred at 100°C for 15 hours. The mixture was cooled to room temperature, and insoluble compounds were filtered and removed. Water was added to the filtrate, and the precipitate was filtered and collected. The obtained solid substance was purified by silica gel column chromatography (chloroform), and thus the title Compound 79 (18 mg, 35%) was obtained.
$^1$H-NMR ($CDCl_3$) δ: 8.22 (1H, d, J = 9.5 Hz), 8.15-8.01 (3H, m), 7.93 (1H, s), 7.60-7.52 (1H, m), 7.21 (1H, ddd, J = 8.2, 8.2, 2.6 Hz), 4.20 (3H, s).
ESI-MS (m/z) 348 $[M+H]^+$.

Example 80

7-cyano-2-(3-fluorophenyl)-2H-[1,2,3]triazolo[4,5-f]-quinoline-9-carboxylic acid (Compound 80)

In a similar manner to Example 79, from Compound 75 (51 mg, 0.15 mmol) obtained in Example 75, the title Compound 80 (15 mg, 31%) was obtained.
$^1$H-NMR (DMSO-$d_6$) δ: 8.28 (1H, d, J = 9.2 Hz), 8.19-8.12 (1H, m), 8.08-8.02 (1H, m), 7.94 (1H, d, J = 9.2 Hz), 7.82

(1H, s), 7.75 (1H, ddd, J = 8.2, 8.2, 6.7 Hz), 7.44 (1H, ddd, J = 8.2, 8.2, 2.3 Hz).
ESI-MS (m/z) 332 $[M-H]^-$.

Example 81

7-chloro-2-(3-chlorophenyl)-2H-[1,2,3]triazolo[4,5-f]-quinoline-9-carboxylic acid methyl ester (Compound 81)

In a similar manner to Step 4 of Example 1, from 3-chloroaniline (0.54 mL, 5.07 mmol) and Compound 4-1 (500 mg, 2.11 mmol) obtained in Step 1 of Example 4 , the title Compound 81 (259 mg, 33%) was obtained.
$^1$H-NMR ($CDCl_3$) δ: 8.34 (1H, dd, J = 1.8, 1.8 Hz), 8.21 (1H, ddd, J = 8.2, 1.8, 1.2 Hz), 8.14 (1H, d, J = 9.2 Hz), 7.96 (1H, d, J = 9.2 Hz), 7.63 (1H, s), 7.51 (1H, dd, J = 8.2, 8.2 Hz), 7.45 (1H, ddd, J = 8.2, 1.8, 1.2 Hz), 4.19 (3H, s).
ESI-MS (m/z) 373 $[M+H]^+$.

Example 82

2-(3-chlorophenyl)-7-(furan-2-yl)-2H-[1,2,3]triazolo[4,5-f] quinoline-9-carboxylic acid methyl ester (Compound 82)

In a similar manner to Step 2 of Example 1, from Compound 81 (100 mg, 0.27 mmol) obtained in Example 81 and 2-tributylstannylfuran (0.10 mL, 0.32 mmol), the title Compound 82 (77 mg, 71%) was obtained.
$^1$H-NMR ($CDCl_3$) δ: 8.34 (1H, dd, J = 2.0, 1.0 Hz), 8.21 (1H, ddd, J = 7.9, 1.6, 1.0 Hz), 8.08 (1H, d, J = 9.5 Hz), 8.02 (1H, d, J = 9.5 Hz), 8.01 (1H, s), 7.65 (1H, dd, J = 1.8, 0.7 Hz), 7.50 (1H, dd, J = 7.9, 7.9 Hz), 7.43 (1H, ddd, J = 7.9, 1.6, 1.0 Hz), 7.30 (1H, dd, J = 3.7, 0.7 Hz), 6.62 (1H, dd, J = 3.7, 1.8 Hz), 4.20 (3H, s).
ESI-MS (m/z) 405 $[M+H]^+$.

Example 83

2-(3-chlorophenyl)-7-(furan-2-yl)-2H-[1,2,3]triazolo[4,5-f] quinoline-9-carboxylic acid (Compound 83)

In a similar manner to Example 6, from Compound 82 (77 mg, 0.19 mmol) obtained in Example 82, the title Compound 83 (74 mg, quantitative yield) was obtained.
$^1$H-NMR (DMSO-$d_6$) δ: 8.32-8.20 (2H, m), 8.12 (1H, d, J = 9.3 Hz), 7.96-7.85 (2H, m), 7.76-7.56 (3H, m), 7.34 (1H, d, J = 3.3 Hz), 6.72 (1H, m).
ESI-MS (m/z) 391 $[M+H]^+$.

Example 84

2-(3-fluorophenyl)-7-(pyrrolidin-1-yl)-2H-[1,2,3]triazolo[4 ,5-f]quinoline-9-carboxylic acid (Compound 84)

Compound 75 (51 mg, 0.15 mmol) obtained in Example 75 was dissolved in pyrrolidine (0.25 mL, 3.0 mmol), and the mixture was stirred at 90˚C for 2 hours. After the mixture was cooled to room temperature, hydrochloric acid was added thereto. The precipitate was filtered and collected, and thus the title Compound 84 (55 mg, 98%) was obtained.
$^1$H-NMR (DMSO-$d_6$) δ: 8.15-8.07 (2H, m), 8.04 (1H, ddd, J = 10.1, 2.2, 2.2 Hz), 7.78-7.68 (2H, m), 7.41 (1H, ddd, J = 8.3, 8.3, 1.2 Hz), 7.04 (1H, m), 3.66-3.58 (4H, m), 2.06-1.98 (4H, m).
ESI-MS (m/z) 378 $[M+H]^+$.

Example 85

2-(3-fluorophenyl)-7-{(trimethylsilyl)ethynyl}-2H-[1,2,3]-triazolo[4,5-f]quinoline-9-carboxylic-acid methyl ester (Compound 85)

Compound 64 (100 mg, 0.28 mmol) obtained in Example 64, trimethylsilyl acetylene (0.12 mL, 0.84 mmol), copper (I) iodide (21 mg, 0.11 mmol), triethylamine (0.16 mL, 1.1 mmol) and a 1,1'-[bis(diphenylphosphino)ferrocene] palladium(II) chloride dichloromethane adduct (82 mg, 0.11 mmol) were suspended in THF (5.6 mL), and the mixture was stirred at room temperature for 2 hours. Insoluble compounds were filtered and removed, the filtrate was purified by silica gel column chromatography (chloroform), and thus the title Compound 85 (120 mg, quantitative yield) was obtained.
$^1$H-NMR ($CDCl_3$) δ: 8.14-7.98 (4H, m), 7.76 (1H, s), 7.53 (1H, ddd, J = 8.2, 8.2, 6.0 Hz), 7.17 (1H, ddd, J = 8.2, 8.2, 2.6 Hz), 4.17 (3H, s), 0.32 (9H, s).

ESI-MS (m/z) 419 $[M+H]^+$.

Example 86

7-ethynyl-2-(3-fluorophenyl)-2H-[1,2,3]triazolo[4,5-f]-quinoline-9-carboxylic acid methyl ester (Compound 86)

Compound 85 (117 mg, 0.28 mmol) obtained in Example 85 was dissolved in THF (5.6 mL). To this, a tetrabutylammoniumfluoride-THF solution (1 mol/L, 0.42 mL, 0.42 mmol) was added, and the mixture was stirred for 1.5 hours. A saturated ammonium chloride aqueous solution was added thereto, and the aqueous phase was extracted with chloroform, dried over anhydrous magnesium sulfate. After filtration, the solvent in the filtrate was removed in vacuo. The residue was purified by silica gel column chromatography (chloroform), and thus the title Compound 86 (77 mg, 80%) was obtained.
$^1$H-NMR ($CDCl_3$) δ: 8.15-8.10 (2H, m), 8.07-7.99 (2H, m), 7.77 (1H, s), 7.54 (1H, ddd, J = 8.3, 8.3, 6.0 Hz), 7.18 (1H, m), 4.19 (3H, s), 3.36 (1H, s).
ESI-MS (m/z) 347 $[M+H]^+$.

Example 87

7-{1-(2-ethoxy-2-oxoethyl)-1H-1,2,3-triazol-4-yl}-2-(3--fluorophenyl)-2H-[1,2,3]triazolo[4,5-f]quinoline-9--carboxylic acid methyl ester (Compound 87)

Compound 86 (77 mg, 0.22 mmol) obtained in Example 86, azidoethyl acetate (29 mg, 0.22 mmol), sodium ascorbate (88 mg, 0.44 mmol), and copper(II) sulfate (35 mg, 0.22 mmol) were dissolved in a mixed solvent of THF and water (2:1, 45 mL), and the mixture was stirred for 13.5 hours. After the completion of the reaction, the mixture was extracted with chloroform, washed with brine, and dried over anhydrous magnesium sulfate. After filtration, the solvent in the filtrate was removed in vacuo. The residue was purified by silica gel column chromatography (chloroform), and thus the title Compound 87 (67 mg, 63%) was obtained.
$^1$H-NMR ($CDCl_3$) δ: 8.53 (1H, s), 8.50 (1H, s), 8.16-8.02 (3H, m), 7.98 (1H, d, J = 9.3 Hz), 7.54 (1H, ddd, J = 8.3, 8.3, 5.9 Hz), 7.17 (1H, ddd, J = 8.3, 8.3, 2.3 Hz), 5.28 (2H, s), 4.33 (2H, q, J = 7.2 Hz), 4.20 (3H, s), 1.34 (3H, t, J = 7.2 Hz).
ESI-MS (m/z) 476 $[M+H]^+$.

Example 88

7-{(1-carboxymethyl)-1H-1,2,3-triazol-4-yl}-2-(3--fluorophenyl)-2H-[1,2,3]triazolo[4,5-f]quinoline-9-carboxy lic acid (Compound 88)

In a similar manner to Example 6, from Compound 77 (66 mg, 0.14 mmol) obtained in Example 77, the title Compound 88 (58 mg, 96%) was obtained.
$^1$H-NMR (DMSO-$d_6$) δ: 14.22 (1H, s), 13.54 (1H, s), 8.90 (1H, s), 8.36 (1H, s), 8.33 (1H, d, J= 9.3 Hz), 8.18 (1H, m), 8.11 (1H, m), 8.03 (1H, d, J = 9.3 Hz), 7.78 (1H, m), 7.47 (1H, ddd, J = 8.5, 8.5, 2.4 Hz), 5.44 (2H, s).
ESI-MS (m/z) 434 $[M+H]^+$.

Example 89

2-(3-fluorophenyl)-7-{1-(2-methoxyethyl)-1H-pyrrol-3-yl}-2H -[1,2,3]triazolo[4,5-f]quinoline-9-carboxylic acid (Compound 89)

In a similar manner to Example 33, from Compound 70 (47 mg, 0.12 mmol) obtained in Example 70 and 2-chloroethylmethylether (0.044 mL, 0.49 mmol), the title Compound 89 (32 mg, 62%) was obtained.
$^1$H-NMR (DMSO-$d_6$) δ: 8.22 (1H, d, J = 9.5 Hz), 8.15 (1H, dd, J = 8.1, 1.1 Hz), 8.08 (1H, ddd, J = 9.7, 2.3, 2.3 Hz), 8.01 (1H, s), 7.95 (1H, d, J = 9.5 Hz), 7.83-7.71 (2H, m), 7.44 (1H, ddd, J = 8.5, 8.5, 2.3 Hz), 6.93 (1H, dd, J = 2.6, 2.6 Hz), 6.87 (1H, m), 4.14 (2H, t, J = 5.3 Hz), 3.67 (2H, t, J = 5.3 Hz), 3.28 (3H, s).
ESI-MS (m/z) 432 $[M+H]^+$.

Example 90

7-(1,5-dimethyl-1H-pyrazol-4-yl)-2-(3-fluorophenyl)-2H-[1,2 ,3]triazolo[4,5-f]quinoline-9-carboxylic acid methyl ester (Compound 90)

In a similar manner to Example 5, from Compound 64 (40 mg, 0.11 mmol) obtained in Example 64 and (1,5-dimethyl--1H-pyrazol-4-yl)boronic acid pinacol ester (30 mg, 0.14 mmol), the title Compound 90 (35 mg, 75%) was obtained.
$^1$H-NMR ($CDCl_3$) δ: 8.17-7.95 (5H, m), 7.77 (1H, s), 7.548 (1H, m), 7.16 (1H, m), 4.20 (3H, s), 3.90 (3H, s), 2.81 (3H, s).
ESI-MS (m/z) 417 $[M+H]^+$.

Example 91

7-(1,5-dimethyl-1H-pyrazol-4-yl)-2-(3-fluorophenyl)-2H-[1,2 ,3]triazolo[4,5-f]quinoline-9-carboxylic acid (Compound 91)

In a similar manner to Example 6, from Compound 90 (34 mg, 0.082 mmol) obtained in Example 90, the title Compound 91 (28 mg, 84%) was obtained.
$^1$H-NMR (DMSO-$d_6$) δ: 8.26-8.20 (2H, m), 8.15 (1H, dd, J = 8.2, 1.6 Hz), 8.12-8.04 (2H, m), 7.97 (1H, d, J = 9.2 Hz), 7.76 (1H, ddd, J = 8.2, 8.2, 6.1 Hz), 7.45 (1H, ddd, J = 8.2, 8.2, 2.0 Hz), 3.84 (3H, s), 2.81 (3H, s).
ESI-MS (m/z) 403 $[M+H]^+$.

Example 92

7-(1,3-dimethyl-1H-pyrazol-4-yl)-2-(3-fluorophenyl)-2H-[1,2 ,3]triazolo[4,5-f]quinoline-9-carboxylic acid methyl ester (Compound 92)

In a similar manner to Example 5, from Compound 64 (40 mg, 0.11 mmol) obtained in Example 64 and (1,3-dimethyl--1H-pyrazol-4-yl) boronic acid pinacol ester (30 mg, 0.14 mmol), the title Compound 92 (38 mg, 82%) was obtained.
$^1$H-NMR ($CDCl_3$) δ: 8.13 (1H, m), 8.09-8.01 (2H, m), 8.00-7.95 (2H, m), 7.72 (1H, s), 7.53 (1H, ddd, J = 8.2, 8.2, 6.1 Hz), 7.16 (1H, ddd, J = 8.2, 8.2, 1.7 Hz), 4.20 (3H, s), 3.94 (3H, s), 2.69 (3H, s).
ESI-MS (m/z) 417 $[M+H]^+$.

Example 93

7-(1,3-dimethyl-1H-pyrazol-4-yl)-2-(3-fluorophenyl)-2H-[1,2 ,3]triazolo[4,5-f]quinoline-9-carboxylic acid (Compound 93)

In a similar manner to Example 6, from Compound 92 (37 mg, 0.092 mmol) obtained in Example 92, the title Compound 93 (33 mg, 93%) was obtained.
$^1$H-NMR (DMSO-$d_6$) δ: 8.53 (1H, s), 8.23 (1H, d, J = 9.2 Hz), 8.15 (1H, d, J = 8.4 Hz), 8.08 (1H, m), 8.00-7.92 (2H, m), 7.76 (1H, m), 7.44 (1H, ddd, J = 8.4, 8.4, 2.1 Hz), 3.85 (3H, s), 2.62 (3H, s).
ESI-MS (m/z) 403 $[M+H]^+$.

Example 94

2-(3-fluorophenyl)-7-(1-methyl-1H-pyrazol-5-yl)-2H-[1,2,3]-triazolo[4,5-f]quinoline-9-carboxylic acid methyl ester (Compound 94)

In a similar manner to Example 5, from Compound 64 (40 mg, 0.11 mmol) obtained in Example 64 and (1-methyl--1H-pyrazol-5-yl)boronic acid pinacol ester (28 mg, 0.14 mmol), the title Compound 94 (37 mg, 81%) was obtained.
$^1$H-NMR ($CDCl_3$) δ: 8.19-7.99 (4H, m), 7.90 (1H, s), 7.60-7.50 (2H, m), 7.19 (1H, ddd, J = 8.3, 8.3, 2.4 Hz), 6.81 (1H, d, J = 2.3 Hz), 4.43 (3H, s), 4.21 (3H, s).
ESI-MS (m/z) 403 $[M+H]^+$.

Example 95

2-(3-fluorophenyl)-7-(1-methyl-1H-pyrazol-5-yl)-2H-[1,2,3]-triazolo[4,5-f]quinoline-9-carboxylic acid (Compound 95)

In a similar manner to Example 6, from Compound 94 (36 mg, 0.089 mmol) obtained in Example 94, the title Compound 95 (32 mg, 93%) was obtained.
$^1$H-NMR (DMSO-$d_6$) δ: 8.32 (1H, d, J = 9.5 Hz), 8.27 (1H, s), 8.21-8.05 (3H, m), 7.77 (1H, ddd, J = 8.2, 8.2, 6.4 Hz), 7.59 (1H, d, J = 2.2 Hz), 7.46 (1H, ddd, J = 8.3, 2.0, 1.0 Hz), 7.21 (1H, d, J = 2.2 Hz), 4.37 (3H, s).
ESI-MS (m/z) 389 $[M+H]^+$.

Example 96

2-(3-fluorophenyl)-7-(thiophen-3-yl)-2H-[1,2,3]triazolo[4,5 -f]quinoline-9-carboxylic acid methyl ester (Compound 96)

In a similar manner to Example 5, from Compound 64 (40 mg, 0.11 mmol) obtained in Example 64 and (thiophen-3--yl)boronic acid (17 mg, 0.14 mmol), the title Compound 96 (41 mg, 91%) was obtained.
$^1$H-NMR ($CDCl_3$) δ: 8.16-8.10 (2H, m), 8.09-8.01 (3H, m), 7.95 (1H, s), 7.88 (1H, dd, J = 4.9, 1.0 Hz), 7.58-7.46 (2H, m), 7.16 (1H, ddd, J = 8.2, 8.2, 2.3 Hz), 4.21 (3H, s).

Example 97

2-(3-fluorophenyl)-7-(thiophen-3-yl)-2H-[1,2,3]triazolo[4,5 -f]quinoline-9-carboxylic acid (Compound 97)

In a similar manner to Example 6, from Compound 96 (40 mg, 0.099 mmol) obtained in Example 96, the title Compound 97 (33 mg, 85%) was obtained.
$^1$H-NMR (DMSO-$d_6$) δ: 14.16 (1H, s), 8.58 (1H, dd, J = 2.9, 1.1 Hz), 8.36 (1H, s), 8.28 (1H, d, J = 9.2 Hz), 8.17 (1H, dd, J = 7.9, 1.6 Hz), 8.10 (1H, ddd, J = 9.9, 2.2, 2.2 Hz), 8.06-8.00 (2H, m), 7.82-7.72 (2H, m), 7.46 (1H, ddd, J = 8.5, 8.5, 2.2 Hz).
ESI-MS (m/z) 391 $[M+H]^+$.

Example 98

2-(3-fluorophenyl)-7-(pyridin-2-yl)-2H-[1,2,3]triazolo[4,5-f]quinoline-9-carboxylic acid methyl ester (Compound 98)

In a similar manner to Step 2 of Example 1, from Compound 64 (41 mg, 0.12 mmol) obtained in Example 64 and 2-tributylstannylpyridine (0.45 mL, 0.14 mmol), the title Compound 98 (16 mg, 34%) was obtained.
$^1$H-NMR ($CDCl_3$) δ: 8.81 (1H, s), 8.75 (1H, dd, J = 4.6, 0.9 Hz), 8.67 (1H, m), 8.15-8.01 (4H, m), 7.90 (1H, m), 7.53 (1H, ddd, J = 8.2, 8.2, 6.0 Hz), 7.39 (1H, m), 7.16 (1H, ddd, J = 8.2, 8.2, 2.6 Hz), 4.20 (3H, s).
ESI-MS (m/z) 400 $[M+H]^+$.

Example 99

2-(3-fluorophenyl)-7-(pyridin-2-yl)-2H-[1,2,3]triazolo[4,5-f]quinoline-9-carboxylic acid (Compound 99)

In a similar manner to Example 6, from Compound 98 (15 mg, 0.038 mmol) obtained in Example 98, the title Compound 99 (11 mg, 76%) was obtained.
$^1$H-NMR (DMSO-$d_6$) δ: 8.80 (1H, d, J = 3.7 Hz), 8.70 (1H, s), 8.65 (1H, d, J = 8.8 Hz), 8.35 (1H, d, J = 9.9 Hz), 8.21-8.05 (3H, m), 7.78 (1H, m), 7.59 (1H, m), 7.47 (1H, m), 7.18 (1H, m).
ESI-MS (m/z) 386 $[M+H]^+$.

Example 100

7-chloro-2-(3-methylphenyl)-2H-[1,2,3]triazolo[4,5-f]-quinoline-9-carboxylic acid methyl ester (Compound 100)

In a similar manner to Step 4 of Example 1, fromm-toluidine (0.54 mL, 5.07 mmol) and Compound 4-1 (500 mg, 2.11 mmol) obtained in Step 1 of Example 4 , the title Compound 100 (138 mg, 19%) was obtained.
$^1$H-NMR ($CDCl_3$) δ: 8.19-8.07 (3H, m), 7.94 (1H, d, J = 9.5 Hz), 7.62 (1H, s), 7.45 (1H, t, J = 7.7 Hz), 7.29 (1H, d, J = 7.7 Hz), 4.19 (3H, s), 2.51 (3H, s).

Example 101

7-(furan-2-yl)-2-(3-methylphenyl)-2H-[1,2,3]triazolo[4,5-f] quinoline-9-carboxylic acid methyl ester (Compound 101)

In a similar manner to Step 2 of Example 1, from Compound 100 (66 mg, 0.19 mmol) obtained in Example 100 and 2-tributylstannylfuran (0.070 mL, 0.23 mmol), the title Compound 101 (61 mg, 85%) was obtained.
$^1$H-NMR ($CDCl_3$) δ: 8.17-8.00 (5H, m), 7.65 (1H, m), 7.44 (1H, dd, J = 7.9, 7.9 Hz), 7.29-7.27 (2H, m), 6.62 (1H, dd, J = 3.5, 1.8 Hz), 4.20 (3H, s), 2.50 (3H, s).

Example 102

7-(furan-2-yl)-2-(3-methylphenyl)-2H-[1,2,3]triazolo[4,5-f] quinoline-9-carboxylic acid (compound 102)

In a similar manner to Example 6, from Compound 101 (60 mg, 0.16 mmol) obtained in Example 101, the title Compound 102 (54 mg, 93%) was obtained.
$^1$H-NMR (DMSO-$d_6$) δ: 14.17 (1H, s), 8.29 (1H, d, J = 9.5 Hz), 8.16-8.07 (3H, m), 8.03-7.98 (2H, m), 7.59 (1H, dd, J = 7.9, 7.9 Hz), 7.50 (1H, d, J = 3.3 Hz), 7.40 (1H, d, J = 8.1 Hz), 6.77 (1H, dd, J = 3.3, 1.8 Hz), 2.50 (3H, s).
ESI-MS (m/z) 371 $[M+H]^+$.

Example 103

7-chloro-2-(3-trifluoromethylphenyl)-2H-[1,2,3]triazolo[4,5 -f]quinoline-9-carboxylic acid methyl ester (Compound 103)

In a similar manner to Step 4 of Example 1, from 3-trifluoromethylaniline (0.63 mL, 5.07 mmol) and Compound 4-1 (500 mg, 2.11 mmol) obtained in Step 1 of Example 4 , the title Compound 103 (246 mg, 29%) was obtained.
$^1$H-NMR ($CDCl_3$) δ: 8.64-8.60 (1H, m), 8.52 (1H, m), 8.16 (1H, d, J = 9.2 Hz), 7.97 (1H, d, J = 9.5 Hz), 7.76-7.71 (2H, m), 7.64 (1H, s), 4.19 (3H, s).

Example 104

7-(furan-2-yl)-2-(3-trifluoromethylphenyl)-2H-[1,2,3]-triazolo[4,5-f]quinoline-9-carboxylic acid methyl ester (Compound 104)

In a similar manner to Step 2 of Example 1, from Compound 103 (61 mg, 0.15 mmol) obtained in Example 103 and 2-tributylstannylfuran (0.057 mL, 0.18 mmol), the title Compound 104 (61 mg, 92%) was obtained.
$^1$H-NMR ($CDCl_3$) δ: 8.64-8.60 (1H, m), 8.51 (1H, m), 8.12-8.01 (3H, m), 7.74-7.69 (2H, m), 7.65 (1H, d, J = 1.0 Hz), 7.31 (1H, d, J = 2.6 Hz), 6.63 (1H, dd, J = 3.5, 1.8 Hz), 4.20 (3H, s).

Example 105

7-(furan-2-yl)-2-(3-trifluoromethylphenyl)-2H-[1,2,3]-triazolo[4,5-f]quinoline-9-carboxylic acid (Compound 105)

In a similar manner to Example 6, from Compound 104 (60 mg, 0.14 mmol) obtained in Example 104, the title Compound 105 (56 mg, 97%) was obtained..
$^1$H-NMR (DMSO-$d_6$) δ: 8.60 (1H, m), 8.54 (1H, m), 8.32 (1H, d, J = 9.3 Hz), 8.18 (1H, s), 8.04 (1H, d, J = 9.3 Hz), 8.01-7.95 (3H, m), 7.52 (1H, d, J = 3.6 Hz), 6.78 (1H, dd, J = 3.6, 1.7 Hz).
ESI-MS (m/z) 425 $[M+H]^+$.

Example 106

7-(4-acetylpiperazin-1-yl)-2-(3-fluorophenyl)-2H-[1,2,3]-triazolo[4,5-f]quinoline-9-carboxylic acid (Compound 106)

Compound 75 (40 mg, 0.12 mmol) obtained in Example 75 and 1-acetylpiperazine (148 mg, 1.17 mmol) were dissolved in N,N-dimethylacetamide (0.47 mL), and the mixture was stirred at 80°C for 24 hours. After the mixture was cooled to room temperature, hydrochloric acid was added thereto. The precipitate was filtered and collected, and thus the title Compound 106 (41 mg, 82%) was obtained.
$^1$H-NMR (DMSO-$d_6$) δ: 8.19-7.96 (3H, m), 7.82-7.67 (2H, m), 7.51-7.36 (2H, m), 3.88-3.72 (4H, m), 3.69-3.53 (4H, m), 2.08 (3H, s).

ESI-MS (m/z) 435 $[M+H]^+$.

Example 107

2-(3-fluorophenyl)-7-{1-(2-morpholinoethyl)-1H-pyrazol-4--yl}-2H-[1,2,3]triazolo[4,5-f]quinoline-9-carboxylic acid methyl ester (Compound 107)

In a similar manner to Example 5, from Compound 64 (105 mg, 0.29 mmol) obtained in Example 64 and 1-(2-morpholinoethyl)-1H-pyrazol-4-ylboronic acid pinacol ester (109 mg, 0.35 mmol), the title Compound 144 (92 mg, 62%) was obtained.
$^1$H-NMR ($CDCl_3$) δ: 8.21 (1H, s), 8.20-8.00 (3H, m), 7.96 (1H, d, J = 9.6 Hz), 7.76 (1H, s), 7.62-7.36 (2H, m), 7.16 (1H, ddd, J = 8.2, 8.2, 2.5 Hz), 4.34 (2H, t, J = 6. 6 Hz), 4.20 (3H, s), 3.78-3.67 (4H, m), 2.90 (2H, t, J = 6.6 Hz), 2.58-2.50 (4H, m).
ESI-MS (m/z) 502 $[M+H]^+$.

Example 108

2-(3-fluorophenyl)-7-{1-(2-morpholinoethyl)-1H-pyrazol-4--yl}-2H-[1,2,3]triazolo[4,5-f]quinoline-9-carboxylic acid (Compound 108)

In a similar manner to Example 6, from Compound 107 (91 mg, 0.18 mmol) obtained in Example 107, the title Compound 108 (74 mg, 84%) was obtained.
$^1$H-NMR (DMSO-$d_6$) δ: 8.56 (1H, s), 8.21 (1H, s), 8.16-8.09 (2H, m), 8.04 (1H, m), 7.87 (1H, d, J = 9.3 Hz), 7.70 (1H, ddd, J = 8.2, 8.2, 6.1 Hz), 7.53 (1H, m), 7.39 (1H, ddd, J = 8.2, 8.2, 2.3 Hz), 4.32 (2H, t, J = 6.3 Hz), 3.60-3.52 (4H, m), 2.79 (2H, t, J = 6.3 Hz), 2.48-2.41 (4H, m).
ESI-MS (m/z) 488 $[M+H]^+$.

Example 109

7-{4-(ethoxycarbonyl)piperazin-1-yl}-2-(3-fluorophenyl)-2H-[1,2,3]triazolo[4,5-f]quinoline-9-carboxylic acid (Compound 109)

In a similar manner to Example 106, from Compound 75 (55 mg, 0.16 mmol) obtained in Example 75 and 1-(ethoxycarbonyl) - piperazine (0.24 mL, 1. 61 mmol), the title Compound 109 (61 mg, 81%) was obtained.
$^1$H-NMR (DMSO-$d_6$) δ: 8.14-8.00 (3H, m), 7.79-7.65 (2H, m), 7.46-7.35 (2H, m), 4.09 (2H, q, J = 7.0 Hz), 3.83-3.75 (4H, m), 3.59-3.40 (4H, m), 1.22 (3H, t, J = 6.9 Hz).
ESI-MS (m/z) 465 $[M+H]^+$.

Example 110

7-{4-(ethylsulfonyl)piperazin-1-yl}-2-(3-fluorophenyl)-2H-[ 1,2,3]triazolo[4,5-f]quinoline-9-carboxylic acid (Compound 110)

In a similar manner to Example 106, from Compound 75 (52 mg, 0.15 mmol) obtained in Example 75 and 1-(ethylsulfonyl)piperazine (270 mg, 1.52 mmol), the title Compound 110 (74 mg, quantitative yield) was obtained.
$^1$H-NMR (DMSO-$d_6$) δ: 8.14-8.07 (2H, m), 8.04 (1H, ddd, J = 9.9, 2.1, 2.1 Hz), 7.78-7.67 (2H, m), 7.45-7.37 (2H, m), 3.95-3.83 (4H, m), 3.37-3.29 (4H, m), 3.11 (2H, q, J = 7.4 Hz), 1.24 (3H, t, J = 7.3 Hz).
ESI-MS (m/z) 485 $[M+H]^+$.

Example 111

2-(3-fluorophenyl)-7-(pyridin-3-yl)-2H-[1,2,3]triazolo[4,5-f]quinoline-9-carboxylic acid methyl ester (Compound 111)

In a similar manner to Step 2 of Example 1, from Compound 64 (70 mg, 0.20 mmol) obtained in Example 64 and 3-tributylstannylpyridine (0.13 mL, 0.39 mmol), the title Compound 111 (25 mg, 32%) was obtained.
$^1$H-NMR ($CDCl_3$) δ: 9.42 (1H, d, J = 2.2 Hz), 8.75 (1H, dd, J = 4.9, 1.8 Hz), 8.54 (1H, ddd, J = 8.3, 2.2, 1.8 Hz), 8.19-8.02 (5H, m), 7.62-7.47 (2H, m), 7.18 (1H, ddd, J = 8.3, 8.2, 2.4 Hz), 4.23 (3H, s).
ESI-MS (m/z) 400 $[M+H]^+$.

Example 112

2-(3-fluorophenyl)-7-(pyridin-3-yl)-2H-[1,2,3]triazolo-[4,5-f]quinoline-9-carboxylic acid (Compound 112)

In a similar manner to Example 6, from Compound 111 (23 mg, 0.058 mmol) obtained in Example 111, the title Compound 112 (20 mg, 88%) was obtained.
$^1$H-NMR (DMSO-$d_6$) δ: 9.56-9.48 (1H, m), 8.76-8.67 (2H, m), 8.51 (1H, m), 8.32 (1H, d, J = 9.5 Hz), 8.20-8.06 (3H, m), 7.77 (1H, m), 7.62 (1H, dd, J = 7.9, 4.9 Hz), 7.46 (1H, ddd, J = 8.2, 8.2, 2.1 Hz).
ESI-MS (m/z) 386 $[M+H]^+$.

Example 113

2-(3-fluorophenyl)-7-(pyridin-4-yl)-2H-[1,2,3]triazolo[4,5-f]quinoline-9-carboxylic acid methyl ester (Compound 113)

In a similar manner to Step 2 of Example 1, from Compound 64 (70 mg, 0.20 mmol) obtained in Example 64 and 4-tributylstannylpyridine (144 mg, 0.39 mmol), the title Compound 113 (21 mg, 27%) was obtained.
$^1$H-NMR ($CDCl_3$) δ: 8.83 (2H, d, J = 6.2 Hz), 8.20-8.02 (7H, m), 7.56 (1H, ddd, J = 8.2, 8.2, 5.9 Hz), 7.19 (1H, ddd, J = 8.2, 8.2, 2.3 Hz), 4.23 (3H, s).
ESI-MS (m/z) 400 $[M+H]^+$.

Example 114

2-(3-fluorophenyl)-7-(pyridin-4-yl)-2H-[1,2,3]triazolo-[4,5-f]quinoline-9-carboxylic acid (compound 114)

In a similar manner to Example 6, from Compound 113 (21 mg, 0.053 mmol) obtained in Example 113, the title Compound 114 (15 mg, 75%) was obtained.
$^1$H-NMR (DMSO-$d_6$) δ: 8.90 (2H, d, J = 6.2 Hz), 8.67 (1H, s), 8.51 (2H, d, J = 6.2 Hz), 8.38 (1H, d, J = 9.5 Hz), 8.21-8.08 (3H, m), 7.78 (1H, ddd, J = 8.2, 8.2, 6.4 Hz), 7.48 (1H, ddd, J = 8.2, 8.2, 2.1 Hz).
ESI-MS (m/z) 386 $[M+H]^+$.

Example 115

7-(1-benzyl-1H-pyrazol-4-yl)-2-(3-fluorophenyl)-2H-[1,2,3]-triazolo[4,5-f]quinoline-9-carboxylic acid methyl ester (Compound 115)

In a similar manner to Example 5, from Compound 64 (63 mg, 0.18 mmol) obtained in Example 64 and 1-benzyl-1H--pyrazol-4-ylboronic acid pinacol ester (60 mg, 0.21 mmol), the title Compound 115 (84 mg, 99%) was obtained.
$^1$H-NMR ($CDCl_3$) δ: 8.19 (1H, s), 8.14-8.00 (3H, m), 7.95 (1H, d, J = 9.3 Hz), 7.74 (1H, s), 7.55 (1H, m), 7.43-7.12 (6H, m), 5.40 (2H, s), 4.19 (3H, s).
ESI-MS (m/z) 479 $[M+H]^+$.

Example 116

7-(1-benzyl-1H-pyrazol-4-yl)-2-(3-fluorophenyl)-2H-[1,2,3]-triazolo[4,5-f]quinoline-9-carboxylic acid (Compound 116)

In a similar manner to Example 6, from Compound 115 (84 mg, 0.18 mmol) obtained in Example 115, the title Compound 116 (69 mg, 85%) was obtained.
$^1$H-NMR (DMSO-$d_6$) δ: 8.71 (1H, s), 8.33 (1H, s), 8.24 (1H, d, J = 9.5 Hz), 8.19-8.12 (2H, m), 8.08 (1H, ddd, J = 9.8, 2.2, 2.2 Hz), 7.96 (1H, d, J = 9.5 Hz), 7.76 (1H, ddd, J = 8.2, 8.2, 6.2 Hz), 7.49-7.29 (6H, m), 5.44 (2H, s).
ESI-MS (m/z) 465 $[M+H]^+$.

Example 117

7-{4-(benzoyl)piperazin-1-yl}-2-(3-fluorophenyl)-2H-[1,2,3] triazolo[4,5-f]quinoline-9-carboxylic acid (Compound 117)

In a similar manner to Example 106, from Compound 75 (63 mg, 0.18 mmol) obtained in Example 75 and 1-benzoylpiperazine (270 mg, 1.52 mmol), the title Compound 117 (47 mg, 51%) was obtained.

$^1$H-NMR (DMSO-$d_6$) δ: 8.32 (1H, s), 8.06 (2H, dd, J = 8.4, 1.2 Hz), 8.00-7.92 (3H, m), 7.66-7.55 (2H, m), 7.51-7.42 (3H, m), 7.31 (1H, td, J = 8.4, 2.2 Hz), 3.67-3.46 (8H, m).
ESI-MS (m/z) 497 $[M+H]^+$.

Example 118

7-{5-(methoxycarbonyl)-1H-pyrrol-3-yl}-2-(3-fluorophenyl)-2 H-[1,2,3]triazolo[4,5-f]quinoline-9-carboxylic acid (Compound 118)

Compound 75 (120 mg, 0.35 mmol) obtained in Example 75, 1-(tert-butoxycarbonyl)-2-(methoxycarbonyl) pyrrole-4--boronic acid pinacol ester (148 mg, 0.42 mmol), diisopropylethylamine (0.27 mL, 2.1 mmol), and a 1,1'-[bis (diphenylphosphino)ferrocene]palladium(II) chloride dichloromethane adduct (29 mg, 0.035 mmol) were suspended in a mixed solvent of acetonitrile and water (6:1, 3.5 mL), and the mixture was refluxed for 23 hours. After the mixture was cooled to room temperature, DMF was added thereto. Insoluble compounds were filtered and removed, and hydrochloric acid was added to the filtrate. The precipitate was filtered and collected, and thus the title Compound 118 (138 mg, 91%) was obtained.
$^1$H-NMR (DMSO-$d_6$) δ: 12.45 (1H, s), 8.28-8.22 (2H, m), 8.18-7.94 (4H, m), 7.76 (1H, ddd, J = 8.3, 8.3, 6.3 Hz), 7.65 (1H, dd, J = 2.1, 2.1 Hz), 7.45 (1H, ddd, J = 8.8, 8.8, 2.5 Hz), 3.83 (3H, s).
ESI-MS (m/z) 430 $[M+H]^+$.

Example 119

7-(piperidin-1-yl)-2-(3-fluorophenyl)-2H-[1,2,3]triazolo[4, 5-f]quinoline-9-carboxylic acid (Compound 119)

In a similar manner to Example 106, from Compound 75 (49 mg, 0.14 mmol) obtained in Example 75 and piperidine (0.86 mL, 2.86 mmol), the title Compound 119 (53 mg, 95%) was obtained.
$^1$H-NMR (DMSO-$d_6$) δ: 8.10 (2H, d, J = 9.3 Hz), 8.04 (1H, dt, J = 9.9, 2.1 Hz), 7.79-7.68 (2H, m), 7.45-7.36 (2H, m), 4.00-3.55 (4H, m), 1.75-1.55 (6H, m).
ESI-MS (m/z) 392 $[M+H]^+$.

Example 120

(S)-2-(3-fluorophenyl)-7-(3-hydroxypyrrolidin-1-yl)-2H-[1,2 ,3]triazolo[4,5-f]quinoline-9-carboxylic acid (Compound 120)

In a similar manner to Example 106, from Compound 75 (50 mg, 0.15 mmol) obtained in Example 75 and (S)-(-)-3--hydroxypyrrolidine (0.24 mL, 2.92 mmol), the title Compound 120 (51 mg, 89%) was obtained. -
$^1$H-NMR (DMSO-$d_6$) δ: 8.22 (1H, d, J = 9.3 Hz), 8.12 (1H, dd, J = 7.9, 2.2 Hz), 8.09-7.92 (2H, m), 7.74 (1H, ddd, J = 8.2, 8.2, 6.4 Hz), 7.43 (1H, ddd, J = 8.2, 8.2, 2.2 Hz), 7.22 (1H, s), 4.50 (1H, m), 4.00-3.50 (4H, m), 2.19-1.94 (2H, m).
ESI-MS (m/z) 394 $[M+H]^+$.

Example 121

(S)-2-(3-fluorophenyl)-7-(3-dimethylaminopyrrolidin-1-yl)--2H-[1,2,3]triazolo[4,5-f]quinoline-9-carboxylic acid (Compound 121)

In a similar manner to Example 106, from Compound 75 (52 mg, 0.15 mmol) obtained in Example 75 and (S)-(-)-3-dimethylaminopyrrolidine (0.39 mL, 3.03 mmol), the title Compound 121 (34 mg, 54%) was obtained.
$^1$H-NMR (DMSO-$d_6$) δ: 11.07 (1H, s), 8.12 (2H, d, J = 9.5 Hz), 8.04 (1H, ddd, J = 9.9, 2.2, 2.2 Hz), 7.79-7.69 (2H, m), 7.41 (1H, ddd, J = 8.6, 8.6, 2.2 Hz), 7.09 (1H, s), 4.12-3.79 (5H, m), 2.94 (3H, s), 2.78 (3H, s), 2.42-2.15 (2H, m).
ESI-MS (m/z) 421 $[M+H]^+$.

Example 122

(S)-2-(3-fluorophenyl)-7-(3-acetamidepyrrolidin-1-yl)-2H-[1 ,2,3]triazolo[4,5-f]quinoline-9-carboxylic acid (Compound 122)

In a similar manner to Example 106, from Compound 75 (53 mg, 0.16 mmol) obtained in Example 75 and (S)-(-)-3--acetamidopyrrolidine (396 mL, 3.09 mmol), the title Compound 122 (13 mg, 19%) was obtained.

$^1$H-NMR (DMSO-$d_6$) δ: 8.23-8.00 (3H, m), 7.79-7.69 (2H, m), 7.41 (1H, m), 7.03 (1H, s), 4.47 (1H, m), 3.87-3.62 (4H, m), 2.04-1.90 (2H, m), 1.83 (3H, s).
ESI-MS (m/z) 435 $[M+H]^+$.

Example 123

2-(3-fluorophenyl)-7-(4-ethoxycarbonylpiperidin-1-yl)-2H-[1 ,2,3]triazolo[4,5-f]quinoline-9-carboxylic acid (Compound 123)

In a similar manner to Example 106, from Compound 75 (47 mg, 0.14 mmol) obtained in Example 75 and ethyl isonipecotate (0.42 mL, 2.7 mmol), the title Compound 123 (56 mg, 87%) was obtained.
ESI-MS (m/z) 464 $[M+H]^+$.

Example 124

2-(3-fluorophenyl)-7-{4-(2-hydroxyethyl)piperidin-1-yl}-2H-[1,2,3]triazolo[4,5-f]quinoline-9-carboxylic acid (Compound 124)

In a similar manner to Example 106, from Compound 75 (49 mg, 0.14 mmol) obtained in Example 75 and 4-(2-hydroxyethyl)-piperidine (369 mg, 2.86 mmol), the title Compound 124 (55 mg, 89%) was obtained.
$^1$H-NMR (DMSO-$d_6$) δ: 8.14-8.07 (2H, m), 8.03 (1H, ddd, J = 9.9, 2.2, 2.2 Hz), 7.78-7.68 (2H, m), 7.45-7.36 (2H, m), 4.60 (2H, d, J = 12.8 Hz), 3.72-3.18 (2H, m), 3.00 (2H, t, J = 12.1 Hz), 1.85-1.74 (3H, m), 1.40 (2H, q, J = 6.6 Hz), 1.28-1.12 (2H, m).
ESI-MS (m/z) 422 $[M+H]^+$.

Example 125

2-(3-fluorophenyl)-7-{4-(hydroxymethyl)piperidin-1-yl}-2H-[ 1,2,3]triazolo[4,5-f]quinoline-9-carboxylic acid (Compound 125)

In a similar manner to Example 106, from Compound 75 (40 mg, 0.12 mmol) obtained in Example 75 and 4-(hydroxymethyl)piperidine (269 mg, 2.33 mmol), the title Compound 125 (47 mg, 95%) was obtained.
$^1$H-NMR (DMSO-$d_6$) δ: 8.17-8.08 (2H, m), 8.04 (1H, ddd, J = 10.0, 2.2, 2.2 Hz), 7.86 (1H, d, J = 8.8 Hz), 7.74 (1H, ddd, J = 8.2, 8.2, 6.2 Hz), 7.49-7.37 (2H, m), 4.63 (2H, d, J= 13.2 Hz), 3.28 (1H, m), 3.09-3.05 (4H, m), 1.86-1.68 (4H, m).
ESI-MS (m/z) 422 $[M+H]^+$.

Example 126

2-(3-fluorophenyl)-7-(4-hydroxypiperidin-1-yl)-2H-[1,2,3]-triazolo[4,5-f]quinoline-9-carboxylic acid (Compound 126)

In a similar manner to Example 106, from Compound 75 (40 mg, 0.12 mmol) obtained in Example 75 and 4-hydroxypiperidine (236 mg, 2.33 mmol), the title Compound 126 (43 mg, 90%) was obtained.
$^1$H-NMR (DMSO-$d_6$) δ: 8.14-8.06 (2H, m), 8.03 (1H, ddd, J = 10.1, 2.2, 2.2 Hz), 7.78-7.68 (2H, m), 7.45-7.35 (2H, m), 4.30-4.18 (2H, m), 3.79 (1H, m), 3.47-3.32 (2H, m), 1.91-1.78 (2H, m), 1.51-1.36 (2H, m).
ESI-MS (m/z) 408 $[M+H]^+$.

Example 127

(R)-2-(3-fluorophenyl)-7-(3-acetamidepyrrolidin-1-yl)-2H-[1 ,2,3]triazolo[4,5-f]quinoline-9-carboxylic acid (Compound 127)

In a similar manner to Example 106, from Compound 75 (43 mg, 0.13 mmol) obtained in Example 75 and (R)-(-)-3--acetamidopyrrolidine (322 mg, 2.51 mmol), the title Compound 127 (29 mg, 53%) was obtained.
$^1$H-NMR (DMSO-$d_6$) δ: 8.23-8.00 (3H, m), 7.79-7.69 (2H, m), 7.41 (1H, m), 7.03 (1H, s), 4.47 (1H, m), 3.87-3.62 (4H, m), 2.04-1.90 (2H, m), 1.83 (3H, s).
ESI-MS (m/z) 435 $[M+H]^+$.

Example 128

(R)-2-(3-fluorophenyl)-7-(3-dimethylaminopyrrolidin-1-yl)-2 H-[1,2,3]triazolo[4,5-f]quinoline-9-carboxylic acid (Compound 128)

In a similar manner to Example 106, from Compound 75 (41 mg, 0.12 mmol) obtained in Example 75 and (R)-(-)-3--dimethylaminopyrrolidine (0.27 mL, 2.39 mmol), the title Compound 128 (21 mg, 42%) was obtained.
$^1$H-NMR (DMSO-$d_6$) δ: 11.07 (1H, s), 8.12 (2H, d, J = 9.5 Hz), 8.04 (1H, ddd, J = 9.9, 2.2, 2.2 Hz), 7.79-7.69 (2H, m), 7.41 (1H, ddd, J = 8.6, 8.6, 2.2 Hz), 7.09 (1H, s), 4.12-3.79 (5H, m), 2.94 (3H, s), 2.78 (3H, s), 2.42-2.15 (2H, m).
ESI-MS (m/z) 421 $[M+H]^+$.

Example 129

7-{4-(2-ethoxy-2-oxoethyl)piperidin-1-yl}-2-(3-fluorophenyl )-2H-[1,2,3]triazolo[4,5-f]quinoline-9-carboxylic acid (Compound 129)

In a similar manner to Example 106, from Compound 75 (40 mg, 0.12 mmol) obtained in Example 75 and ethyl 2-(piperidin-4-yl) acetate (400 mg, 2.3 mmol), the title Compound 129 (54 mg, 97%) was obtained.
$^1$H-NMR (DMSO-$d_6$) δ: 8.14-8.07 (2H, m), 8.03 (1H, ddd, J = 9.8, 2.4, 2.4 Hz), 7.78-7.68 (2H, m), 7.45-7.37 (2H, m), 4.64-4.56 (2H, m), 4.08 (2H, q, J = 7.1 Hz), 3.10-2.97 (2H, m), 2.29 (2H, d, J = 6.6 Hz), 1.84-1.75 (2H, m), 1.31-1.15 (6H, m).
ESI-MS (m/z) 478 $[M+H]^+$.

Example 130

2-(3-fluorophenyl)-7-{3-(hydroxymethyl)piperidin-1-yl}-2H-[ 1,2,3]triazolo[4,5-f]quinoline-9-carboxylic acid (Compound 130)

In a similar manner to Example 106, from Compound 75 (40 mg, 0.12 mmol) obtained in Example 75 and 3-(hydroxymethyl)piperidine (268 mg, 2.3 mmol), the title Compound 130 (37 mg, 75%) was obtained.
$^1$H-NMR (DMSO-$d_6$) δ: 8.19-8.08 (2H, m), 8.04 (1H, ddd, J = 9.8, 2.2, 2.2 Hz), 7.89 (1H, d, J = 9.2 Hz), 7.74 (1H, ddd, J = 8.2, 8.2, 6.2 Hz), 7.47-7.37 (2H, m), 4.57-4.44 (2H, m), 3.42-3.36 (2H, m), 3.15 (1H, m), 2.95 (1H, m), 1.88-1.64 (3H, m), 1.54 (1H, m), 1.30 (1H, m).
ESI-MS (m/z) 422 $[M+H]^+$.

Example 131

2-(3-fluorophenyl)-7-{4-(3-hydroxyphenyl)piperidin-1-yl}-2H -[1,2,3]triazolo[4,5-f]quinoline-9-carboxylic acid (Compound 131)

In a similar manner to Example 106, from Compound 75 (40 mg, 0.12 mmol) obtained in Example 75 and 4-(3--hydroxyphenyl)piperidine (413 mg, 2.3 mmol), the title Compound 131 (47 mg, 83%) was obtained.
$^1$H-NMR (DMSO-$d_6$) δ: 8.14-8.00 (3H, m), 7.78-7.68 (2H, m), 7.45-7.36 (2H, m), 7.08 (1H, dd, J = 7.7, 7.7 Hz), 6.71-6.53 (4H, m), 4.82-4.70 (2H, m), 3.16-3.00 (2H, m), 1.95-1.52 (5H, m).
ESI-MS (m/z) 484 $[M+H]^+$.

Example 132

7-{4-(4-ethoxycarbonylphenyl)piperazin-1-yl}-2-(3--fluorophenyl)-2H-[1,2,3]triazolo[4,5-f]quinoline-9--carboxylic acid (Compound 132)

In a similar manner to Example 106, from Compound 75 (40 mg, 0.12 mmol) obtained in Example 75 and 1-(4-ethoxycarbonylphenyl)piperazine (545 mg, 2.3 mmol), the title Compound 132 (42 mg, 66%) was obtained.
$^1$H-NMR (DMSO-$d_6$) δ: 8.16-8.02 (3H, m), 7.87-7.69 (4H, m), 7.46-7.37 (2H, m), 7.05 (2H, d, J = 9.2 Hz), 4.25 (2H, q, J = 7.1 Hz), 3.99-3.92 (4H, m), 3.57-3.50 (4H, m), 1.30 (3H, t, J = 7.0 Hz).
ESI-MS (m/z) 541 $[M+H]^+$.

Example 133

Tablet

In a usual manner, a tablet having the following composition is prepared. Compound 6 (40 g), 286.8 g of lactose, and 60 g of potato starch are mixed, and to this mixture, 120 g of a 10% hydroxypropylcellulose aqueous solution is added. The obtained mixture is kneaded, granulated, dried, and fine-granulated in a usual manner to prepare granules for tableting. Magnesium stearate 1.2 g is added to and mixed with this, and tableting is performed with use of a tableting machine (Model RT-15, made by KIKUSUI) having a pestle 8 mm in diameter to give tablets (containing 20 mg of the active ingredient per tablet).

Table 13

| | | |
|---|---|---|
| Formula | Compound 6 | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

Example 134

Injection

In a usual manner, an injection having the following composition is prepared. Compound 8 (1 g) is added to and mixed with distilled water for injection, then hydrochloric acid and an aqueous sodium hydroxide solution is added thereto for adjusting the pH to 7, and then distilled water for injection is further added until the total volume reaches 1000 mL. The obtained mixed solution is aseptically introduced into glass vials (2 mL each) to give an injection (containing 2 mg of active ingredient per vial).

Table 14

| | | |
|---|---|---|
| Formula | Compound 8 | 2 mg |
| | Hydrochloric acid | q.s. |
| | Aqueous sodium hydroxide sol. | q.s. |
| | distilled water for injection | q.s. |
| | | 2.00 mL |

Example 135

Tablet

In a usual manner, a tablet having the following composition is prepared. Compound 133 (40 g), 286.8 g of lactose, and 60 g of potato starch are mixed, and to this mixture, 120 g of a 10% hydroxypropylcellulose aqueous solution is added. The obtained mixture is kneaded, granulated, dried, and fine-granulated in a usual manner to prepare granules for tableting. Magnesium stearate 1.2 g is added to and mixed with this, and tableting is performed with use of a tableting machine (Model RT-15, made by KIKUSUI) having a pestle 8 mm in diameter to give tablets (containing 20 mg of the active ingredient per tablet).

Table 15

| | | |
|---|---|---|
| Formula | Compound 133 | 20 mg |
| | Lactose | 143.4 mg |
| | Potato starch | 30 mg |
| | Hydroxypropylcellulose | 6 mg |
| | Magnesium stearate | 0.6 mg |

(continued)

| | | |
|---|---|---|
| Formula | Compound 133 | 20 mg |
| | | 200 mg |

Example 136

Injection

In a usual manner, an injection having the following composition is prepared. Compound 133 (1 g) is added to and mixed with distilled water for injection, then hydrochloric acid and an aqueous sodium hydroxide solution is added thereto for adjusting the pH to 7, and then distilled water for injection is further added until the total volume reaches 1000 mL. The obtained mixed solution is aseptically introduced into glass vials (2 mL each) to give an injection (containing 2 mg of active ingredient per vial).

Table 16

| | | |
|---|---|---|
| Formula | Compound 133 | 2 mg |
| | Hydrochloric acid | q.s. |
| | Aqueous sodium hydroxide sol. | q.s. |
| | distilled water for injection | q.s. |
| | | 2.00 mL |

INDUSTRIAL APPLICABILITY

The present invention provides a pharmaceutical composition comprising an aromatic heterocyclic compound or a pharmaceutically acceptable salt thereof [for example, a H-PGDS inhibitor; a therapeutic and/or preventive agent or the like for, for example, a disease involving allergic inflammation (such as asthma, allergic rhinitis, or atopic dermatitis); COPD; a myodegenerative disease (such as muscular dystrophy or polymyositis); and the like]; and an aromatic heterocyclic compound, a pharmaceutically acceptable salt thereof, or the like, having H-PGDS inhibitory effect, and, for example, being useful as a therapeutic and/or preventive agent or the like for, for example, a disease involving allergic inflammation (such as asthma, allergic rhinitis, or atopic dermatitis); COPD; a myodegenerative disease (such as muscular dystrophy or polymyositis); and the like.

**Claims**

1. A pharmaceutical composition comprising as an active ingredient, an aromatic heterocyclic compound represented by the formula (I):

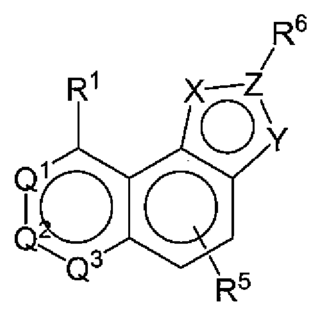

(I)

{wherein $Q^1$ represents $CR^2$ [wherein $R^2$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, -$OR^7$ (wherein $R^7$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or -$NR^8R^9$ (wherein $R^8$ and $R^9$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^8$ and $R^9$ are combined together with the adjacent nitrogen atom to form an optionally substituted nitrogen-containing heterocyclic group)], or a nitrogen atom;
when $Q^1$ is $CR^2$ (wherein $R^2$ has the same meaning as defined above),
$Q^2$ represents $CR^3$ [wherein $R^3$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, -$OR^{10}$ (wherein $R^{10}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or -$NR^{11}R^{12}$ (wherein $R^{11}$ and $R^{12}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{11}$ and $R^{12}$ are combined together with the adjacent nitrogen atom to form an optionally substituted nitrogen-containing heterocyclic group)], or a nitrogen atom;
when $Q^2$ is $CR^3$ (wherein $R^3$ has the same meaning as defined above), $Q^3$ represents a nitrogen atom;
when $Q^2$ is a nitrogen atom, $Q^3$ represents $CR^4$ [wherein $R^4$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, -$OR^{13}$ (wherein $R^{13}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or -$NR^{14}R^{15}$ (wherein $R^{14}$ and $R^{15}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{14}$ and $R^{15}$ are combined together with the adjacent nitrogen atom to form an optionally substituted nitrogen-containing heterocyclic group)];
when $Q^1$ is a nitrogen atom, $Q^2$ represents $CR^3$ (wherein $R^3$ has the same meaning as defined above) and $Q^3$ represents $CR^4$ (wherein $R^4$ has the same meaning as defined above) or a nitrogen atom;
$R^1$ represents -C (=O) $OR^{16}$ (wherein $R^{16}$ represents a hydrogen atom, optionally substituted lower alkyl, optionally substituted cycloalkyl, optionally substituted aryl, optionally substituted aroyl, an optionally substituted aliphatic heterocyclic group, or an optionally substituted aromatic heterocyclic group), -C(=O)$NR^{17}R^{18}$ [wherein $R^{17}$ and $R^{18}$ may be the same or different and each represent a hydrogen atom, optionally substituted lower alkyl, optionally substituted cycloalkyl, optionally substituted aryl, an optionally substituted aromatic heterocyclic group, or -$SO_2R^{19}$ (wherein $R^{19}$ represents optionally substituted lower alkyl, optionally substituted aryl, or an optionally substituted aromatic heterocyclic group), or $R^{17}$ and $R^{18}$ are combined together with the adjacent nitrogen atom to form an optionally substituted nitrogen-containing heterocyclic group], tetrazolyl, or the formula (III) :

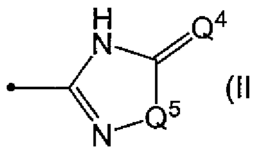
(III)

(wherein $Q^4$ and $Q^5$ may be the same or different and each represent an oxygen atom or a sulfur atom);
$R^5$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{20}$ (wherein $R^{20}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{21}R^{22}$ (wherein $R^{21}$ and $R^{22}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{21}$ and $R^{22}$ are combined together with the adjacent nitrogen atom to form an optionally substituted nitrogen-containing heterocyclic group);
$R^6$ represents optionally substituted cycloalkyl, optionally substituted aryl, or an optionally substituted aromatic heterocyclic group;
X and Y may be the same or different and each represent CH in which H may be substituted with a substituent, NH in which H may be substituted with a substituent, a nitrogen atom, an oxygen atom, or a sulfur atom; and Z represents a nitrogen atom or a carbon atom}, or a pharmaceutically acceptable salt thereof.

2. The pharmaceutical composition according to claim 1, wherein $Q^1$, $Q^2$, and $Q^3$ are $CR^2$ (wherein $R^2$ has the same meaning as defined above), $CR^3$ (wherein $R^3$ has the same meaning as defined above), and a nitrogen atom, respectively.

3. The pharmaceutical composition according to claim 1, which comprises, as an active ingredient, an aromatic heterocyclic compound represented by the formula (I-1) :

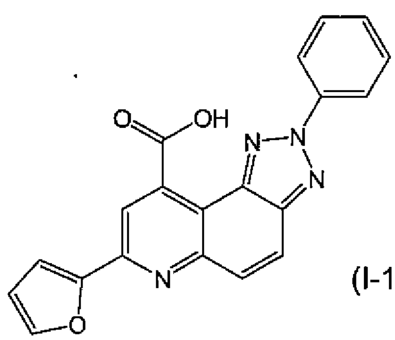
(I-1)

,

or a pharmaceutically acceptable salt thereof.

4. The pharmaceutical composition according to any one of claims 1 to 3, which is an inhibitor of hematopoietic prostaglandin $D_2$ synthase (H-PGDS).

5. The pharmaceutical composition according to any one of claims 1 to 3, which is a therapeutic and/or preventive agent for a disease involving H-PGDS.

6. The pharmaceutical composition according to any one of claims 1 to 3, which is a therapeutic and/or preventive

agent for a disease involving allergic inflammation, COPD, or a myodegenerative disease.

**7.** The pharmaceutical composition according to any one of claims 1 to 3, which is a therapeutic and/or preventive agent for a disease selected from the group consisting of asthma, allergic rhinitis, atopic dermatitis, COPD, muscular dystrophy, and polymyositis.

**8.** A method for inhibiting H-PGDS, comprising a step of administering an effective amount of the aromatic heterocyclic compound or a pharmaceutically acceptable salt thereof described in any one of claims 1 to 3.

**9.** A method for treating and/or preventing a disease involving H-PGDS, comprising a step of administering an effective amount of the aromatic heterocyclic compound or a pharmaceutically acceptable salt thereof described in any one of claims 1 to 3.

**10.** A method for treating and/or preventing a disease involving allergic inflammation, COPD, or a myodegenerative disease, comprising a step of administering an effective amount of the aromatic heterocyclic compound or a pharmaceutically acceptable salt thereof described in any one of claims 1 to 3.

**11.** A method for treating and/or preventing a disease selected from the group consisting of asthma, allergic rhinitis, atopic dermatitis, COPD, muscular dystrophy, and polymyositis, comprising a step of administering an effective amount of the aromatic heterocyclic compound or a pharmaceutically acceptable salt thereof described in any one of claims 1 to 3.

**12.** The aromatic heterocyclic compound or a pharmaceutically acceptable salt thereof described in any one of claims 1 to 3 for use in inhibiting H-PGDS.

**13.** The aromatic heterocyclic compound or a pharmaceutically acceptable salt thereof described in any one of claims 1 to 3 for use in treating and/or preventing a disease involving H-PGDS.

**14.** The aromatic heterocyclic compound or a pharmaceutically acceptable salt thereof described in any one of claims 1 to 3 for use in treating and/or preventing a disease involving allergic inflammation, COPD, or a myodegenerative disease.

**15.** The aromatic heterocyclic compound or a pharmaceutically acceptable salt thereof described in any one of claims 1 to 3 for use in treating and/or preventing a disease selected from the group consisting of asthma, allergic rhinitis, atopic dermatitis, COPD, muscular dystrophy, and polymyositis.

**16.** Use of the aromatic heterocyclic compound or a pharmaceutically acceptable salt thereof described in any one of claims 1 to 3 for the manufacture of a H-PGDS inhibitor.

**17.** Use of the aromatic heterocyclic compound or a pharmaceutically acceptable salt thereof described in any one of claims 1 to 3 for the manufacture of a therapeutic and/or preventive agent for a disease involving H-PGDS.

**18.** Use of the aromatic heterocyclic compound or a pharmaceutically acceptable salt thereof described in any one of claims 1 to 3 for the manufacture of a therapeutic and/or preventive agent for a disease involving allergic inflammation, COPD, or a myodegenerative disease.

**19.** Use of the aromatic heterocyclic compound or a pharmaceutically acceptable salt thereof described in any one of claims 1 to 3 for the manufacture of a therapeutic and/or preventive agent for a disease selected from the group consisting of asthma, allergic rhinitis, atopic dermatitis, COPD, muscular dystrophy, and polymyositis.

**20.** An aromatic heterocyclic compound represented by the formula (I-A):

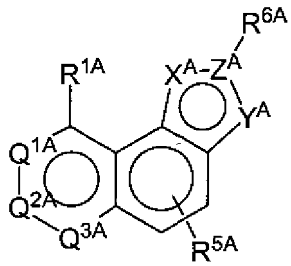

(I-A)

{wherein $Q^{1A}$ represents $CR^{2A}$ [wherein $R^{2A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{7A}$ (wherein $R^{7A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{8A}R^{9A}$ (wherein $R^{8A}$ and $R^{9A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{8A}$ and $R^{9A}$ are combined together with the adjacent nitrogen atom to form an optionally substituted nitrogen-containing heterocyclic group)], or a nitrogen atom;
when $Q^{1A}$ is $CR^{2A}$ (wherein $R^{2A}$ has the same meaning as defined above),
$Q^{2A}$ represents $CR^{3A}$ [wherein $R^{3A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{10A}$ (wherein $R^{10A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{11A}R^{12A}$ (wherein $R^{11A}$ and $R^{12A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{11A}$ and $R^{12A}$ are combined together with the adjacent nitrogen atom to form an optionally substituted nitrogen-containing heterocyclic group)], or a nitrogen atom;
when $Q^{2A}$ is $CR^{3A}$ (wherein $R^{3A}$ has the same meaning as defined above), $Q^{3A}$ represents a nitrogen atom;
when $Q^{2A}$ is a nitrogen atom, $Q^{3A}$ represents $CR^{4A}$ [wherein $R^{4A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{13A}$ (wherein $R^{13A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{14A}R^{15A}$ (wherein $R^{14A}$ and $R^{15A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{14A}$ and $R^{15A}$ are combined together with the adjacent nitrogen atom to form an optionally substituted nitrogen-containing heterocyclic group)];
when $Q^{1A}$ is a nitrogen atom, $Q^{2A}$ represents $CR^{3A}$ (wherein $R^{3A}$ has the same meaning as defined above) and

$Q^{3A}$ represents $CR^{4A}$ (wherein $R^{4A}$ has the same meaning as defined above) or a nitrogen atom;
$R^{1A}$ represents $-C(=O)OR^{16A}$ (wherein $R^{16A}$ represents a hydrogen atom, optionally substituted lower alkyl, optionally substituted cycloalkyl, optionally substituted aryl, optionally substituted aroyl, an optionally substituted aliphatic heterocyclic group, or an optionally substituted aromatic heterocyclic group), $-C(=O)NR^{17A}R^{18A}$ [wherein $R^{17A}$ and $R^{18A}$ may be the same or different and each represent a hydrogen atom, optionally substituted lower alkyl, optionally substituted cycloalkyl, optionally substituted aryl, an optionally substituted aromatic heterocyclic group, or $-SO_2R^{19A}$ (wherein $R^{19A}$ represents optionally substituted lower alkyl, optionally substituted aryl, or an optionally substituted aromatic heterocyclic group), or $R^{17A}$ and $R^{18A}$ are combined together with the adjacent nitrogen atom to form an optionally substituted nitrogen-containing heterocyclic group], tetrazolyl, or the formula (III-A):

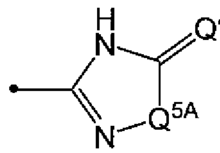
(III-A)

(wherein $Q^{4A}$ and $Q^{5A}$ may be the same or different and each represent an oxygen atom or a sulfur atom);
$R^{5A}$ represents a hydrogen atom, halogen, cyano, optionally substituted lower alkyl, optionally substituted lower alkenyl, optionally substituted lower alkynyl, optionally substituted lower alkylsulfanyl, optionally substituted lower alkylsulfinyl, optionally substituted lower alkylsulfonyl, optionally substituted arylsulfonyl, optionally substituted aryl, an optionally substituted aromatic heterocyclic group, optionally substituted cycloalkyl, an optionally substituted aliphatic heterocyclic group, optionally substituted lower alkylcarbamoyl, optionally substituted di-lower alkylcarbamoyl, optionally substituted lower alkoxycarbonyl, optionally substituted lower alkanoyl, optionally substituted aroyl, optionally substituted aromatic heterocyclic carbonyl, $-OR^{20A}$ (wherein $R^{20A}$ represents a hydrogen atom, optionally substituted lower alkyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl), or $-NR^{21A}R^{22A}$ (wherein $R^{21A}$ and $R^{22A}$ may be the same or different and each represent a hydrogen atom, optionally substituted lower alkyl, optionally substituted lower alkylsulfonyl, optionally substituted cycloalkyl, optionally substituted aryl, an optionally substituted aromatic heterocyclic group, an optionally substituted aliphatic heterocyclic group, optionally substituted aroyl, or optionally substituted lower alkanoyl, or $R^{21A}$ and $R^{22A}$ are combined together with the adjacent nitrogen atom to form an optionally substituted nitrogen-containing heterocyclic group);
$R^{6A}$ represents optionally substituted cycloalkyl, optionally substituted aryl, or an optionally substituted aromatic heterocyclic group;
$X^A$ and $Y^A$ may be the same or different and each represent CH in which H may be substituted with a substituent, NH in which H may be substituted with a substituent, a nitrogen atom, an oxygen atom, or a sulfur atom; and $Z^A$ represents a nitrogen atom or a carbon atom
(excluding the case where $Q^{1A}$, $Q^{2A}$, and $Q^{3A}$ are CH, $CR^{3A}$, and, a nitrogen atom, respectively; $R^{3A}$ is phenyl, 4-nitrophenyl, 4-dimethylaminophenyl, 3-hydroxy-2-methoxyphenyl, 4-methoxycarbonylphenyl, 4-hydroxyphenyl, 3-hydroxynaphthalen-2-yl, 4-methoxyphenyl, or furan-2-yl; $R^{1A}$ is carboxy; $R^{5A}$ is a hydrogen atom; $X^A$, $Y^A$, and $Z^A$ are each a nitrogen atom; and $R^{6A}$ is phenyl)},
or a pharmaceutically acceptable salt thereof.

**21.** The aromatic heterocyclic compound or a pharmaceutically acceptable salt thereof according to claim 20, wherein $Q^{1A}$, $Q^{2A}$, and $Q^{3A}$ are $CR^{2A}$ (wherein $R^{2A}$ has the same meaning as defined above), $CR^{3A}$ (wherein $R^{3A}$ has the same meaning as defined above), and a nitrogen atom, respectively.

**22.** The aromatic heterocyclic compound or a pharmaceutically acceptable salt thereof according to claim 20 or 21, wherein $Q^{1A}$ is CH.

**23.** The aromatic heterocyclic compound or a pharmaceutically acceptable salt thereof according to any one of claims 20 to 22, wherein $R^{1A}$ is $-C(=O)OR^{16B}$ (wherein $R^{16B}$ represents a hydrogen atom or optionally substituted lower alkyl) or $-C(=O)NR^{17B}R^{18B}$ [wherein $R^{17B}$ and $R^{18B}$ may be the same or different and each represent a hydrogen atom, optionally substituted lower alkyl, or $-SO_2R^{19B}$ (wherein $R^{19B}$ represents optionally substituted lower alkyl)].

**24.** The aromatic heterocyclic compound or a pharmaceutically acceptable salt thereof according to any one of claims

20 to 22, wherein $R^{1A}$ is -C(=O)$OR^{16C}$ (wherein $R^{16C}$ represents a hydrogen atom or lower alkyl) or -C(=O)$NR^{17C}R^{18C}$ [wherein $R^{17C}$ and $R^{18C}$ may be the same or different and each represent a hydrogen atom, lower alkyl, or -$SO_2R^{19C}$ (wherein $R^{19C}$ represents lower alkyl)].

**25.** The aromatic heterocyclic compound or a pharmaceutically acceptable salt thereof according to any one of claims 20 to 22, wherein $R^{1A}$ is carboxy.

**26.** The aromatic heterocyclic compound or a pharmaceutically acceptable salt thereof according to any one of claims 20 to 25, wherein $Q^{2A}$ represents $CR^{3B}$ (wherein $R^{3B}$ represents halogen, optionally substituted aryl, an optionally substituted aromatic heterocyclic group, or an optionally substituted aliphatic heterocyclic group).

**27.** The aromatic heterocyclic compound or a pharmaceutically acceptable salt thereof according to any one of claims 20 to 25, wherein $Q^{2A}$ represents $CR^{3C}$ (wherein $R^{3C}$ represents an optionally substituted aromatic heterocyclic group).

**28.** The aromatic heterocyclic compound or a pharmaceutically acceptable salt thereof according to any one of claims 20 to 27, wherein $R^{5A}$ is a hydrogen atom.

**29.** The aromatic heterocyclic compound or a pharmaceutically acceptable salt thereof according to any one of claims 20 to 28, wherein $X^A$, $Y^A$, and $Z^A$ are each a nitrogen atom.

**30.** The aromatic heterocyclic compound or a pharmaceutically acceptable salt thereof according to any one of claims 20 to 28, wherein $X^A$ is an oxygen atom, $Y^A$ is a nitrogen atom, and $Z^A$ is a carbon atom.

**31.** The aromatic heterocyclic compound or a pharmaceutically acceptable salt thereof according to any one of claims 20 to 30, wherein $R^{6A}$ is optionally substituted aryl, or an optionally substituted aromatic heterocyclic group.

**32.** The aromatic heterocyclic compound or a pharmaceutically acceptable salt thereof according to any one of claims 20 to 30, wherein $R^{6A}$ is optionally substituted aryl.

**33.** A pharmaceutical composition comprising, as an active ingredient, the aromatic heterocyclic compound or a pharmaceutically acceptable salt thereof described in any one of claims 20 to 32.

**34.** A H-PGDS inhibitor comprising, as an active ingredient, the aromatic heterocyclic compound or a pharmaceutically acceptable salt thereof described in any one of claims 20 to 32.

**35.** A therapeutic and/or preventive agent for a disease involving H-PGDS comprising, as an active ingredient, the aromatic heterocyclic compound or a pharmaceutically acceptable salt thereof described in any one of claims 20 to 32.

**36.** A therapeutic and/or preventive agent for a disease involving allergic inflammation, COPD, or a myodegenerative disease comprising, as an active ingredient, the aromatic heterocyclic compound or a pharmaceutically acceptable salt thereof described in any one of claims 20 to 32.

**37.** A therapeutic and/or preventive agent for a disease selected from the group consisting of asthma, allergic rhinitis, atopic dermatitis, COPD, muscular dystrophy, and polymyositis comprising, as an active ingredient, the aromatic heterocyclic compound or a pharmaceutically acceptable salt thereof described in any one of claims 20 to 32.

**38.** A method for inhibiting H-PGDS, comprising a step of administering an effective amount of the aromatic heterocyclic compound or a pharmaceutically acceptable salt thereof described in any one of claims 20 to 32.

**39.** A method for treating and/or preventing a disease involving H-PGDS, comprising a step of administering an effective amount of the aromatic heterocyclic compound or a pharmaceutically acceptable salt thereof described in any one of claims 20 to 32.

**40.** A method for treating and/or preventing a disease involving allergic inflammation, COPD, or a myodegenerative disease, comprising a step of administering an effective amount of the aromatic heterocyclic compound or a pharmaceutically acceptable salt thereof described in any one of claims 20 to 32.

**41.** A method for treating and/or preventing a disease selected from the group consisting of asthma, allergic rhinitis, atopic dermatitis, COPD, muscular dystrophy, and polymyositis, comprising a step of administering an effective amount of the aromatic heterocyclic compound or a pharmaceutically acceptable salt thereof described in any one of claims 20 to 32.

**42.** The aromatic heterocyclic compound or a pharmaceutically acceptable salt thereof described in any one of claims 20 to 32 for use in inhibiting H-PGDS.

**43.** The aromatic heterocyclic compound or a pharmaceutically acceptable salt thereof described in any one of claims 20 to 32 for use in treating and/or preventing a disease involving H-PGDS.

**44.** The aromatic heterocyclic compound or a pharmaceutically acceptable salt thereof described in any one of claims 20 to 32 for use in treating and/or preventing a disease involving allergic inflammation, COPD, or a myodegenerative disease.

**45.** The aromatic heterocyclic compound or a pharmaceutically acceptable salt thereof described in any one of claims 20 to 32 for use in treating and/or preventing a disease selected from the group consisting of asthma, allergic rhinitis, atopic dermatitis, COPD, muscular dystrophy, and polymyositis.

**46.** Use of the aromatic heterocyclic compound or a pharmaceutically acceptable salt thereof described in any one of claims 20 to 32 for the manufacture of a H-PGDS inhibitor.

**47.** Use of the aromatic heterocyclic compound or a pharmaceutically acceptable salt thereof described in any one of claims 20 to 32 for the manufacture of a therapeutic and/or preventive agent for a disease involving H-PGDS.

**48.** Use of the aromatic heterocyclic compound or a pharmaceutically acceptable salt thereof described in any one of claims 20 to 32 for the manufacture of a therapeutic and/or preventive agent for a disease involving allergic inflammation, COPD, or a myodegenerative disease.

**49.** Use of the aromatic heterocyclic compound or a pharmaceutically acceptable salt thereof described in any one of claims 20 to 32 for the manufacture of a therapeutic and/or preventive agent for a disease selected from the group consisting of asthma, allergic rhinitis, atopic dermatitis, COPD, muscular dystrophy, and polymyositis.

**INTERNATIONAL SEARCH REPORT**

International application No.
PCT/JP2010/067511

A. CLASSIFICATION OF SUBJECT MATTER
*C07D471/04*(2006.01)i, *A61K31/4745*(2006.01)i, *A61P11/02*(2006.01)i, *A61P11/06*(2006.01)i, *A61P17/04*(2006.01)i, *A61P37/08*(2006.01)i, *A61P43/00*(2006.01)i, *C07D498/04*(2006.01)i

According to International Patent Classification (IPC) or to both national classification and IPC

B. FIELDS SEARCHED

Minimum documentation searched (classification system followed by classification symbols)
C07D471/04, A61K31/4745, A61P11/02, A61P11/06, A61P17/04, A61P37/08, A61P43/00, C07D498/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | RN 303100-52-9 REGISTRY<br>ED Entered STN: 17 Nov 2000<br>CN 2H-1,2,3-Triazolo[4,5-f]quinoline-9-carboxylic acid, 7-(3-hydroxy-2-naphthalenyl)-2-phenyl- (CA INDEX NAME)<br>MF C26 H16 N4 O3<br>SR Chemical Library<br>Supplier: AsInEx<br>LC STN Files: CHEMCATS<br>URL: https://stnweb-japan.cas.org/<br>retrieval date 13 December 2010 (13.12.2010) | 12-15<br>1-2<br>3-7,16-37,<br>42-49 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 December, 2010 (13.12.10) | Date of mailing of the international search report<br>28 December, 2010 (28.12.10) |
| Name and mailing address of the ISA/<br>Japanese Patent Office | Authorized officer |
| Facsimile No. | Telephone No. |

Form PCT/ISA/210 (second sheet) (July 2009)

INTERNATIONAL SEARCH REPORT

International application No. PCT/JP2010/067511

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | RN 303100-53-0 REGISTRY<br>ED Entered STN: 17 Nov 2000<br>CN 2H-1,2,3-Triazolo[4,5-f]quinoline-9-carboxylic acid, 7-(4-hydroxyphenyl)-2-phenyl- (CA INDEX NAME)<br>MF C22 H14 N4 O3<br>SR Chemical Library<br>Supplier: AsInEx<br>URL: https://stnweb-japan.cas.org/<br>retrieval date 13 December 2010 (13.12.2010) | 12-15<br>1-2<br>3-7,16-37, 42-49 |
| X<br>Y<br>A | RN 303100-54-1 REGISTRY<br>ED Entered STN: 17 Nov 2000<br>CN 2H-1,2,3-Triazolo[4,5-f]quinoline-9-carboxylic acid, 7-[4-(methoxycarbonyl)phenyl]-2-phenyl- (CA INDEX NAME)<br>MF C24 H16 N4 O4<br>SR Chemical Library<br>Supplier: AsInEx<br>URL: https://stnweb-japan.cas.org/<br>retrieval date 13 December 2010 (13.12.2010) | 12-15<br>1-2<br>3-7,16-37, 42-49 |
| X<br>Y<br>A | RN 491858-20-9 REGISTRY<br>ED Entered STN: 19 Feb 2003<br>CN 2H-1,2,3-Triazolo[4,5-f]quinoline-9-carboxylic acid, 7-(3-hydroxy-2-methoxyphenyl)-2-phenyl- (CA INDEX NAME)<br>MF C23 H16 N4 O4<br>SR Chemical Library<br>URL: https://stnweb-japan.cas.org/<br>retrieval date 13 December 2010 (13.12.2010) | 12-15<br>1-2<br>3-7,16-37, 42-49 |
| X<br>Y<br>A | RN 508226-84-4 REGISTRY<br>ED Entered STN: 01 May 2003<br>CN 2H-1,2,3-Triazolo[4,5-f]quinoline-9-carboxylic acid, 7-[4-(dimethylamino)phenyl]-2-phenyl- (CA INDEX NAME)<br>MF C24 H19 N5 O2<br>SR Chemical Library<br>URL: https://stnweb-japan.cas.org/<br>retrieval date 13 December 2010 (13.12.2010) | 12-15<br>1-2<br>3-7,16-37, 42-49 |
| X<br>Y<br>A | RN 575496-29-6 REGISTRY<br>ED Entered STN: 29 Aug 2003<br>CN 2H-1,2,3-Triazolo[4,5-f]quinoline-9-carboxylic acid, 7-(4-nitrophenyl)-2-phenyl- (CA INDEX NAME)<br>MF C22 H13 N5 O4<br>SR Chemical Library<br>Supplier: AsInEx<br>LC STN Files: CHEMCATS<br>URL: https://stnweb-japan.cas.org/<br>retrieval date 13 December 2010 (13.12.2010) | 12-15<br>1-2<br>3-7,16-37, 42-49 |

Form PCT/ISA/210 (continuation of second sheet) (July 2009)

**INTERNATIONAL SEARCH REPORT**

International application No.
PCT/JP2010/067511

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | RN 663947-48-6 REGISTRY<br>ED Entered STN: 17 Mar 2004<br>CN 2H-1,2,3-Triazolo[4,5-f]quinoline-9-carboxylic acid, 2,7-diphenyl- (CA INDEX NAME)<br>MF C22 H14 N4 O2<br>SR Chemical Library<br>Supplier: SPECS<br>URL: https://stnweb-japan.cas.org/<br>retrieval date 13 December 2010 (13.12.2010) | 12-15<br>1-2<br>3-7,16-37,<br>42-49 |
| X<br>Y<br>A | RN 959290-53-0 REGISTRY<br>ED Entered STN: 21 Dec 2007<br>CN 2H-1,2,3-Triazolo[4,5-f]quinoline-9-carboxylic acid, 7-(4-methoxyphenyl)-2-phenyl- (CA INDEX NAME)<br>MF C23 H16 N4 O3<br>SR Other Sources<br>Database: NIST Mass Spectral Library (National Institute of Standards and Technology)<br>URL: https://stnweb-japan.cas.org/<br>retrieval date 13 December 2010 (13.12.2010) | 12-15<br>1-2<br>3-7,16-37,<br>42-49 |
| Y<br>A | JP 50-088099 A (YAMANOUCHI PHARM CO., LTD.),<br>15 July 1975 (15.07.1975),<br>entire text<br>(Family: none) | 1-2<br>3-7,12-37,<br>42-49 |
| A | JP 58-177993 A (USV PHARM CORP.),<br>18 October 1983 (18.10.1983),<br>entire text<br>& EP 90360 A2 | 1-7,12-37,<br>42-49 |
| A | Heleyova, K. et al.,<br>Synthesis and spectral properties of substituted 4-chlorooxazoloquinolines,<br>Monatshefte fuer Chemie (1995), 126(12),<br>p1359-1365 | 1-7,12-37,<br>42-49 |

Form PCT/ISA/210 (continuation of second sheet) (July 2009)

**INTERNATIONAL SEARCH REPORT**

International application No.
PCT/JP2010/067511

**Box No. II Observations where certain claims were found unsearchable (Continuation of item 2 of first sheet)**

This international search report has not been established in respect of certain claims under Article 17(2)(a) for the following reasons:

1. [X] Claims Nos.: 8-11,38-41
because they relate to subject matter not required to be searched by this Authority, namely:
Claims 8 to 11 and 38 to 41 pertain to methods for treatment of the human body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007002313 A
- WO 2001056607 A
- WO 2001047891 A
- WO 2005035526 A
- WO 2003045313 A
- EP 581500 A
- WO 2002056507 A
- WO 200345313 A


### Non-patent literature cited in the description


- *The Japanese Journal of Pharmacology,* 2004, vol. 123, 5-13
- *N. Eng. J. Med.,* 1986, vol. 315, 800-804
- *Am. J. Rhinol.,* 2006, vol. 20, 342-348
- *J. Invest. Dermatol.,* 2002, vol. 119, 609-616
- *The Japanese Journal of Pharmacology,* 2004, vol. 123, 15-22
- *Nat. Rev. Drug Discov.,* 2007, vol. 6, 313-325
- *Mol Cell Pharmacol,* 2010, vol. 2 (3), 89-96
- *Acta Neuropathol.,* 2002, vol. 104, 377-384
- *Am. J. Pathol.,* 2009, vol. 174, 1735-1744
- *J. Biol. Chem.,* 2006, vol. 281, 15277-15286
- *Jpn. J. Pharmacol.,* 1998, vol. 78, 1-10
- *Jpn. J. Pharmacol.,* 1998, vol. 78, 11-22
- **T. W. GREENE.** Protective Groups in Organic Synthesis. John Wiley & Sons Inc, 1999
- *J. Med. Chem.,* 1970, vol. 13, 1117-1124
- *Bioorg. Med. Chem. Lett.,* 2007, vol. 17, 1403-1407
- *Jikken Kagaku Kouza,* 480-495
- **R. C. LAROCK.** Comprehensive Organic Transformations. Vch Verlagsgesellschaft Mbh, 1999
- **INAGAKI et al.** *Int. Arch. Allergy Appl. Immunol.,* 1988, vol. 86 (3), 325-30
- *Laryngoscope,* 2003, vol. 113, 1118-1122
- *Nat. Immunol.,* 2005, vol. 6 (5), 524-531
- *J. Anat.,* 2002, vol. 200, 69-79